# EUROPEAN PATENT APPLICATION

(11) **EP 3 626 339 A1**
(43) Date of publication of application: **25.03.2020**
(21) Application number: 18818393.3
(22) Date of filing: 18.06.2018
(51) Int. Cl.: B01J 31/02, A01N 33/12, A01N 59/08, A01P 3/00, A61K 31/19, A61K 33/04, A61K 33/42, A61P 1/00, A61P 31/00

(54) **RADICAL-GENERATING CATALYST, RADICAL PRODUCTION METHOD, OXIDATION REACTION PRODUCT PRODUCTION METHOD, CHEMICAL AGENT, AND CHEMICAL AGENT FOR AGRICULTURE AND LIVESTOCK**

(30) Priority: 17.06.2017 JP 2017119188; 17.06.2017 JP 2017119189
(71) Applicant: Acenet Inc., Tokyo 105-0021 (JP)
(72) Inventor: TAKAMORI Kiyoto, Tokyo 105-0021 (JP); SHIBATA Takekatsu, Tokyo 105-0021 (JP)
(74) Representative: J A Kemp LLP
(86) International application number: PCT/JP2018/023168
(87) International publication number: WO 2018/230743

(57) **Abstract**

The present invention is intended to provide a radical generating catalyst that can generate (produce) radicals under mild conditions. In order to achieve the above object, the first radical generating catalyst of the present invention includes: at least one selected from the group consisting of amino acids, peptides, phospholipids, and salts thereof. The second or third radical generating catalyst of the present invention includes an ammonium salt represented by the following chemical formula (XI) (excluding peroxodisulfate) and having a Lewis acidity of 0.4 eV or more. 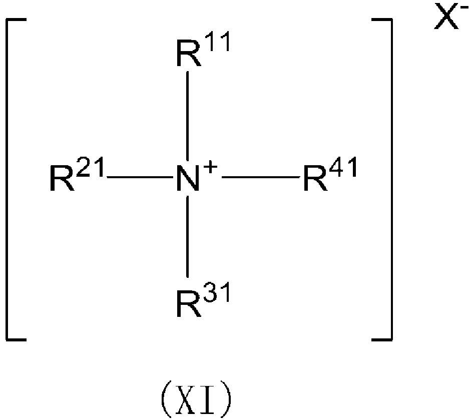 In the chemical formula (XI), R¹¹, R²¹, R³¹, and R⁴¹ are each a hydrogen atom or an aromatic ring, or an alkyl group, the alkyl group may include an ether bond, a carbonyl group, an ester bond, or an amide bond, or an aromatic ring, and R¹¹, R²¹, R³¹, and R⁴¹ may be identical to or different from each other; or two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents. X⁻ is an anion (excluding peroxodisulfate ion).

## Description

### TECHNICAL FIELD

The present invention relates to a radical generating catalyst, a method for producing radicals, a method for producing an oxidation reaction product, a drug, and a drug for use in agriculture and livestock industry.

### BACKGROUND ART

Owing to its high reactivity, a radical is an important chemical species that is used widely. For example, sodium chlorite (NaClO₂) is a non-toxic inexpensive oxidizing reagent and has been used as a precursor of a chlorine dioxide radical (ClO₂˙) (Non Patent Literatures 1 to 4).

### Citation List

### Non Patent Literatures

[Non Patent Literature 1] H. Dodgen and H. Taube, J. Am. Chem. Soc., 1949, 71, 2501-2504.
[Non Patent Literature 2] J. K. Leigh, J. Rajput, and D. E. Richardson, Inorg. Chem., 2014, 53, 6715-6727.
[Non Patent Literature 3] C. L. Latshaw, Tappi J., 1994, 163-166.
[Non Patent Literature 4] (a) J. J. Leddy, in Riegel's Handbook of Industrial Chemistry, 8th edn. Ed., J. A. Kent, Van Nostrand Reinhold Co. Inc, New York, 1983, pp. 212-235; (b) I. Fabian, Coord. Chem. Rev., 2001, 216-217, 449-472.

### SUMMARY OF INVENTION

### Technical Problem

However, high energy is generally required for generating radicals. Thus, heating or the like to raise the temperature is required, which causes problems in cost and reaction control.

On this account, it is an object of the present invention to provide a radical generating catalyst that can generate (produce) radicals under mild conditions, a method for producing radicals using the radical generating catalyst, a method for producing an oxidation reaction product using the radical production method, a drug, and a drug for use in agriculture and livestock industry.

### Solution to Problem

In order to achieve the above object, the present invention provides a first radical generating catalyst including: at least one selected from the group consisting of amino acids, proteins, peptides, phospholipids, and salts thereof.

The present invention also provides a second radical generating catalyst including: an ammonium salt represented by the following chemical formula (XI) (excluding peroxodisulfate) and having a Lewis acidity of 0.4 eV or more. The radical generating catalyst catalyzes radical generation from a radical source in a liquid that is not acidic. The radical source is at least one selected from the group consisting of halogenous acids, halite ions, and halites. 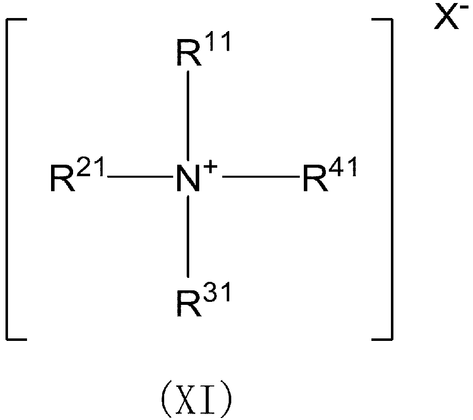 In the chemical formula (XI), R¹¹, R²¹, R³¹, and R⁴¹ are each a hydrogen atom or an aromatic ring, or an alkyl group, the alkyl group may include an ether bond, a carbonyl group, an ester bond, or an amide bond, or an aromatic ring, and R¹¹, R²¹, R³¹, and R⁴¹ may be identical to or different from each other; or two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents. X⁻ is an anion (excluding peroxodisulfate ion).

The present invention also provides a third radical generating catalyst including: an ammonium salt represented by the following chemical formula (XI) and having a Lewis acidity of 0.4 eV or more. The radical generating catalyst catalyzes radical generation from a radical source in the presence of an oxidizing agent. The oxidizing agent is O₂. The radical source is at least one selected from the group consisting of: nitrogen-containing aromatic cation derivatives represented by the following formulae (A-1) to (A-8); 9-substituted acridinium ions represented by the following formula (A-9); quinolinium ion derivatives represented by the following formula (I); stereoisomers and tautomers thereof; and salts thereof. 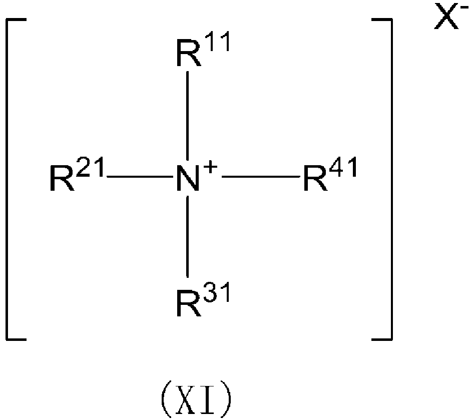 In the chemical formula (XI), R¹¹, R²¹, R³¹, and R⁴¹ are each a hydrogen atom or an aromatic ring, or an alkyl group, the alkyl group may include an ether bond, a carbonyl group, an ester bond, or an amide bond, or an aromatic ring, and R¹¹, R²¹, R³¹, and R⁴¹ may be identical to or different from each other; or two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents. X⁻ is an anion. 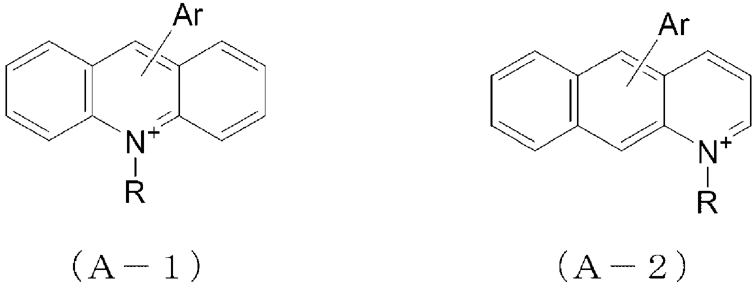 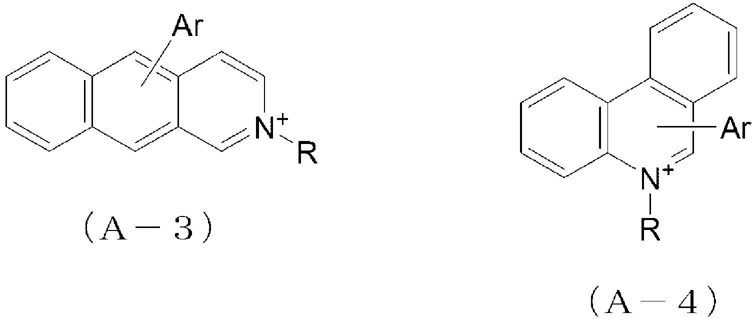 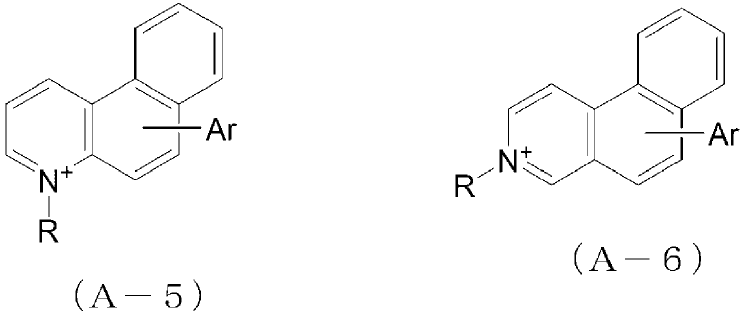 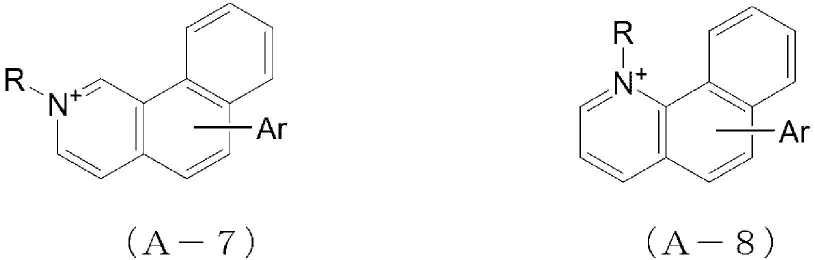 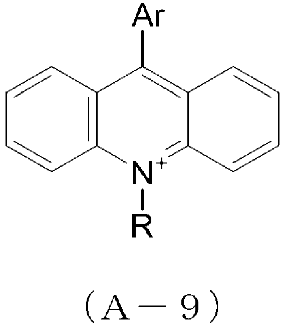 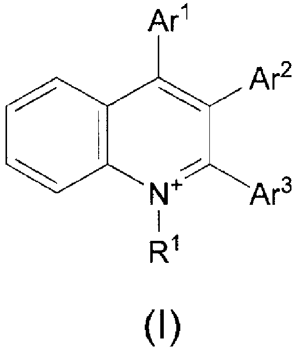 In the formulae (A-1) to (A-8) and (A-9), R is a hydrogen atom or any substituent, Ar is an electron donor group, and the number of Ars may be one or more, and when a plurality of Ars are present, they may be identical to or different from each other, and a nitrogen-containing aromatic ring that forms a nitrogen-containing aromatic cation may or may not have at least one substituent other than R and Ar. In the formula (I), R¹ is a hydrogen atom or any substituent, Ar¹ to Ar³ are each a hydrogen atom or the electron donor group and may be identical to or different from each other, and at least one of Ar¹ to Ar³ is the electron donor group.

In the following description, the first radical generating catalyst of the present invention, the second radical generating catalyst of the present invention, and the third radical generating catalyst of the present invention may be collectively referred to as "the radical generating catalyst of the present invention".

The present invention also provides a first method for producing a radical, the method including: a mixing step of mixing the radical generating catalyst according to the present invention with the radical source.

The present invention also provides a second method for producing a radical, the method including: a mixing step of mixing a Lewis acid having a Lewis acidity of 0.4 eV or more (excluding peroxodisulfate) with a radical source; and a reaction step of reacting the Lewis acid with the radical source in a liquid that is not acidic. The radical source is at least one selected from the group consisting of halogenous acids, halite ions, and halites.

The present invention also provides a third method for producing a radical, the method including: a mixing step of mixing a Lewis acid having a Lewis acidity of 0.4 eV or more, O₂, and a radical source together; and a reaction step of reacting the Lewis acid, the O₂, and the radical source with each other in a liquid. The Lewis acid is the third radical generating catalyst according to the present invention. The radical source is at least one selected from the group consisting of: nitrogen-containing aromatic cation derivatives represented by the formulae (A-1) to (A-8); 9-substituted acridinium ions represented by the formula (A-9); quinolinium ion derivatives represented by the formula (I); stereoisomers and tautomers thereof; and salts thereof.

The present invention also provides a fourth method for producing a radical, the method including: a mixing step of mixing a Lewis acid having a Lewis acidity of 0.4 eV or more (excluding peroxodisulfate) with a radical source; and a reaction step of reacting the Lewis acid with the radical source in a liquid. The Lewis acid having a Lewis acidity of 0.4 eV or more includes an inorganic substance. The radical source is at least one selected from the group consisting of halogenous acids, halite ions, and halites.

In the following description, the first method for producing the radical of the present invention, the second method for producing the radical of the present invention, the third method for producing the radical of the present invention, and the fourth method for producing the radical of the present invention may be collectively referred to as "the method for producing the radical of the present invention".

The present invention also provides a method for producing an oxidation reaction product by oxidizing a substance to be oxidized, the method including: a radical production step of producing a radical by the method according to the present invention; and an oxidation reaction step of reacting the substance to be oxidized with an oxidizing agent by action of the radical, thereby generating the oxidation reaction product.

The present invention also provides a drug including: a radical generating catalyst; and a radical source. The radical generating catalyst is the radical generating catalyst according to the present invention.

The present invention also provides a drug for use in agriculture and livestock industry including: a radical generating catalyst; and a radical source. The radical generating catalyst is the radical generating catalyst according to the present invention.

### Advantageous Effects of Invention

According to the radical generating catalyst, radical-generating agent, and radical production method of the present invention, it is possible to generate (produce) radicals under mild conditions. While the radical generating catalyst, radical-generating agent, and radical production method of the present invention can be used in, for example, the oxidation reaction product production method of the present invention, the use thereof is not limited thereto, and they are applicable to a wide variety of uses.

### BRIEF DESCRIPTION OF DRAWINGS

[FIG. 1] FIG. 1 shows an ultraviolet-visible absorption spectrum of NaClO₂ (5.0 mM) collected 0, 4, and 16 hours after mixing with Sc(OTf)₃ (10 mM) in an aqueous solution at 298 K.
[FIGs. 2A and 2B] FIG. 2A shows a time profile of UV-Vis absorption at 358 nm in formation of Sc³⁺(ClO₂˙) by a reaction between Sc(OTf)₃ (10 mM) and NaClO₂ (5.0 mM) in an aqueous solution (0.20 M acetate buffer having a pH of 2.9) at 298 K. FIG. 2B shows a secondary plot.
[FIGs. 3A and 3B] FIG. 3A shows a time profile of UV-Vis absorption at 358 nm in consumption of Sc³⁺(ClO₂˙) in the presence of styrene (30 to 90 mM) in a MeCN/F₂O (1 : 1 v/v) solution at 298 K. FIG. 3B shows a pseudo first-order rate-styrene concentration plot.
[FIG. 4] FIG. 4 shows EPR spectra of MeCN solutions measured at 298 K. In FIG. 4, (a) shows a spectrum of a MeCN solution that contains NaClO₂ (0.10 mM) at 353 K after 1-hour reflux; (b) shows a spectrum of a MeCN solution that contains NaClO₂ (0.10 mM) and CF₃COOH (10 mM); and (c) shows a spectrum of a MeCN solution that contains NaClO₂ (0.10 mM) and Sc(OTf)₃ (10 mM).
[FIG. 5] FIG. 5 shows bond lengths (Å) of optimized structures calculated by DFT at the level of CAM-B3LYP/6-311+G(d, p). In FIG. 5, (a) shows the result obtained regarding ClO₂; (b) shows the result obtained regarding H⁺ClO₂; and (c) shows the result obtained regarding Sc³⁺ClO₂.
[FIG. 6] FIG. 6 is a spectral diagram showing the result of tracing the reaction of styrene (2.0 mM) by NaClO₂ (20 mM) in an aqueous MeCN solution (MeCN/H₂O 1 : 1 v/v) at room temperature (25°C) utilizing ¹HNMR.
[FIG. 7] FIG. 7 shows ¹HNMR spectra of CD₃CN/D₂O (4 : 1 v/v) that contains styrene (66 mM) and NaClO₂ (200 mM) at 60°C (333 K) collected 0 hours and 25 hours after mixing. The mark "*" indicates the peak derived from styrene oxide.
[FIG. 8] FIG. 8 shows ¹HNMR spectra of CD₃CN/D₂O (1 : 1 v/v) that contains styrene (2.0 mM), NaClO₂ (20 mM), and Sc(OTf)₃ (30 mM) at 25°C collected 0.6 hours and 17 hours after mixing. The mark "*" and the mark "†" indicate the peak derived from 1-phenylethane-1,2-diol and the peak derived from 2-chloro-1-phenylethanol, respectively.
[FIG. 9] FIG. 9 shows ¹HNMR spectra of CD₃CN/D₂O (1 : 1 v/v) that contains styrene (2.0 mM), NaClO₂ (20 mM), and CF₃COOD (30 mM) collected 0.5 hours and 17 hours after mixing. The mark "*" and the mark "†" indicate the peak derived from 1-phenylethane-1,2-diol and the peak derived from 2-chloro-1-phenylethanol, respectively.
[FIG. 10] FIG. 10 is a diagram showing spin distributions calculated by DFT at the level of CAM-B3LYP/6-311+G(d, p). In FIG. 10, (a) shows a spin distribution of H⁺ClO₂˙; and (b) shows a spin distribution of Sc³⁺ClO₂˙.
[FIG. 11] In FIG. 11, (a) is a graph showing the time course of an ultraviolet-visible absorption spectrum of a solution obtained by adding benzethonium chloride(Bzn⁺) to an oxygen saturated solution of a cobalt (II) tetraphenylporphyrin complex Co(II)TPP ([CoTPP] = 9.0 × 10⁻⁶ M, [O₂] = 13 mM) ([Bzn⁺Cl⁻] = 30 mM); and (b) is a graph showing the time course of an increase in the absorption band at 433 nm shown in the graph (a).
[FIG. 12] FIG. 12 show the structure of Bzn⁺ optimized by density functional calculation (B3LYP/6-31G(d) level).
[FIG. 13] FIG. 13 shows an ultraviolet-visible absorption spectrum of NaClO₂ (20 mM) collected after mixing with Sc(OTf)₃ (40 mM) in an aqueous solution at 298 K.
[FIG. 14] In FIG. 14, (a) to (c) are graphs each showing the time course of a reaction when 10-methyl-9,10-dihydroacridine (AcrH₂) (1.4 mM) and sodium chlorite (NaClO₂) (2.8 mM) were added to a mixed solution containing deoxygenated acetonitrile and water (deoxygenated acetonitrile : water = 1 : 1 v/v).
[FIG. 15] In FIG. 15, (a) and (b) are graphs each showing the time course of a reaction when the same mixed solution as in (a) to (c) of FIG. 14 was prepared and Bzn⁺ (0.56 mM) was further added thereto.
[FIG. 16] In FIG. 16, (a) and (b) are graphs each showing the time course of a reaction when the same mixed solution as in (a) and (b) of FIG. 15 was prepared and Sc(OTf)₃ (3.0 mM) was further added thereto.
[FIG. 17] FIG. 17 is a schematic view showing an example of a presumed reaction mechanism of an oxygenation (oxidation) reaction from AcrH₂ to 10-methylacridone.
[FIG. 18] In FIG. 18, (a) is an ultraviolet-visible absorption spectrum showing the result of tracing an oxidation reaction of triphenylphosphine using NaClO₂ and scandium triflate; and (b) is a graph showing the relationship between an initial concentration of Ph₃P and a concentration of generated Ph₃P = O in the reaction shown in (a) of FIG. 18.
[FIG. 19] FIG. 19 shows ¹HNMR spectra of CD₃CN/D₂O (1 : 1 v/v) that contains styrene (2.0 mM), NaClO₂ (6.0 mM), and Sc(OTf)₃ (5.6 mM) at 25°C in the Ar atmosphere collected 0 hours and 45 hours after mixing.
[FIG. 20] FIG. 20 shows the yield etc. in an example where an oxidation reaction product (benzoic acid) was obtained by performing an oxidation reaction of a raw material aromatic compound (benzaldehyde) in acetonitrile in the presence of perchlorate (Acr⁺-Mes ClO₄⁻) of 9-mesityl-10-methylacridinium (Acr⁺-Mes) and oxygen.
[FIG. 21] FIG. 21 is a graph showing the Lewis acidities of benzethonium chloride [Bzn⁺Cl⁻] and various metal complexes.
[FIG. 22] In FIG. 22, (a) is an ultraviolet-visible absorption spectrum showing conversion of triphenylphosphine to triphenylphosphine oxide over time; and (b) is a graph showing the change of a triphenylphosphine (Ph₃P) concentration over time in the presence and the absence of Sc(OTf)₃ (SC³⁺).
[FIG. 23] FIG. 23 is an ESR spectral diagram of the drug of Example.
[FIG. 24] FIG. 24 is an ESR spectral diagram of the drug of Example.
[FIG. 25] FIG. 25 is an ESR spectral diagram of the drug of Example.
[FIG. 26] FIG. 26 is an ESR spectral diagram of the drug of Example.
[FIG. 27] FIG. 27 is a graph showing the suppression effect of the drug of Example on ulcerative colitis.
[FIG. 28] FIG. 28 are graphs showing changes in intestinal bacteria flora with the drug of Example.

### DESCRIPTION OF EMBODIMENTS

The present invention will be described more specifically below with reference to illustrative examples. It is to be noted, however, that the present invention is by no means limited by the following descriptions.

### [1. Radical generating catalyst]

The use of the first to third radical generating catalysts of the present invention is not particularly limited, and for example, as described above, the first to third radical generating catalysts can be used in the first method for producing a radical in the present invention. The first to third radical generating catalysts of the present invention can also be used, for example, in the second to fourth methods for producing the radical of the present invention.
Furthermore, a Lewis acid having a Lewis acidity of 0.4 eV or more can be used in the second to fourth methods for producing a radical of the present invention as described above. It is considered that the Lewis acid having a Lewis acidity of 0.4 eV or more serves as a radical generating catalyst. In the following description, unless otherwise specified, "the radical generating catalyst of the present invention" is not limited to the first to third radical generating catalysts of the present invention, and includes a Lewis acid having a Lewis acidity of 0.4 eV or more.

The radical generating catalyst of the present invention may be, for example, an organic compound or an inorganic substance. The organic substance may be, for example, at least one selected from the group consisting of ammonium, amino acids, peptides, phospholipids, and salts thereof. The inorganic substance may include one or both of metal ions and nonmetal ions. The metal ion may include one or both of typical metal ions and transition metal ions. The inorganic substance may be, for example, at least one selected from the group consisting of alkali earth metal ions, rare earth ions, Sc³⁺, Li⁺, Fe²⁺, Fe³⁺, Al³⁺, silicate ions, and borate ions. Examples of the alkali earth metal ion include ions of calcium, strontium, barium, and radium. More specifically, examples of the alkali earth metal ion include Ca²⁺, Sr²⁺, Ba²⁺, and Ra²⁺. Furthermore the "rare earth metal" is a generic name of a set of seventeen elements, specifically, two elements such as scandium₂₁Sc and yttrium₃₉Y and fifteen elements (lanthanoids) from lanthanum₅₇La to lutetium₇₁Lu. Examples of the rare earth ion include corresponding trivalent cations of the seventeen elements.

The Lewis acid (including the counter ion) may be, for example, at least one selected from the group consisting of CaCl₂, MgCl₂, FeCl₂, FeCl₃, AlCl₃, AlMeCl₂, AlMe₂Cl, BF₃, BPh₃, BMe₃, TiCl₄, SiF₄, and SiCl₄. It is to be noted that the "Ph" indicates a phenyl group and the "Me" indicates a methyl group.

In the radical generating catalyst of the present invention, the radical generating catalyst can be selected appropriately depending on the intended use thereof, with consideration given to the reactivity, acidity, safety, and the like.

The inventors of the present invention found out through research that ammonium (in particular, organic ammonium), amino acids, peptides, and phospholipids function as radical generating catalysts. As a result of further research, the inventors of the present invention found out that ammonium, amino acids, peptides, and phospholipids that function as radical generating catalysts may have properties as a Lewis acid. That is, although the reason why the ammonium, amino acids, peptides, and phospholipids function as radical generating catalysts is not clear, it is presumably because the ammonium, amino acids, peptides, and phospholipids each have a function as a Lewis acid. As a result of still further research, the inventors of the present invention found out a radical generating catalyst including an organic compound having Lewis acidic properties and/or Brønsted acidic properties. In the present invention, the "Lewis acid" refers to a substance that serves as a Lewis acid with respect to the radical source, for example.

The Lewis acidity of the radical generating catalyst of the present invention is, for example, 0.4 eV or more, 0.5 eV or more, or 0.6 eV or more. The upper limit of the Lewis acidity is not particularly limited, and is, for example, 20 eV or less. In the present invention, a criterion for judging that the Lewis acidity is equal to, greater than, or less than the above-described numerical value is, for example, whether the measured value by any one of the "Lewis acidity measuring method (1)" and the "Lewis acidity measuring method (2)" described below is equal to, greater than, or less than the above-described numerical value.

The Lewis acidity can be measured, for example, by the method described in Ohkubo, K.; Fukuzumi, S. Chem. Eur. J., 2000, 6, 4532, J. Am. Chem. Soc. 2002, 124, 10270-10271 or the method described in J. Org. Chem. 2003, 68, 4720-4726. Specifically, the Lewis acidity can be measured by the following "Lewis acidity measuring method (1)".

### (Lewis acidity measuring method (1))

As to acetonitrile (MeCN) that contains cobalt tetraphenylporphyrin, saturated O₂, and an object whose Lewis acidity is to be measured (e.g., a cation of a metal or the like, represented by Mⁿ⁺ in the following chemical reaction formula (1a)) in the following chemical reaction formula (1a), the change of the ultraviolet-visible absorption spectrum is measured at room temperature. On the basis of the obtained reaction rate constant (k_{cat}), the ΔE value (eV), which is an indicator of the Lewis acidity, can be calculated. The higher the k_{cat}, the stronger the Lewis acidity. Furthermore, the Lewis acidity of an organic compound can be estimated from the energy level of the lowest unoccupied molecular orbital (LUMO) calculated by the quantum chemical calculation. The higher the value at the positive side, the stronger the Lewis acidity.

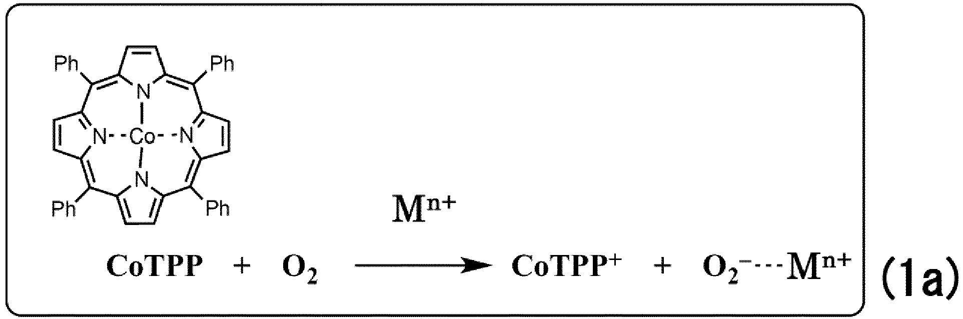

Examples of the reaction rate constant of reaction between CoTPP and oxygen in the presence of a Lewis acid, which is an indicator of the Lewis acidity measured (calculated) by the above-described measurement method, are shown below. In the following table, the numerical value expressed in the unit "k_{cat}, M⁻²s⁻¹" is a reaction rate constant of reaction between CoTPP and oxygen in the presence of a Lewis acid. The numerical value expressed in the unit "LUMO, eV" is the energy level of LUMO. The "benzethonium chloride" means benzethonium chloride, "benzalkonium chloride" means benzalkonium chloride, "tetramethylammonium hexafluorophosphate" means tetramethylammonium hexafluorophosphate, "tetrabutylammonium hexafluorophosphate" means tetrabutylammonium hexafluorophosphate, and "ammonium hexafluorophosphate" means ammonium hexafluorophosphate (Note from translator: in the original text in Japanese, the above sentence explains the meanings of the English terms in the table in Japanese).

**[Table tpp]**

| | LUMO, eV | *k*_{cat}, M⁻² s⁻¹ |
|---|---|---|
| benzethonium chloride | -4.12 | 0.24 |
| benzalkonium chloride | -4.02 | 0.18 |
| tetramethylammonium hexafluorophosphate | -3.58 | > 0.1 |
| tetrabutylammonium hexafluorophosphate | -2.07 | > 0.1 |
| ammonium hexafluorophosphate | -5.73 | 20 |

In the present invention, the Lewis acidity may be measured by reducing ubiquinone 1 using ubiquinone 1 (Q1) instead of oxygen molecule (O₂) to generate an anion radical of ubiquinone 1 in the Lewis acidity measuring method (1). In the following description, such a Lewis acidity measuring method may be referred to as the "Lewis acidity measuring method (2)". In the Lewis acidity measuring method (2), the measurement can be performed in the same manner as in the Lewis acidity measuring method (1), except that ubiquinone 1 (Q1) is used instead of oxygen molecule (O₂). In the Lewis acidity measuring method (2), similarly to the Lewis acidity measuring method (1), the ΔE value (eV), which is an index of Lewis acidity, can be calculated from the obtained reaction rate constant (k_{cat}). The Lewis acidity measuring method (2) is, for example, described in Ohkubo, K.; Fukuzumi, S. Chem. Eur. J., 2000, 6, 4532, and can be performed according to or based on the method described therein.

The Lewis acidity measuring method (2) can be performed by measuring the reaction rate constant (k_{cat}) with respect to the following chemical reaction formula (1b). 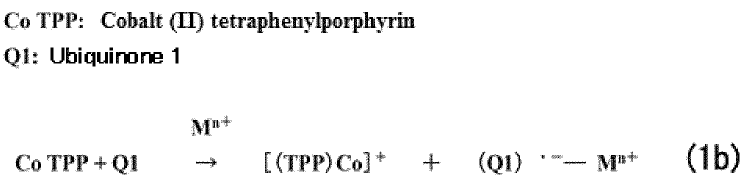 in the chemical formula (1b),
Mⁿ⁺ represents the radical generating catalyst,
CoTPP represents cobalt (II) tetraphenylporphyrin,
Q1 represents ubiquinone 1,
[(TPP)Co]⁺ represents cobalt (III) tetraphenylporphyrin cation, and
(Q1)˙⁻ represents an anionic radical of ubiquinone 1.

The Lewis acidity of the radical generating catalyst of the present invention may have a reaction rate constant (k_{cat}) for the chemical reaction formula (1b), i.e., a measured value (K_{obs}) of the reaction rate constant (k_{cat}) measured by the "Lewis acidity measuring method (2)", of, for example, 1.0 × 10⁻⁵S⁻¹ or more, 2.0 × 10⁻⁵S⁻¹ or more, 3.0 × 10⁻⁵S⁻¹ or more, 4.0 × 10⁻⁵S⁻¹ or more, 5.0 × 10⁻⁵S⁻¹ or more, 6.0 × 10⁻⁵S⁻¹ or more, 7.0 × 10⁻⁵S⁻¹ or more, 8.0 × 10⁻⁵S⁻¹ or more, 9.0 × 10⁻⁵S⁻¹ or more, 1.0 × 10⁻⁴S⁻¹ or more, 2.0 × 10⁻⁴S⁻¹ or more, 3.0 × 10⁻⁴S⁻¹ or more, 4.0 × 10⁻⁴S⁻¹ or more, 5.0 × 10⁻⁴S⁻¹ or more, 6.0 × 10⁻⁴S⁻¹ or more, 7.0 × 10⁻⁴S⁻¹ or more, 8.0 × 10⁻⁴S⁻¹ or more, 9.0 × 10⁻⁴S⁻¹ or more, 1.0 × 10⁻³S⁻¹ or more, 2.0 × 10⁻³S⁻¹ or more, 3.0 × 10⁻³S⁻¹ or more, 4.0 × 10⁻³S⁻¹ or more, 5.0 × 10⁻³S⁻¹ or more, 6.0 × 10⁻³S⁻¹ or more, 7.0 × 10⁻³S⁻¹ or more, 8.0 × 10⁻³S⁻¹ or more, 9.0 × 10⁻³S⁻¹ or more, 1.0 × 10⁻²S⁻¹ or more, 2.0 × 10⁻²S⁻¹ or more, 3.0 × 10⁻²S⁻¹ or more, 4.0 × 10⁻²S⁻¹ or more, 5.0 × 10⁻²S⁻¹ or more, 6.0 × 10⁻²S⁻¹ or more, 7.0 × 10⁻²S⁻¹ or more, 8.0 × 10⁻²S⁻¹ or more, or 9.0 × 10⁻²S⁻¹ or more; or 1.0 × 10⁻¹S⁻¹ or less, 9.0 × 10⁻²S⁻¹ or less, 8.0 × 10⁻²S⁻¹ or less, 7.0 × 10⁻²S⁻¹ or less, 6.0 × 10⁻²S⁻¹ or less, 5.0 × 10⁻²S⁻¹ or less, 4.0 × 10⁻²S⁻¹ or less, 3.0 × 10⁻²S⁻¹ or less, 2.0 × 10⁻²S⁻¹ or less, 1.0 × 10⁻²S⁻¹ or less, 9.0 × 10⁻³S⁻¹ or less, 8.0 × 10⁻³S⁻¹ or less, 7.0 × 10⁻³S⁻¹ or less, 6.0 × 10⁻³S⁻¹ or less, 5.0 × 10⁻³S⁻¹ or less, 4.0 × 10⁻³S⁻¹ or less, 3.0 × 10⁻³S⁻¹ or less, 2.0 × 10⁻³S⁻¹ or less, 1.0 × 10⁻³S⁻¹ or less, 9.0 × 10⁻⁴S⁻¹ or less, 8.0 × 10⁻⁴S⁻¹ or less, 7.0 × 10⁻⁴S⁻¹ or less, 6.0 × 10⁻⁴S⁻¹ or less, 5.0 × 10⁻⁴S⁻¹ or less, 4.0 × 10⁻⁴S⁻¹ or less, 3.0 × 10⁻⁴S⁻¹ or less, 2.0 × 10⁻⁴S⁻¹ or less, 1.0 × 10⁻⁴S⁻¹ or less, 9.0 × 10⁻⁵S⁻¹ or less, 8.0 × 10⁻⁵S⁻¹ or less, or 7.0 × 10⁻⁵S⁻¹ or less.

In the radical generating catalyst of the present invention, the ammonium may be, for example, quaternary ammonium, or tertiary, secondary, primary or zero ammonium. The ammonium is not particularly limited, and may be, for example, a nucleic acid base or the like, or an amino acid, a peptide, or the like described below.

In the radical generating catalyst of the present invention, at least one selected from the group consisting of ammonium, amino acids, peptides, phospholipids, and salts thereof (the first radical generating catalyst according to the present invention) or the compound having Lewis acidic properties and/or Brønsted acidic properties (the second radical generating catalyst according to the present invention) may be, for example, a cationic surfactant, which may be a quaternary ammonium-type cationic surfactant. Examples of the quaternary ammonium-type cationic surfactant include benzalkonium chloride, benzethonium chloride, cetylpyridinium chloride, hexadecyltrimethylammonium bromide, dequalinium chloride, edrophonium, didecyldimethylammonium chloride, tetramethylammonium chloride, tetrabutylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines (e.g., choline chlorides [such as benzoyl choline chloride and a lauroylcholine chloride hydrate], phosphocholine, acetylcholine, choline, dipalmitoylphosphatidylcholine, and choline bitartrate). It is to be noted, however, that, in the radical production method of the present invention, the quaternary ammonium is not limited to a surfactant.

In the radical generating catalyst of the present invention, the ammonium may be ammonium represented by the following chemical formula (XI), for example.

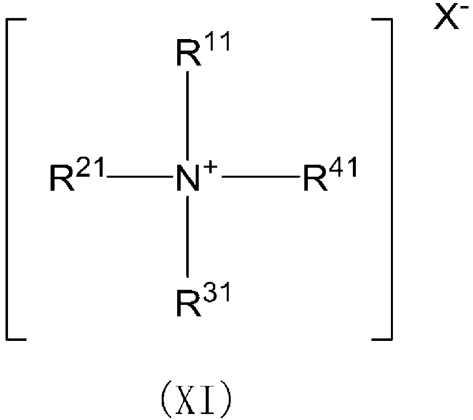

In the chemical formula (XI), R¹¹, R²¹, R³¹, and R⁴¹ are each a hydrogen atom or an aromatic ring, or an alkyl group and the alkyl group may include an ether bond, a carbonyl group, an ester bond, or an amide bond, or an aromatic ring, and R¹¹, R²¹, R³¹, and R⁴¹ may be identical to or different from each other, or two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents, and X⁻ is an anion, and X⁻ is, for example, an anion excluding peroxodisulfate ion. In R¹¹, R²¹, R³¹, and R⁴¹, the aromatic ring is not particularly limited, and may or may not contain a heteroatom, and may or may not have a substituent, for example. Examples of the aromatic ring containing a heteroatom (heteroaromatic ring) include a nitrogen-containing aromatic ring, a sulfur-containing aromatic ring, and an oxygen aromatic ring. Examples of the aromatic ring not containing a heteroatom include a benzene ring, a naphthalene ring, an anthracene ring, and a phenanthrene ring. Examples of the heteroaromatic ring include a pyridine ring, a thiophene ring, and a pyrene ring. The nitrogen-containing aromatic ring may or may not have a positive charge, for example. Examples of the nitrogen-containing aromatic ring having no positive charge include a pyrroline ring, a pyridine ring, a pyridazine ring, a pyrimidine ring, a pyrazine ring, a quinoline ring, an isoquinoline ring, an acridine ring, a 3,4-benzoquinoline ring, a 5,6-benzoquinoline ring, a 6,7-benzoquinoline ring, a 7,8-benzoquinoline ring, a 3,4-benzoisoquinoline ring, a 5,6-benzoisoquinoline ring, a 6,7-benzoisoquinoline ring, and a 7,8-benzoisoquinoline ring. Examples of the nitrogen-containing aromatic ring having a positive charge include a pyrrolinium ring, a pyridinium ring, a pyridazinium ring, a pyrimidinium ring, a pyrazinium ring, a quinolinium ring, an isoquinolinium ring, an acridinium ring, a 3,4-benzoquinolinium ring, a 5,6-benzoquinolinium ring, a 6,7-benzoquinolinium ring, a 7,8-benzoquinolinium ring, a 3,4-benzoisoquinolinium ring, a 5,6-benzoisoquinolinium ring, a 6,7-benzoisoquinolinium ring, and a 7,8-benzoisoquinolinium ring. The oxygen-containing aromatic ring or the sulfur-containing aromatic ring may be, for example, an aromatic ring in which at least one of a carbon atom or a nitrogen atom of the above-described heteroatom-free aromatic ring or nitrogen-containing aromatic ring is substituted with at least one of an oxygen atom and a sulfur atom. In R¹¹, R²¹, R³¹, and R⁴¹, when the alkyl group or the aromatic ring has a substituent, the substituent is not particularly limited, is optional, and examples thereof include a sulfo group, a nitro group, and a diazo group.

The ammonium represented by the chemical formula (XI) may be ammonium represented by the following chemical formula (XII), for example.

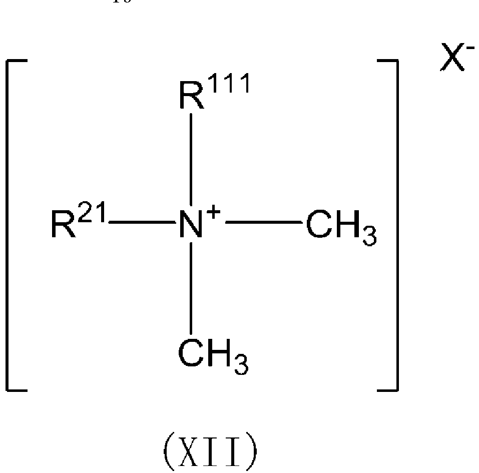

In the chemical formula (XII), R¹¹¹ is an alkyl group having 5 to 40 carbon atoms and may include an ether bond, a ketone (carbonyl group), an ester bond, or an amide bond, substituent, or an aromatic ring, and R²¹ and X⁻ are the same as those in the chemical formula (XI). In R¹¹¹, the aromatic ring is not particularly limited, and may or may not contain a heteroatom, and may or may not have a substituent, for example. In R¹¹¹, the aromatic ring is not particularly limited, and specific examples are the same as those in R¹¹, R²¹, R³¹, and R⁴¹ of the chemical formula (XI). In R¹¹¹, when the alkyl group or the aromatic ring has a substituent, the substituent is not particularly limited, is optional, and, for example, are the same as those in R¹¹, R²¹, R³¹, and R⁴¹ of the chemical formula (XI).

In the chemical formula (XII), R²¹ may be a methyl group or a benzyl group, for example. In the benzyl group, one or more hydrogen atoms on the benzene ring may or may not be substituted with any substituent. The substituent may be, for example, an alkyl group, an unsaturated aliphatic hydrocarbon group, an aryl group, a heteroaryl group, a halogen, a hydroxy group (-OH), a mercapto group (-SH), or an alkylthio group (-SR, where R is an alkyl group).

The ammonium salt represented by the chemical formula (XII) may be ammonium represented by the following chemical formula (XIII), for example.

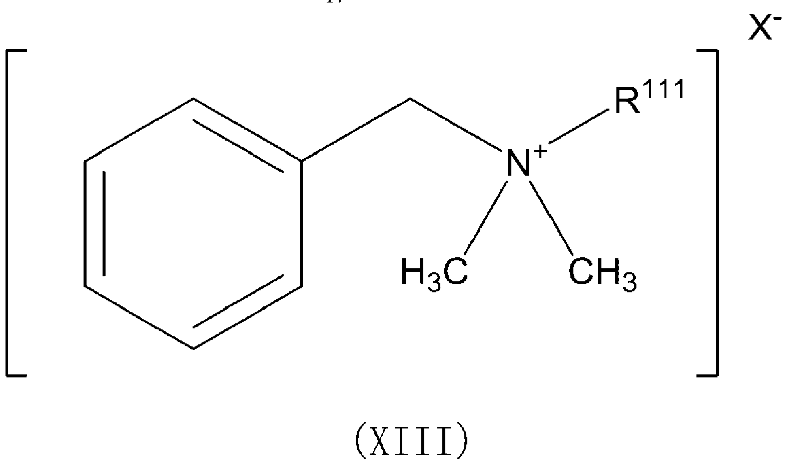

In the chemical formula (XIII), R¹¹¹ and X⁻ are the same as those in the chemical formula (XII).

The ammonium represented by the chemical formula (XI) may be ammonium salt represented by the following chemical formula (XIV), for example. 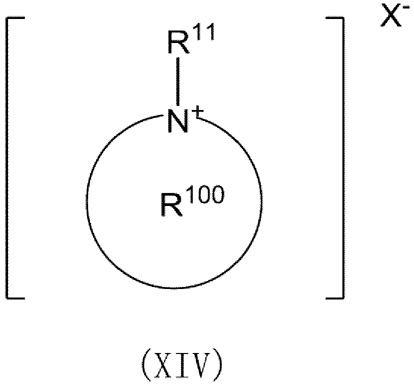 where in the chemical formula (XIV), R¹⁰⁰ may form a ring structure, which may be saturated or unsaturated, aromatic or non-aromatic, and may or may not have one or more substituents, and R¹¹ and X⁻ are the same as those in the chemical formula (XI).

The ammonium salt represented by the chemical formula (XI) may be ammonium salt represented by the following chemical formula (XV), for example.

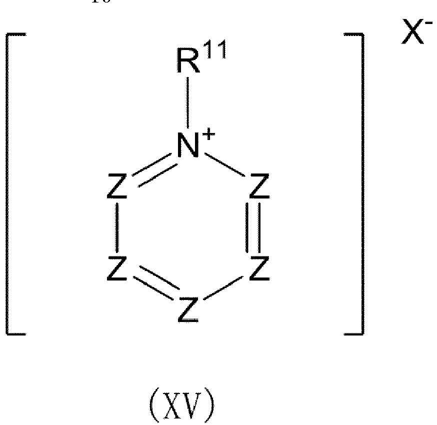

In the chemical formula (XV), Zs are each CH or N, may be identical to or different from each other, and in the case of CH, H may be substituted with a substituent, and R¹¹ and X⁻ are the same as those in the chemical formula (XI).

The ammonium salt represented by the chemical formula (XI) may be ammonium salt represented by the following chemical formula (XVI), for example.

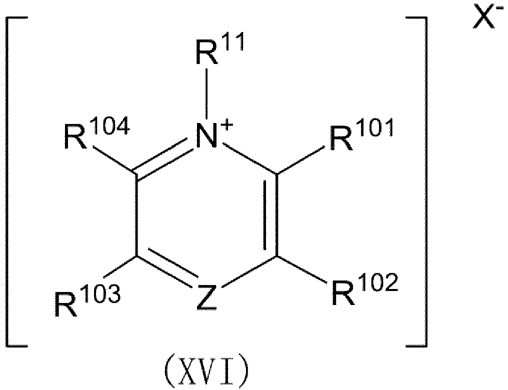

In the chemical formula (XVI), R¹⁰¹, R¹⁰², R¹⁰³, and R¹⁰⁴ are each a hydrogen atom or a substituent, and R¹⁰¹, R¹⁰², R¹⁰³, and R¹⁰⁴ may be identical to or different from each other, or two or more of R¹⁰¹, R¹⁰², R¹⁰³, and R¹⁰⁴ may be integrated to form a ring structure with N⁺ to which they are bonded, and the ring structure may be saturated or unsaturated, aromatic or non-aromatic, and may or may not have one or more substituents, Z is CH or N, and in the case of CH, H may be substituted with a substituent, and R¹¹ and X⁻ are the same as those in the chemical formula (XI).

The ammonium salt represented by the chemical formula (XI) may be ammonium salt represented by the following chemical formula (XVII), for example.

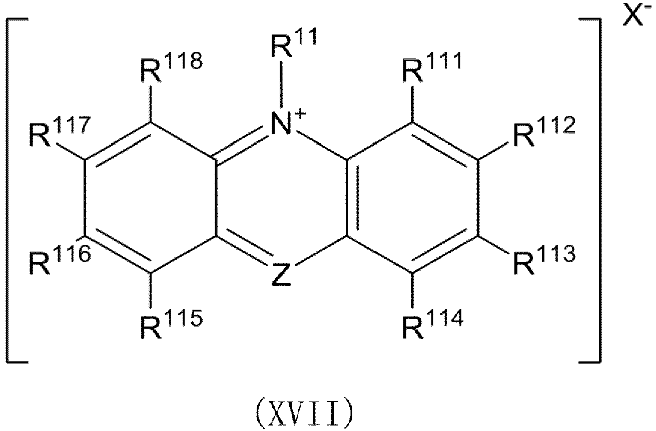

In the chemical formula (XVII), R¹¹¹ to R¹¹⁸ are each a hydrogen atom or a substituent, and may be identical to or different from each other, or two or more of R¹¹¹ to R¹¹⁸ may be integrated to form a ring structure, which may be aromatic or non-aromatic and may or may not have one or more substituents, Z is CH or N, and in the case of CH, H may be substituted with a substituent, and R¹¹ and X⁻ are the same as those in the chemical formula (XI).

The ammonium salt represented by the chemical formula (XI) may be, for example, at least one selected from the group consisting of benzethonium chloride, benzalkonium chloride, hexadecyltrimethylammonium chloride, tetramethylammonium chloride, ammonium chloride, methylammonium chloride, and tetrabutylammonium chloride. It is particularly preferable that the ammonium salt represented by the chemical formula (XII) is benzethonium chloride.

Benzethonium chloride (Bzn⁺Cl⁻) can be represented by the following chemical formula, for example. Benzalkonium chloride can be, for example, a compound represented by the chemical formula (XIII) where R¹¹¹ is an alkyl group having 8 to 18 carbon atoms and X⁻ is a chloride ion.

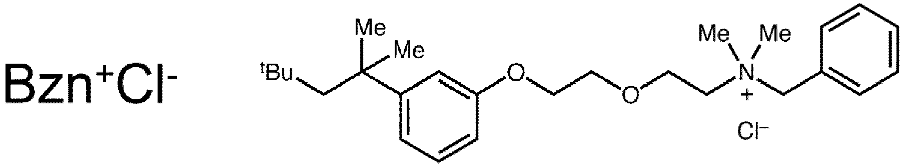

In the chemical formulae (XI), (XII), (XIII), (XIV), (XV), (XVI), and (XVII), X⁻ may be any anion and is not particularly limited. X⁻ is not limited to a monovalent anion, and may be an anion with any valence, such as a divalent anion or a trivalent anion. When the anion is an anion with a plurality of electric charges, such as a divalent anion or a trivalent anion, the number of molecules of the ammonium (monovalent) in each of the chemical formulae (XI), (XII), (XIII), (XIV), (XV), (XVI), and (XVII) is determined by, for example, [the number of molecules of the anion × the valence of the anion] (e.g., when the anion is divalent, the number of molecules of the ammonium (monovalent) is twice the number of molecules of the anion). X⁻ may be, for example, a halogen ion (a fluoride ion, a chloride ion, a bromide ion, or an iodide ion), an acetate ion, a nitrate ion, or a sulfate ion.

In the present invention, the radical generating catalyst is not limited to the chemical formulae (XI), (XII), (XIII), (XIV), (XV), (XVI) and (XVII), and may be ammonium having any structure containing an aromatic ring. The aromatic ring is not particularly limited, and may be, for example, an aromatic ring exemplified in R¹¹, R²¹, R³¹, and R⁴¹ of the chemical formula (XI).

In the present invention, the radical generating catalyst may be, for example, a sulfonic acid amine or ammonium thereof. The sulfonic acid amine is, for example, amine having a sulfonic group (sulfonic acid group) in its molecule. Examples of the sulfonic acid amine include taurine, sulfamic acid, 3-amino-4-hydroxy-1-naphthalenesulfonic acid, sulfamic acid, p-toluidine-2-sulfonic acid, o-anisidine-5-sulfonic acid, direct blue 14, 3- [N, N-bis (2-hydroxyethyl)amino]-2-hydroxypropanesulfonic acid, 3-[(3-colamidopropyl)dimethylammonio]-1-propanesulfonate, aminomethanesulfonic acid, 3-sulfopropylamine, 2-aminobenzenesulfonic acid, R(+)-3-aminotetrahydrofuran, toluene, 4-amino-5-hydroxy-1,7-naphthalenedisulfonic acid, N-(2-acetamido)-2-aminoethanesulfonic acid, 4'-amino-3'-methoxyazobenzene-3-sodium sulfonate, Lapatinib ditosylate, N-tris (hydroxymethyl) methyl-2-aminoethanesulfonic acid, 8-amino-1,3,6-naphthalenetrisulfonic acid disodium hydrate, 1-aminonaphthalene-2-sulfonic acid, (2S,3S)-3-Amino-2-methyl-4-oxo-1-azetidinesulfonic acid, sodium 3- (1-naphthylamino) propanesulfonate, 3-methyl-4-aminobenzenesulfonic acid, sodium 3- Cyclohexylamino-2-hydroxypropanesulfonic acid, sodium N-tris (hydroxymethyl) methyl-2-aminoethanesulfonic acid, 4-amino-1-naphthalenesulfonic acid, sodium sulfamate, tricaine, sodium sulfanilate, 1,4-phenylenediamine 2-sulfonic acid, p- anisidine-2-sulfonic acid, 6-amino-1-naphthalenesulfonic acid, 3,4-diaminobenzene sulfonic acid, 3-amino-4-chlorobenzene sulfonic acid, 3-[(4-Amino-3-methylphenyl) azo] benzenesulfonic acid, 3-amino-4-hydroxy-5-nitrobenzenesulfonic acid, 5-amino-6-hydroxy-3-nitrobenzenesulfonic acid, 4-acetamide-2-Aminobenzenesulfonic acid hydrate, 2-aminophenol-4-sulfonic acid, 1-amino-2-methoxy-5-methyl-4-benzenesulfonic acid, dansylic acid, Sulfamic acid [(IS, 2S, 4R)-4- [4-[[(1S)-2,3-dihydro-1H-inden-1-yl] amino]-7H-pyrrolo [2,3-d] pyrimidin-7-yl]-2- hydroxycyclopropyl] methyl ester, 5-sulfo-4'-diethylamino-2,2' dihydroxyazobenzene, 2-aminonaphthalene-6,8-disulfonic acid, sodium 2- [N,N-bis (2-hydroxyethyl) amino]-1-ethanesulfonate, 3-acetyl-2- (methylaminosulfonyl) thiophene, sodium 4-amino-2-chlorotoluene-5-sulfonate, 5- (3-AMINO-5-OXO-2-PYRAZOLIN-1-YL)-2-PHENOXYBENZENESULFONIC ACID, potassium sulfamate, P-AMINOAZOBENZENE MONOSULFONIC ACID, 3-[(3-Cholamidopropyl) dimethylamino]-2-hydroxy-1-propanesulfonate, 3-amino-2,7-naphthalenes disulfonic acid monosodium, 3- [N, N-bis (hydroxyethyl) amino]-2-hydroxypropanesulfonic acid sodium salt, di (amidosulfuric acid) cobalt (II), 3- (4-amino-3-methoxyphenylazo) benzenesulfonic acid, Nickel (II) sulfamate tetrahydrate, sodium 2,4-diaminobenzenesulfonate, 5-amino-2-chlorotoluene-4-sulfonic acid, 2,5-dichlorosulfanilic acid, 4-methylbenzenesulfonic acid, APTS (aminopyrenetrisulfonic acid), 4'-aminoazobenzene-3-sulfonic acid, Pontacyl carmine 2B, p-anisidine-3-sulfonic acid, 4,4'-bis (4-amino-1-naphthylazo)-2,2'-stilbenesulfonic acid, 3-AMINONAPHTHALENE-8-HYDROXY-4, 6-DISULFONIC ACID, sodium 4-amino-1,5-naphthalenedisulfonate, sodium 4-aminoazobenzene-4'-sulfonate, 5-amino-2-methylbenzenesulfonic acid, disodium 7-amino-1,3-naphthalene disulfonate, alizarin safilol SE, sodium 7-amino-2-naphthalenesulfonate, 6-amino-5-bromopyridine-3-sulfonic acid, 2-aminoethanethiol p-toluenesulfonate, sodium 2-amino 1-naphthalenesulfonate, 6-amino-1,3-naphthalenedisulfonic acid disodium hydrate, N,N,N',N'-tetraethylsulfamide, 5-amino-2-ethoxybenzenesulfonic acid, 3,5-diamino-2,4,6-trimethylbenzenes Phosphonic acid, 7-amino-1-naphthalenesulfonic acid, sulfamic acid, guanidine, 2-amino-5-nitrobenzenesulfonic acid, nickel (II) diamide sulfate, 4-amino-4'-nitrostilbene-2,2'-disulfonic acid disodium, aniline-2,5-disulfonic acid monosodium, 5-amino-1-naphthol-3-sulfonic acid hydrate, 2,5-dichlorosulfanilic acid sodium salt, 6-aminohexanoic acid hexyl p-toluenesulfonate, rac- (R*)-2- (4-chlorophenyl)-3-amino-1-propanesulfonic acid, 2-(N,N-dipropyl) amino anisole-4-Sulfonic acid, 2-amino-4-chlorophenol-6-sulfonic acid, 6-amino-1,3-naphthalenedisulfonic acid, 5,10,15,20-tetrakis [4- (trimethylammonio) phenyl]-21H,-23H-porphine tetratosylate, 5-amino-2-[(4-aminophenyl) amino] benzenesulfonic acid, 4-amino-3-chlorobenzenesulfonic acid, 2-aminobenzenesulfonic acid phenyl ester, 4- Acetylamino-4'-isothiocyanatostilbene-2,2'-disulfonic acid disodium salt, (S)-3-AMINO-2-OXETANONE P-TOLUENESULFONIC ACID SALT, 5-acetylamino-4-hydroxy-2,7-naphthalenedisulfonic acid disodium salt, 2-phenylamino-5-aminobenzenesulfonic acid, sodium 4-octadecylamino-4- oxo-2-[(sodiooxy)sulfonyl]butanoate, and 3,5-diamino-4-methylbenzenesulfonic acid.

In the present invention, the radical generating catalyst may be, for example, nicotinic amine or ammonium thereof. The nicotinic amine is, for example, amine having a ring structure in a molecule, and the ring structure has a nicotine skeleton. Examples of the nicotinic amine include nicotinamide and alkaloid.

In the present invention, the radical generating catalyst may be, for example, nitrite amine or nitrite ammonium. The nitrite amine or nitrite ammonium is, for example, a compound obtained by reacting amine with nitrous acid or a nitrous acid derivative. Examples of the nitrite amine or nitrite ammonium include diazo compounds, diazonium salts, N-nitroso compounds, and C-nitroso compounds.

In the present invention, the ammonium may include a plurality of ammonium structures (N⁺) in one molecule. Further, the ammonium may form a dimer, trimer, or the like by association of a plurality of molecules through a π electron interaction, for example.

In the present invention, the amino acid is not particularly limited. The amino acid may contain, for example, at least one of both an amino group or an imino group and a carboxy group in the molecule. The amino acid may be, for example, an α-amino acid, a β-amino acid, a γ-amino acid, or any other amino acid. The amino acid may be, for example, an amino acid constituting protein, and specifically may be at least one selected from the group consisting of, for example, glycine, alanine, valine, leucine, isoleucine, serine, threonine, aspartic acid, glutamic acid, asparagine, glutamine, lysine, hydroxylysine, arginine, cysteine, cystine, methionine, phenylalanine, tyrosine, tryptophan, histidine, proline, and 4-hydroxyproline.

In the present invention, the peptide is not particularly limited. The peptide may be, for example, one in which two or more of the amino acid molecules are linked by a peptide bond. The peptide may be, for example, at least one of oxidized glutathione (GSSG) and reduced glutathione (GSH).

In the present invention, the phospholipid is not particularly limited. The phospholipid may be, for example, a lipid containing phosphorus atoms in the molecule, and may be, for example, a lipid containing a phosphate ester bond (P-O-C) in the molecule. The phospholipid may or may not have, for example, at least one of an amino group, an imino group, an ammonium group, and an iminium group in a molecule. The phospholipid may be, for example, at least one selected from the group consisting of phosphatidylserine, phosphatidylcholine, phosphatidic acid, phosphatidylethanolamine, phosphatidylglycerol, and cardiolipin.

The radical-generating agent of the present invention may include, for example, a Bronsted acid. The Bronsted acid has an acid dissociation constant pKₐ of, for example, 5 or more. The upper limit of the pKₐ is not particularly limited and is, for example, 50 or less.

The radical generating catalyst of the present invention may, for example, catalyze the radical generation from a radical source *in vitro*, or may catalyze the radical generation from a radical source *in vivo.* The living body may be, for example, a human body, or may be a body of an animal other than a human.

The radical generating catalyst of the present invention may, for example, catalyze the radical generation from a radical source in a digestive organ. The digestive organ may be, for example, at least one selected from the group consisting of an oral cavity, a pharynx, an esophagus, a stomach, a duodenum, a small intestine, and a large intestine. The digestive organ may be, for example, the large intestine. The small intestine may be, for example, at least one selected from the group consisting of duodenum, jejunum and ileum. The large intestine may be at least one selected from the group consisting of, for example, cecum, colon and rectum. The radical generating catalyst of the present invention may be used, for example, for sterilizing in the digestive organ, induction of changes in intestinal bacterial flora, treatment or suppression of symptoms of ulcerative colitis, and the like.

### [2. Radical production method]

The method for producing the radical of the present invention will be described below.

As described above, the radical production method according to the present invention includes a missing step of mixing the radical generating catalyst of the present invention with a radical source. The mixture obtained in the mixing step may or may not further contain any substance other than the radical generating catalyst of the present invention and the radical source. For example, in the mixing step, it is preferable to further mix a solvent from the viewpoint of reactivity and the like. In the present invention, the "solvent" may or may not dissolve the radical generating catalyst, the radical source, and the like. For example, after the mixing step, the radical generating catalyst of the present invention and the radical source may each be in a state of being dissolved in the solvent, or may each be in a state of being dispersed or precipitated in the solvent.

The radical production method of the present invention includes, for example, after the mixing step, a radical production step of producing radicals through a reaction in the obtained mixture. As described above, the mixture may be in the form of a solution, a suspension, or a colloid, for example. From the viewpoint of reactivity, it is preferable that the mixture is in the form of a solution or a colloid, for example. In the radical production step, the mixture may be merely allowed to stand still at room temperature, or may be subjected to heating, light irradiation, or the like when necessary, for example. The reaction temperature and the reaction time in the radical production step are not particularly limited, and can be set as appropriate depending on the type of the reactant (raw material), the type of a desired product, etc., for example. When the mixture is irradiated with light, the wavelength of the light is not particularly limited, and can be set as appropriate depending on the absorption band of the reactant (raw material), etc., for example. The reaction time and the reaction temperature also can be adjusted by, for example, adjusting the concentrations of the radical source and the radical generating catalyst of the present invention in the mixture. The reaction time can be shortened by setting the concentrations higher, for example. It is to be noted, however, that the present invention is not limited by this description.

The concentration of the radical generating catalyst of the present invention is not particularly limited, for example, the reaction mol/l relative to the solvent is not particularly limited, and can be set as appropriate depending on the type of the reactant (raw material), the type of a desired product, etc., for example. Also, the solvent is not particularly limited. For example, the solvent may be either water or an organic solvent. The organic solvent may be, for example: a halogenated solvent such as methylene chloride, chloroform, or carbon tetrachloride; ketone such as acetone; a nitrile solvent such as acetonitrile; an alcohol solvent such as methanol or ethanol; an acetic acid solvent; or a sulfuric acid solvent. Only one type of solvent may be used, or two or more types of solvents may be used in combination, for example. The acetic acid solvent and sulfuric acid solvent may be, for example, solvents obtained by dissolving acetic acid and sulfuric acid in water, respectively. They are solvents and, at the same time, also serve as a Lewis acid or a Brønsted acid, for example. The type of the solvent may be selected as appropriate depending on the solubility of the solutes (e.g., the radical generating catalyst of the present invention, the radical source, and the like) etc., for example.

In the radical production method of the present invention, the reaction may be performed by heating the mixture, as described above. Also, it is possible to produce radicals by performing the reaction by merely irradiating the mixture with light without heating or by merely allowing the mixture to stand still at room temperature without heating or light irradiation. The definition of the "room temperature" is not particularly limited, and is from 5°C to 35°C, for example. Since the radical production method of the present invention can be performed without heating, the cost for the heating with an electric furnace or the like is not necessary, which allows drastic reduction in cost for producing radicals, for example. Besides, since the radical production method of the present invention can be performed without heating, an unexpected runaway reaction caused by a radical chain reaction and accumulation of peroxides is prevented, which greatly improves the safety of the reaction and allows still further reduction in cost, for example. It is to be noted, however, that these descriptions are merely illustrative, and do not limit the present invention by any means.

The radical production method of the present invention may further include, for example, a light irradiation step of irradiating the mixture obtained in the mixing step with light. Then, as described above, radicals may be produced through a reaction caused by the light irradiation. The wavelength of the irradiation light is as described above, for example. A light source is not particularly limited. For example, by using visible light contained in natural light such as sunlight, excitation can be performed easily. Also, for example, instead of or in addition to the natural light, a light source such as a xenon lamp, a halogen lamp, a fluorescent lamp, or a mercury lamp may be used when necessary or may not be used. Further, a filter that cuts wavelengths other than a necessary wavelength may be used when necessary or may not be used.

In the radical production method of the present invention, the radical source may include, for example, at least one selected from the group consisting of halogen ions, hypohalite ions, halite ions, halate ions, and perhalate ions. It is particularly preferable that the radical source contains, for example, chlorite ions. The radical source may include, for example, an oxoacid or a salt thereof (e.g., a halogen oxoacid or a salt thereof). Examples of the oxoacid include boric acid, carbonic acid, orthocarbonic acid, carboxylic acid, silicic acid, nitrous acid, nitric acid, phosphorous acid, phosphoric acid, arsenic acid, sulfurous acid, sulfuric acid, sulfonic acid, sulfinic acid, chromic acid, dichromic acid, and permanganic acid. Examples of the halogen oxoacid include: chlorine oxoacids such as hypochlorous acid, chlorous acid, chloric acid, and perchloric acid; bromine oxoacids such as hypobromous acid, bromous acid, bromic acid, and perbromic acid; and iodine oxoacids such as hypoiodous acid, iodous acid, iodic acid, and periodic acid.

The radical source may be selected as appropriate depending on the use thereof, with consideration given to the intensity of reactivity of a radical species, etc., for example. For example, hypochlorous acid exhibiting high reactivity or chlorous acid exhibiting somewhat lower reactivity than the hypochlorous acid and allowing a reaction to be controlled more easily may be used as appropriate depending on the intended use.

In the radical production method of the present invention, the radical source may include an electron donor-acceptor linked molecule, for example. The electron donor-acceptor linked molecule is not particularly limited. For example, the electron donor-acceptor linked molecule may be such that an electron donor moiety is composed of one or more electron donor groups and an electron acceptor moiety is composed of one or more aromatic cations. In this case, the aromatic cation may be either a monocyclic ring or a condensed ring, and the aromatic ring may or may not include a heteroatom and may or may not have a substituent other than the electron donor group. Furthermore, an aromatic ring that forms the aromatic cation may be, for example, any of a 5- to 26-membered ring, although the number of atoms constituting the ring is not particularly limited.

The aromatic ring that forms the aromatic cation preferably is at least one selected from the group consisting of a pyrrolinium ring, a pyridinium ring, a quinolinium ring, an isoquinolinium ring, an acridinium ring, a 3,4-benzoquinolinium ring, a 5,6-benzoquinolinium ring, a 6,7-benzoquinolinium ring, a 7,8-benzoquinolinium ring, a 3,4-benzoisoquinolinium ring, a 5,6-benzoisoquinolinium ring, a 6,7-benzoisoquinolinium ring, a 7,8-benzoisoquinolinium ring, and rings obtained by substitution of at least one carbon atom of these rings with a heteroatom. For example, when the aromatic ring is a macrocyclic (having many π electrons) aromatic cation such as an acridinium ring, a benzoquinolinium ring, or a benzoisoquinolinium ring, for example, visible light excitation becomes possible if the absorption band shifts toward the longer wavelength side so as to be in the visible region.

The electron donor group preferably is at least one selected from the group consisting of a hydrogen atom, alkyl groups, and aromatic rings. In this case, the aromatic ring further may have one or more substituents on the ring, and when a plurality of substituents are present, they may be the same or different from each other. When a plurality of electron donor groups are present, they may be the same or different from each other. Furthermore, in the electron donor group in this case, it is more preferable that the alkyl group is a straight-chain or branched alkyl group having 1 to 6 carbon atoms. Furthermore, in the electron donor group, it is more preferable that the aromatic ring is at least one selected from the group consisting of a benzene ring, a naphthalene ring, an anthracene ring, a phenanthrene ring, a pyridine ring, a thiophene ring, and a pyrene ring. In the electron donor group, it is more preferable that the substituent on the aromatic ring is at least one selected from the group consisting of alkyl groups, alkoxy groups, primary to tertiary amines, carboxylic acids, and carboxylate esters. In Ar, it is more preferable that the substituent on the aromatic ring is at least one selected from the group consisting of straight-chain or branched alkyl groups having 1 to 6 carbon atoms, straight-chain or branched alkoxy groups having 1 to 6 carbon atoms, primary to tertiary amines, carboxylic acids, and carboxylate esters. In the substituent on the aromatic ring, a "carboxylic acid" refers to a carboxyl group or a group having a carboxyl group added to its end (e.g., a carboxyalkyl group), and a "carboxylate ester" refers to a carboxylate ester group such as an alkoxycarbonyl group or a phenoxycarbonyl group, or an acyloxy group. An alkyl group in the carboxyalkyl group preferably is a straight-chain or branched alkyl group having 1 to 6 carbon atoms, for example. An alkoxy group in the alkoxycarbonyl group preferably is a straight-chain or branched alkoxy group having 1 to 6 carbon atoms, for example.

It is still more preferable that the electron donor group is at least one selected from the group consisting of a phenyl group, an o-tolyl group, an m-tolyl group, a p-tolyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a mesityl group (2,4,6-trimethylphenyl group), and a 3,4,5-trimethylphenyl group. Among these, a mesityl group is particularly preferable from the viewpoint of the lifetime of the electron-transfer state (charge-separated state) and the like. Although the reasons why a mesityl group can bring about a particularly excellent effect is not clear, they are speculated to be as follows, for example: two methyl groups are present in the ortho position, so that the benzene rings of the mesityl groups easily cross at right angles to the aromatic ring of the aromatic cation; and hyperconjugation does not occur very often inside the mesityl group. This, however, merely is an example of a presumable mechanism, and does not limit the present invention by any means.

The electron donor-acceptor linked molecule preferably is at least one selected from the group consisting of: nitrogen-containing aromatic cation derivatives represented by the following formulae (A-1) to (A-8); quinolinium ion derivatives represented by the following formula (I); stereoisomers and tautomers thereof; and salts thereof, from the viewpoints of the lifetime, oxidizing power, reducing power, and the like of the electron-transfer state (charge-separated state).

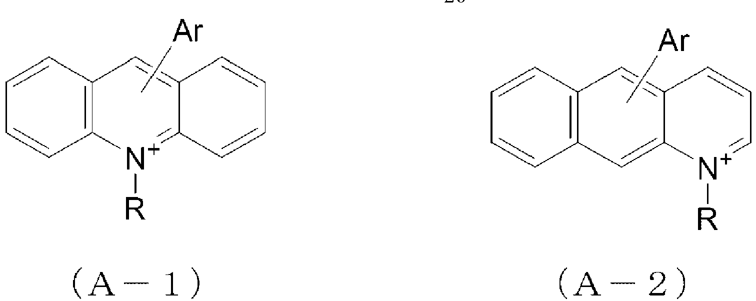

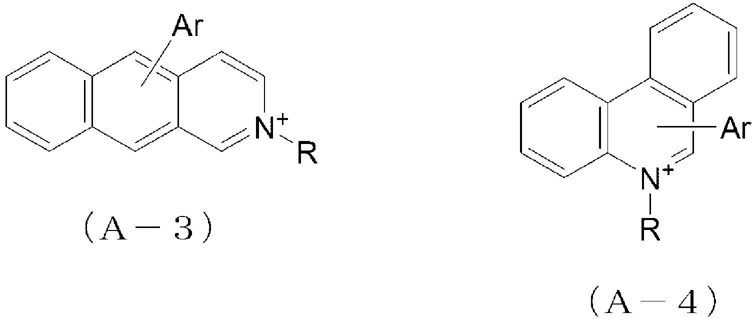

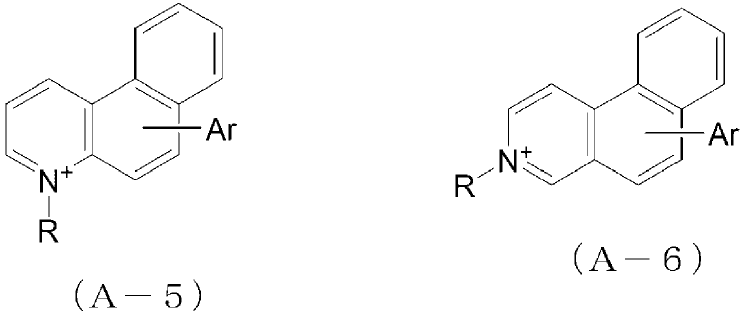

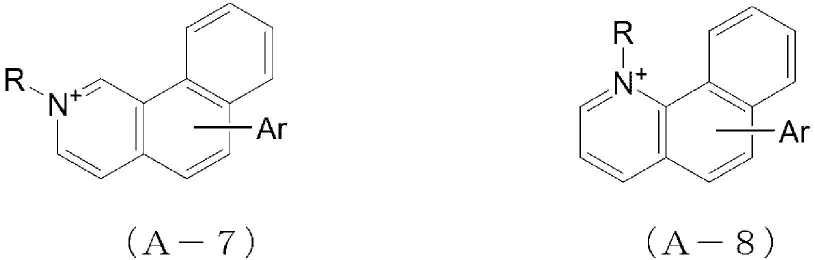

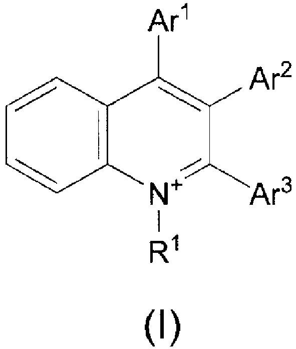

In the formulae (A-1) to (A-8), R is a hydrogen atom or any substituent, Ar is the electron donor group, and the number of Ars may be one or more, and when a plurality of Ars are present, they may be the same or different from each other, and the nitrogen-containing aromatic ring that forms a nitrogen-containing aromatic cation may or may not have at least one substituent other than R and Ar. In the formula (I), R¹ is a hydrogen atom or any substituent, Ar¹ to Ar³ are each a hydrogen atom or the electron donor group and may be the same or different from each other, and at least one of Ar¹ to Ar³ is the electron donor group.

In the formulae (A-1) to (A-8), R preferably is a hydrogen atom, an alkyl group, a benzyl group, a carboxyalkyl group (an alkyl group with a carboxyl group added to its end), an aminoalkyl group (an alkyl group having an amino group added to its end), or a polyether chain. More preferably, R is a hydrogen atom, a straight-chain or branched alkyl group having 1 to 6 carbon atoms, a benzyl group, a straight-chain or branched alkyl group having 1 to 6 carbon atoms with a carboxyl group added to its end, a straight-chain or branched alkyl group having 1 to 6 carbon atoms with an amino group added to its end, or a polyethylene glycol (PEG) chain. The PEG chain is an example of the polyether chain. The type of the polyether chain is not limited thereto, and the polyether chain may be of any type. In R, the degree of polymerization of the polyether chain is not particularly limited, and is, for example, 1 to 100, preferably 1 to 50, and more preferably 1 to 10. In the case where the polyether chain is a PEG chain, the degree of polymerization is not particularly limited, and is, for example, 1 to 100, preferably 1 to 50, and more preferably 1 to 10.

It is more preferable that the electron donor-acceptor linked molecule is at least one selected from the group consisting of 9-substituted acridinium ions represented by the following formula (A-9), tautomers thereof, and stereoisomers thereof.

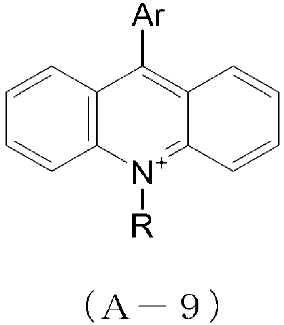

In the formula (A-9), R and Ar are the same as those in the formula (A-1).

Furthermore, it is particularly preferable that the electron donor-acceptor linked molecule is a 9-mesityl-10-methylacridinium ion represented by the following formula (A-10). By photoexcitation of this 9-mesityl-10-methylacridinium ion, it is possible to generate a long-lived electron-transfer state (charge-separated state) having a high oxidizing power and a high reducing power. As excitation light for the photoexcitation, it is possible to use visible light, for example.

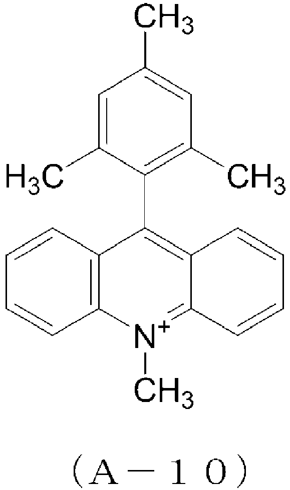

Examples of the 9-substituted acridinium ion represented by the formula (A-9) further include compounds (A-101) to (A-116) shown in the following table, in addition to the one represented by the above formula (A-10).

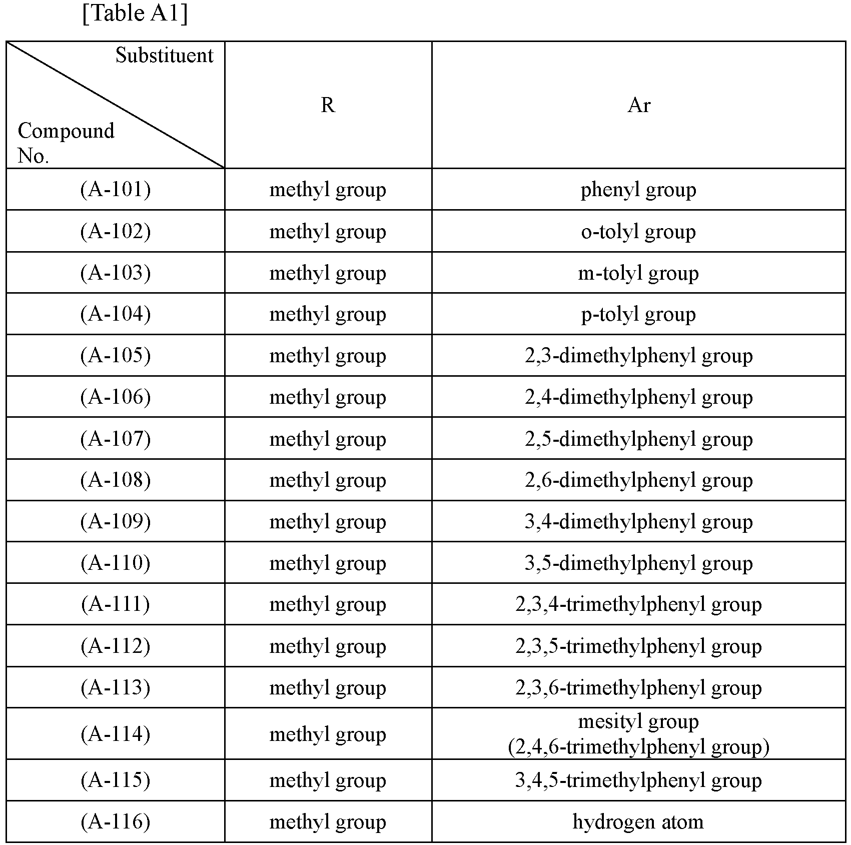

In the quinolinium ion derivative represented by the formula (I), R¹ preferably is a hydrogen atom, an alkyl group, a benzyl group, a carboxyalkyl group (an alkyl group with a carboxyl group added to its end), an aminoalkyl group (an alkyl group having an amino group added to its end), or a polyether chain, for example. More preferably, R¹ is a hydrogen atom, a straight-chain or branched alkyl group having 1 to 6 carbon atoms, a benzyl group, a straight-chain or branched alkyl group having 1 to 6 carbon atoms with a carboxyl group added to its end, a straight-chain or branched alkyl group having 1 to 6 carbon atoms with an amino group added to its end, or a polyethylene glycol (PEG) chain, for example. The PEG chain is an example of the polyether chain. The type of the polyether chain is not limited thereto, and the polyether chain may be of any type. In R¹, the degree of polymerization of the polyether chain is not particularly limited, and is, for example, 1 to 100, preferably 1 to 50, and more preferably 1 to 10. In the case where the polyether chain is a PEG chain, the degree of polymerization is not particularly limited, and is, for example, 1 to 100, preferably 1 to 50, and more preferably 1 to 10. Furthermore, Ar¹ to Ar³ preferably are each a hydrogen atom, an alkyl group, or an aromatic ring, for example, and the alkyl group more preferably is a straight-chain or branched alkyl group having 1 to 6 carbon atoms. In Ar¹ to Ar³, the aromatic ring further may have one or more substituents on the ring, and when a plurality of substituents are present, they may be the same or different from each other.

In Ar¹ to Ar³ in the formula (I), the aromatic ring more preferably is a benzene ring, a naphthalene ring, an anthracene ring, a phenanthrene ring, a pyridine ring, a thiophene ring, or a pyrene ring, for example. Furthermore, in Ar¹ to Ar³, the substituent on the aromatic ring more preferably is an alkyl group, an alkoxy group, any one of primary to tertiary amines, a carboxylic acid, or a carboxylate ester. Still more preferably, the substituent on the aromatic ring is a straight-chain or branched alkyl group having 1 to 6 carbon atoms, a straight-chain or branched alkoxy group having 1 to 6 carbon atoms, any one of primary to tertiary amines, a carboxylic acid, or a carboxylate ester. The secondary amine is not particularly limited, and preferably is an alkylamino group, and more preferably is a straight-chain or branched alkylamino group having 1 to 6 carbon atoms, for example. The tertiary amine is not particularly limited, and preferably is a dialkylamino group, and more preferably is a dialkylamino group with a straight-chain or branched alkyl group having 1 to 6 carbon atoms, for example.

In the substituent on the aromatic ring in Ar¹ to Ar³, a "carboxylic acid" refers to a carboxyl group or a group having a carboxyl group added to its end (e.g., a carboxyalkyl group), and a "carboxylate ester" refers to a carboxylate ester group such as an alkoxycarbonyl group or a phenoxycarbonyl group, or an acyloxy group. An alkyl group in the carboxyalkyl group preferably is a straight-chain or branched alkyl group having 1 to 6 carbon atoms, for example. An alkoxy group in the alkoxycarbonyl group preferably is a straight-chain or branched alkoxy group having 1 to 6 carbon atoms, for example.

Among the quinolinium ion derivatives represented by the formula (I), for example, quinolinium ion derivatives represented by the following formulae 1 to 5 are particularly preferable in terms of a long lifetime, a high oxidizing power, a high reducing power, and the like of the charge-separated state.

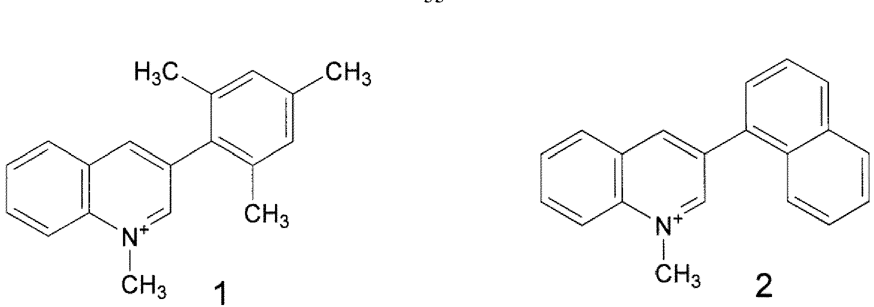

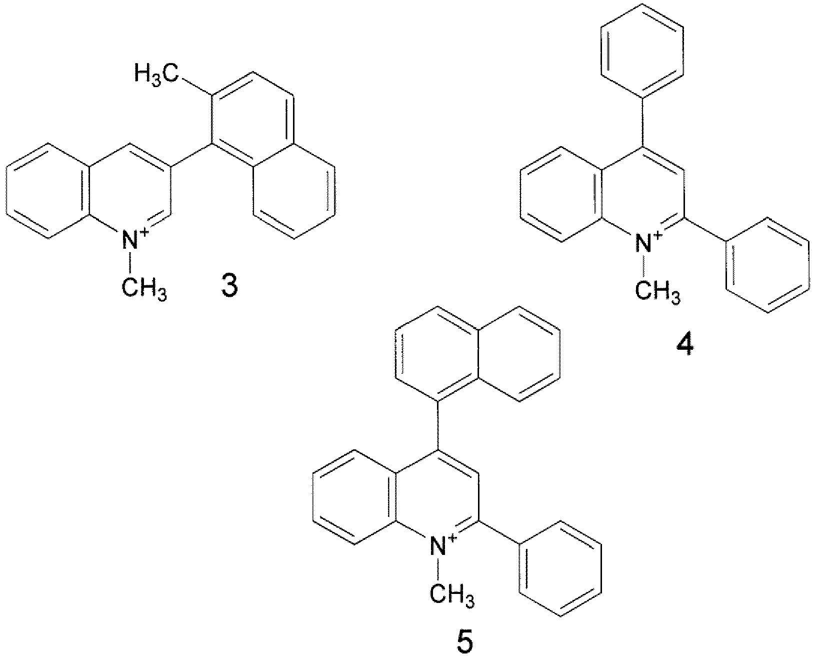

In addition to the above compounds 1 to 5, compounds 6 to 36 shown in Tables 1 and 2 below also are particularly preferable, for example. Tables 2 and 3 show the structures of the compounds 6 to 36 by indicating the combination of R¹ and Ar¹ to Ar³ in the formula (I). Those skilled in the art can produce and use the compounds 6 to 36 easily according to the production and use of the compounds 1 to 5 with reference to examples to be described below, without undue trial and error, complicated and advanced experiments, etc.

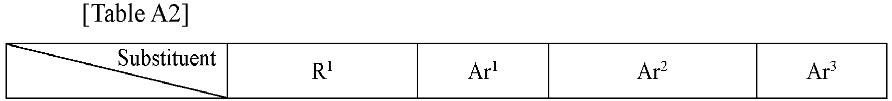

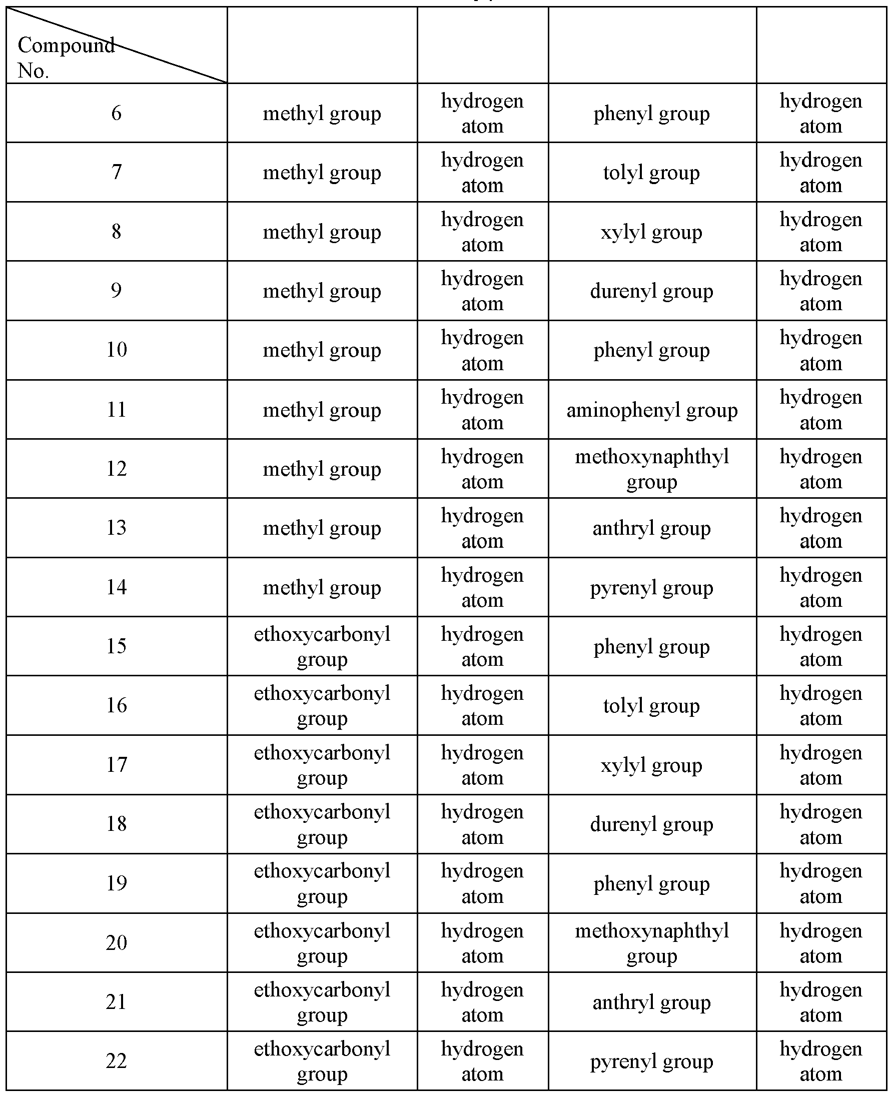

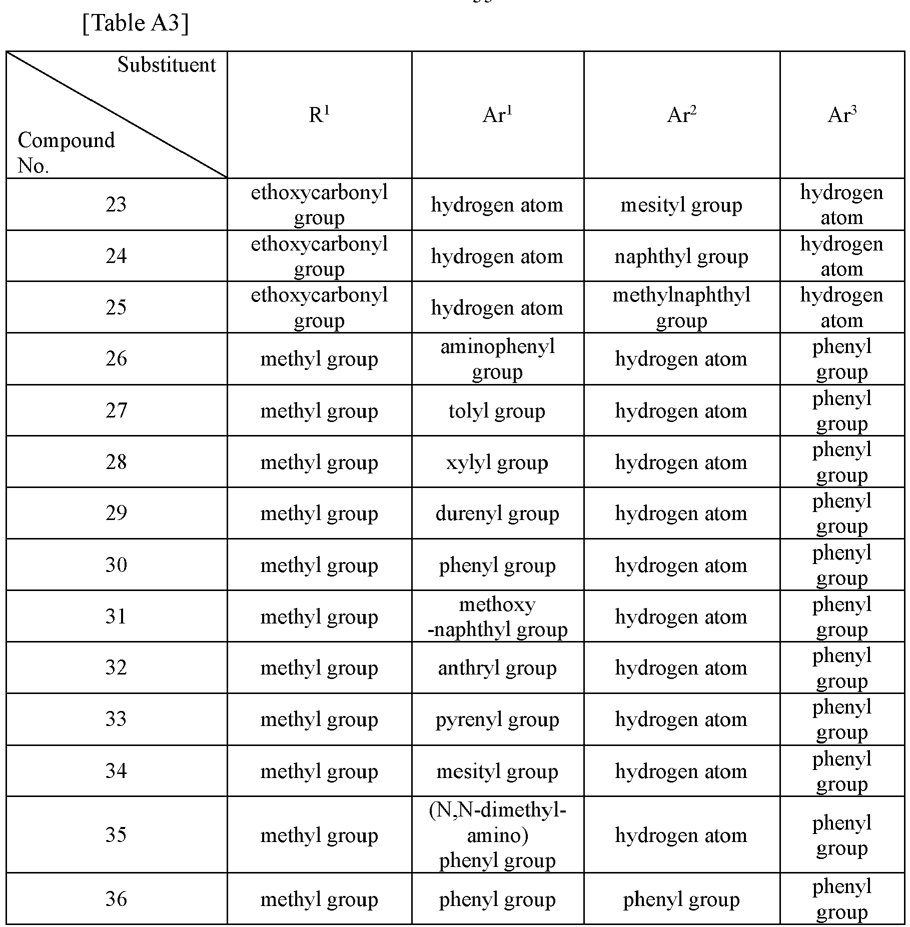

The electron donor-acceptor linked molecule may be a commercially available product or may be produced (synthesized) as appropriate. When the electron donor-acceptor linked molecule is produced, the method for producing it is not particularly limited, and it can be produced as appropriate by a known production method or with reference to a known production method, for example. Specifically, the production method described in Japanese Patent No. 5213142 may be used, for example.

In the present invention, when the compound (e.g., the ammonium, the amino acid, the peptide, the phospholipid, the electron donor-acceptor linked molecule, etc.) has isomers such as tautomers and stereoisomers (e.g., a geometric isomer, a conformer, and an optical isomer), any isomer can be used in the present invention, unless otherwise stated. When the compound (e.g., the electron donor-acceptor linked molecule) can form a salt, the salt also can be used in the present invention, unless otherwise stated. The salt may be an acid addition salt, or may be a base addition salt. Moreover, an acid that forms the acid addition salt may be either an inorganic acid or an organic acid, and a base that forms the base addition salt may be either an inorganic base or an organic base. The inorganic acid is not particularly limited, and examples thereof include sulfuric acid, phosphoric acid, hydrofluoric acid, hydrochloric acid, hydrobromic acid, hydroiodic acid, hypofluorous acid, hypochlorous acid, hypobromous acid, hypoiodous acid, fluorous acid, chlorous acid, bromous acid, iodous acid, fluorine acid, chloric acid, bromic acid, iodic acid, perfluoric acid, perchloric acid, perbromic acid, and periodic acid. The organic acid also is not particularly limited, and examples thereof include p-toluenesulfonic acid, methanesulfonic acid, oxalic acid, p-bromobenzenesulfonic acid, carbonic acid, succinic acid, citric acid, benzoic acid, and acetic acid. The inorganic base is not particularly limited, and examples thereof include ammonium hydroxides, alkali metal hydroxides, alkaline-earth metal hydroxides, carbonates, and hydrogencarbonates. More specifically, the inorganic base may be, for example, sodium hydroxide, potassium hydroxide, potassium carbonate, sodium carbonate, sodium hydrogencarbonate, potassium hydrogencarbonate, calcium hydroxide, and calcium carbonate. The organic base also is not particularly limited, and examples thereof include ethanolamine, triethylamine, and tris(hydroxymethyl)aminomethane. The method for producing these salts also is not particularly limited. For example, they can be produced by adding an acid or a base such as described above to the compound as appropriate by a known method.

Moreover, in the present invention, a chain substituent (e.g., an alkyl group, hydrocarbon groups such as an unsaturated aliphatic hydrocarbon group, etc.) may be straight-chain or branched, unless otherwise stated, and the number of carbons thereof is not particularly limited, and may be, for example, 1 to 40, 1 to 32, 1 to 24, 1 to 18, 1 to 12, 1 to 6, or 1 to 2 (at least 2 in the case of an unsaturated hydrocarbon group). Furthermore, in the present invention, as to a cyclic group (e.g., an aryl group, a heteroaryl group, etc.), the number of ring members (the number of atoms that compose a ring) is not particularly limited and may be, for example, 5 to 32, 5 to 24, 6 to 18, 6 to 12, or 6 to 10. When a substituent or the like has isomers, any isomer can be used, unless otherwise stated. For example, in the case of simply describing as a "naphthyl group", it may be a 1-naphthyl group or a 2-naphthyl group.

### [3. Oxidation reaction product production method]

As described above, the oxidation reaction product production method of the present invention is a method for producing an oxidation reaction product by oxidizing a substance to be oxidized, characterized in that it includes: a radical production step of producing a radical by the radical production method according to the present invention; and an oxidation reaction step of reacting the substance to be oxidized with an oxidizing agent by action of the radical, thereby generating the oxidation reaction product.

The method for carrying out the oxidation reaction product production method of the present invention is not particularly limited. For example, in the mixing step, not only the radical generating catalyst of the present invention and the radical source but also the substance to be oxidized and the oxidizing agent further may be mixed together. At this time, as described above, it is preferable to further mix a solvent. Then, in the radical production step, the substance to be oxidized may be reacted with the oxidizing agent by action of the produced radicals, thereby generating the oxidation reaction product. That is, the oxidation reaction step may be performed at the same time with the radical production step in the same reaction system. In this case, the concentrations of the substance to be oxidized and the oxidizing agent are not particularly limited, for example, the reaction mol/l relative to the solvent is not particularly limited, and can be set as appropriate. In addition, for example, the concentration of the substance to be oxidized is preferably as high as possible in order to increase the reaction rate, and the concentration of the oxidizing agent is preferably not too high in order to allow the oxidation reaction to proceed rapidly. However, this description is merely an example and does not limit the present invention.

In the oxidation reaction product production method of the present invention, the radical also may serve as the oxidizing agent. For example, the radical-generating agent may be an oxoacid, and a radical generated from the oxoacid may be an oxidizing agent. As an illustrative example, the radical-generating agent may be a chlorous acid ion ClO₂⁻, and the oxidation reaction product may be produced by oxidizing the substance to be oxidized with the radical ClO₂˙ generated from the chlorous acid ion ClO₂⁻ as the oxidizing agent.

Alternatively, the radicals and the oxidizing agent may be different substances, for example. For example, the radical-generating agent may be the electron donor-acceptor linked molecule, the oxidizing agent may be an oxygen molecule O₂, and the oxidation reaction product may be produced by oxidizing the substance to be oxidized by action of the radical of the electron donor-acceptor linked molecule and the oxygen molecule.

The substance to be oxidized is not particularly limited, and may be either an organic compound or an inorganic substance, for example. For example, the substance to be oxidized may be triphenylphosphine Ph₃P, and the oxidation reaction product may be triphenylphosphine oxide Ph₃P=O. Also, for example, the substance to be oxidized may be olefin, and the oxidation reaction product may contain epoxide and/or diol.

The substance to be oxidized may be an aromatic compound (may be referred to as "raw material aromatic compound" hereinafter), for example. In the present invention, the raw material aromatic compound is not particularly limited. It is preferable that an electron donor group is bound to an aromatic ring of the raw material aromatic compound, because, for example, this allows an oxidation reaction (including an oxidative substitution reaction) of the raw material aromatic compound to proceed more easily. The number of the electron donor groups may be one or more, and the electron donor group with a strong electron-donating property is preferable. More specifically, it is more preferable that the raw material aromatic compound is such that at least one substituent selected from the group consisting of -OR¹⁰⁰, -NR²⁰⁰₂, and AR¹⁰⁰ is covalently bound to the aromatic ring. R¹⁰⁰ is a hydrogen atom or any substituent, and when a plurality of R¹⁰⁰s are present, they may be the same or different from each other. R²⁰⁰s are each a hydrogen atom or any substituent, and they may be the same or different from each other. AR¹⁰⁰ is an aryl group, and when a plurality of AR¹⁰⁰s are present, they may be the same or different from each other.

AR¹⁰⁰ may be a group derived from any aromatic ring such as a benzene ring, a naphthalene ring, an anthracene ring, a phenanthrene ring, a pyridine ring, a thiophene ring, or a pyrene ring. The aromatic ring further may have one or more substituents thereon, and when a plurality of substituents are present, they may be the same or different from each other. AR¹⁰⁰ may be a phenyl group, for example.

R¹⁰⁰ preferably is at least one selected from the group consisting of a hydrogen atom, alkyl groups, aryl groups, and acyl groups. The alkyl group preferably is a straight-chain or branched alkyl group having 1 to 6 carbon atoms, and a methyl group is particularly preferable. The acyl group preferably is a straight-chain or branched acyl group having 1 to 6 carbon atoms. The aryl group is the same as AR¹⁰⁰, for example, and is a phenyl group, for example.

R²⁰⁰ preferably is at least one selected from the group consisting of a hydrogen atom, alkyl groups, aryl groups, and acyl groups. The alkyl group preferably is a straight-chain or branched alkyl group having 1 to 6 carbon atoms, and a methyl group is particularly preferable. The acyl group preferably is a straight-chain or branched acyl group having 1 to 6 carbon atoms. The aryl group is the same as AR¹⁰⁰, for example, and is a phenyl group, for example.
As -NR²⁰⁰₂, an amino group substituted with an electron donor substituent, such as a dimethylamino group or a diphenylamino group, is preferable because of its particularly high electron-donating properties.

Furthermore, the raw material aromatic compound may be such that, for example, a substituent such as an alkyl group is covalently bound to the aromatic ring, and the substituent may be oxidized in the step of generating the oxidation reaction product. For example, the oxidizing agent may contain an oxygen atom, the raw material aromatic compound may contain a methylene group (-CH2-) covalently bound to the aromatic ring, and in the step of generating the oxidation reaction product, the methylene group (-CH2-) may be converted to a carbonyl group (-CO-) by oxidation. In this case, an atom or atomic group that is bound to the methylene group and the carbonyl group is not particularly limited, and examples thereof include a hydrogen atom, alkyl groups, and aryl groups. The alkyl group preferably is a straight-chain or branched alkyl group having 1 to 6 carbon atoms. The alkyl group and aryl group may further be substituted with one or more substituents. When they are substituted with a plurality of substituents, the substituents may be the same or different from each other. For example, the methylene group becomes a methyl group (-CH₃) when hydrogen is bound thereto, and it becomes a formyl group (-CHO) after oxidation. The methylene group becomes an ethyl group (-CH₂CH₃) when a methyl group is bound thereto, and it becomes an acetyl group (-COCH₃) after oxidation. The methylene group becomes a benzyl group (-CH₂Ph) when a phenyl group is bound thereto, and it becomes a benzoyl group (-COPh) after oxidation. Alternatively, for example, the substituent (before being oxidized) covalently bound to an aromatic ring may be a formyl group (-CHO), and may become a carboxy group (-COOH) after oxidation.

In the oxidation reaction product production method of the present invention, for example, the substance to be oxidized may be an olefin, for example, and the olefin may be an aromatic olefin or an aliphatic olefin, for example. The olefin may be an olefin represented by the following chemical formula (A1), for example. Furthermore, the oxidation reaction product of the olefin is not particularly limited, and, for example, may contain at least one of an epoxide and a diol as in the following scheme A. In each of the following chemical formulae (A1), (A2), and (A3), Rs each may be a hydrogen atom or any substituent, and Rs may be the same or different from each other. The substituent may be, for example, an alkyl group, an unsaturated aliphatic hydrocarbon group, an aryl group, a heteroaryl group, a halogen, a hydroxy group (-OH), a mercapto group (-SH), or an alkylthio group (-SR and R are each an alkyl group), and the substituent may or may not be substituted with another substituent. The alkyl group preferably is a straight-chain or branched alkyl group having 1 to 6 carbon atoms. Furthermore, the olefin, which is a substance to be oxidized, may be an olefin containing one olefin bond (carbon-carbon double bond) or an olefin containing two or more olefin bonds.

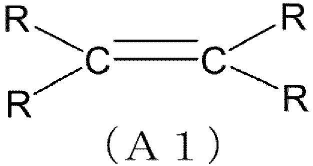

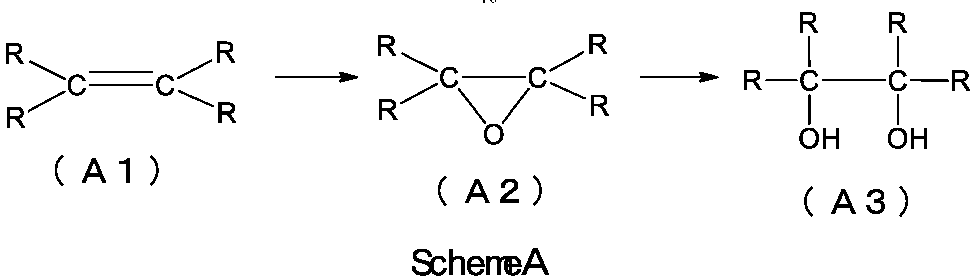

The olefin may be, for example, an aromatic olefin as described above. That is, for example, in the chemical formula (A1), at least one of Rs may be an aromatic ring (an aryl group or a heteroaryl group). The aromatic olefin may be such that at least one substituent selected from the group consisting of -OR¹⁰⁰, -NR²⁰⁰₂, and AR¹⁰⁰ is covalently bound to the aromatic ring, for example, as described as the raw material aromatic compound.

In the olefin oxidation reaction product production method of the present invention, the olefin may be at least one selected from the group consisting of ethylene, propylene, styrene, and butadiene. Furthermore, the oxidation reaction product may be, as described above, at least one of an epoxide and a diol, for example. The examples thereof are shown in the following schemes A1 to A3. It is to be noted, however, that the schemes A1 to A3 are merely illustrative examples, and in the present invention, the oxidation reactions of ethylene, propylene and styrene are not limited thereto.

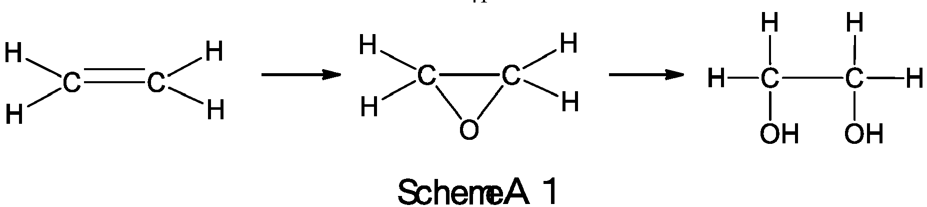

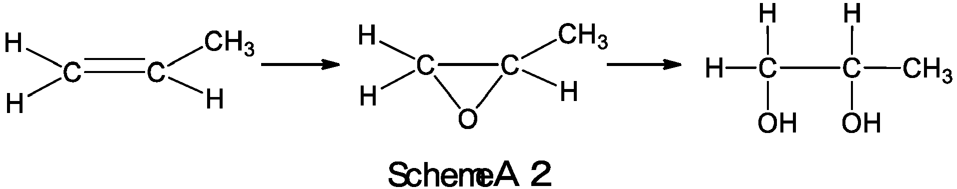

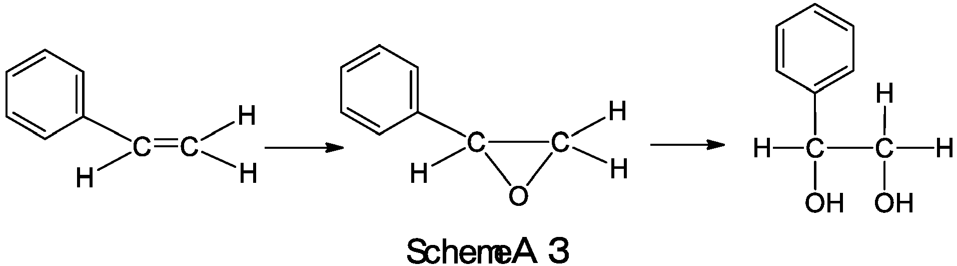

In oxidization of an olefin (for example, the olefin (A1) in the scheme A), for example, by adjusting the concentration of at least one of: the Lewis acid and/or Brønsted acid; the radical source; and the oxidizing agent, oxidation reaction products can be selectively generated. For example, an epoxide is prone to be obtained when the concentrations are low with respect to the substance to be oxidized and a diol is prone to be obtained when the concentrations are high with respect to the substance to be oxidized, although the present invention is not limited thereto. Furthermore, for example, instead of changing the concentrations, by changing the intensity of the reactivity of a radical species generated from the radical source, oxidation reaction products can be selectively generated. For example, an epoxide is prone to be obtained with a radical species having low reactivity and a diol is prone to be obtained with a radical species having high reactivity, although the present invention is not limited thereto. It is to be noted that the use of the oxidation reaction product is not particularly limited. For example, when the substance to be oxidized (raw material aromatic compound) is styrene, styrene oxide can be utilized as an adhesive agent and a diol can be utilized as a perfume. As described above, the epoxide and the diol are in demand for different uses. Thus, the selective production of the epoxide and the diol by controlling the reaction condition allows the present invention to be applied to further wider uses.

### [4. Drug]

As described above, the drug according to the present invention is characterized in that it includes: a radical generating catalyst; and a radical source, wherein the radical generating catalyst is the radical generating catalyst of the present invention. In the drug of the present invention, other configurations or conditions are not particularly limited. The ammonium also may serve as the substance having Lewis acidic properties and/or Brønsted acidic properties.

According to the present invention, it is possible to provide a drug that is highly safe and has a high sterilizing effect.

The drug of the present invention can be used, for example, as a drug for use in agriculture and livestock industry. Hereinafter, a drug of the present invention which can be used as a drug for agriculture and livestock industry may be referred to as a "drug for use in agriculture and livestock industry of the present invention".

The drug for use in agriculture and livestock industry according to the present invention is highly safe and has a high sterilizing effect. Thus, the drug for use in agriculture and livestock industry according to the present invention can be used widely for sterilization, deodorization, etc. in agriculture and livestock industry, for example. Further, the drug for use in agriculture and livestock industry according to the present invention is less liable to cause corrosion, for example. Even when the drug is applied to metals, corrosion of the metals is less liable to occur. Thus, the drug for use in agriculture and livestock industry according to the present invention can be used for a target object containing a metal, for example.

The drug of the present invention may be, for example, a radical generating catalyst that catalyzes radical generation from at least one radical source selected from the group consisting of halogenous acids, halite ions and halites.

In the drug of the present invention, for example, the Lewis acidity of the radical generating catalyst of the present invention is not particularly limited, and is, as described above, for example, 0.4 eV or more, 0.5 eV or more, or 0.6 eV or more, and is, for example, 20 eV or less.

The drug of the present invention may be, for example, liquid, solid or semi-solid. The drug of the present invention may also be acidic, non-acidic, basic, non-basic, neutral or non-neutral.

The drug of the present invention may be a liquid drug that includes a radical generating catalyst; and at least one selected from the group consisting of halogenous acids, halite ions, and halites. The radical generating catalyst of the present invention may be a radical generating catalyst that catalyzes radical generation from at least one radical source selected from the group consisting of halogenous acids, halite ions, and halites. The radical generating catalyst may have a Lewis acidity of 0.4 eV or more. The drug may be non-acidic.

In the drug of the present invention, the radical source may be selected as appropriate depending on the use thereof, with consideration given to the intensity of reactivity of a radical species, etc., for example. For example, hypochlorous acid exhibiting high reactivity or chlorous acid exhibiting somewhat lower reactivity than the hypochlorous acid and allowing a reaction to be controlled more easily may be used as appropriate depending on the intended use.

In the drug of the present invention, the content of the radical source (e.g., oxoacids and the like) is not particularly limited, and is, for example, 0.01 mass ppm or more, 0.05 mass ppm or more, 0.1 mass ppm or more, 1500 mass ppm or less, 1000 mass ppm or less, or 250 mass ppm or less. The amount of the radical source (e.g., oxoacids and the like) mixed in a drug preferably is from 0.01 to 1500 mass ppm, more preferably from 0.05 to 1000 mass ppm, and still more preferably from 0.1 to 250 mass ppm. The concentration of the radical source preferably is low, because it is considered that the safety level increases as the concentration becomes lower. However, if the concentration of the radical source is too low, the sterilizing effect or the like may not be obtained. From the viewpoint of the sterilizing effect and the like, the concentration of the radical source is not particularly limited, and preferably is set as high as possible.

In the drug of the present invention, the content of the radical generating catalyst (e.g., ammonium, a cationic surfactant, or the like) is not particularly limited, and is, for example, 0.01 mass ppm or more, 0.05 mass ppm or more, 0.1 mass ppm or more, 1500 mass ppm or less, 1000 mass ppm or less, 500 mass ppm or less, or 250 mass ppm or less. The amount of the radical generating catalyst (e.g., ammonium, a cationic surfactant, or the like) mixed in the drug preferably is from 0.01 to 1500 mass ppm, more preferably from 0.05 to 1000 mass ppm, still more preferably from 0.05 to 500 mass ppm, and yet more preferably from 0.1 to 250 mass ppm. The concentration of the radical generating catalyst preferably is low, because it is considered that the safety level increases as the concentration becomes lower. However, if the concentration of the radical generating catalyst is too low, the sterilizing effect or the like may not be obtained. From the viewpoint of preventing the risk that the sterilizing effect or the like may not be obtained owing to micelle formation, it is preferable that the concentration of the radical generating catalyst is equal to or lower than the critical micelle concentration.

In the drug of the present invention, the concentration ratio of the radical source and the radical generating catalyst (radical source/radical generating catalyst) in the drug is not particularly limited, and can be set as appropriate.

The drug of the present invention further may contain one or more other substances. Examples of the other substances include water, organic solvents, pH adjusters, and buffers. One type of them may be used, or two or more types of them may be used in combination (the same applies hereinafter). The water is not particularly limited, and preferably is purified water, ion-exchange water, or pure water, for example.

The drug of the present invention preferably contains water and/or an organic solvent. In the present invention, the "solvent" may or may not dissolve the radical generating catalyst of the present invention, the radical source, and the like. For example, after the mixing step, the radical generating catalyst of the present invention and the radical source may each be in a state of being dissolved in the solvent, or may each be in a state of being dispersed or precipitated in the solvent. In the drug of the present invention, it is preferable to use water as a solvent for the radical generating catalyst of the present invention and the radical source from the viewpoint of safety, cost, etc. The organic solvent may be, for example, ketone such as acetone, a nitrile solvent such as acetonitrile, or an alcohol solvent such as ethanol. One type of solvent may be used alone, or two or more types of solvents may be used in combination. The type of the solvent may be selected as appropriate depending on the solubility of the solutes (e.g., the radical generating catalyst of the present invention, the radical source, and the like) etc., for example.

The pH of the drug of the present invention is not particularly limited, and may be, for example, 4.0 or more, 4.5 or more, 5.0 or more, 5.5 or more, 6.0 or more, 6.5 or more, 7.0 or more, or 7.5 or more. The pH of the drug of the present invention may be, for example, 11.5 or less, 11.0 or less, 10.5 or less, 10.0 or less, 9.5 or less, 9.0 or less, 8.5 or less, 8.0 or less, or 7.5 or less.

The drug of the present invention can be produced by, for example, mixing the radical source and the radical generating catalyst, and optionally the water and/or organic solvent when necessary. For example, the drug of the present invention can be obtained in manners described in the following examples. It is to be noted, however, that the method for producing the drug of the present invention is not limited thereto. As described above, the drug further may contain one or more substances other than the radical source and the radical generating catalyst.

The drug for use in agriculture and livestock industry according to the present invention preferably contains water, for example. However, the drug may not necessarily contain water. The amount of the water mixed (the proportion of the water) in the drug for use in agriculture and livestock industry is not particularly limited. The proportion of the water may be the balance of the drug excluding the other components, for example. The drug for use in agriculture and livestock industry further may or may not contain, as the other substance(s), the pH adjuster, a buffer, and/or the like, for example.

The method of using the drug of the present invention is not particularly limited. For example, the drug of the present invention can be used in the same manners as those for conventional bactericides and the like. Specifically, the drug of the present invention may be sprayed on or applied to a target object, for example. Specifically, for example, when the drug is used for deodorization of a space, the drug can be sprayed in the space. When the drug is use for oral cavity, the drug may be in the form of an aqueous solution so that it can be used for gargling and oral rinsing. When the drug is used for disinfection of a decubitus ulcer, the drug can be applied to an affected area. For an affected area such as a self-destructive wound caused by cancer or a lesion caused by ringworm fungi or the like, absorbent cotton or gauze impregnated with the drug may be applied to the affected area. When the drug is used for hand washing, it may be in the form of an aqueous solution so that it can be rubbed onto hands. Medical instruments and the like can be washed with the drug by spraying the drug on them or immersing them in an aqueous solution containing the drug. Further, the drug may be applied to surroundings of beds, tables, doorknobs, and the like for the purpose of sterilization and prevention of infection with bacteria or the like.

### (Bactericide)

The drug of the present invention can be used as a bactericide, for example. Although various types of substances have been used as a bactericide conventionally, the sterilizing effects of these substances are not sufficient. Some of them can exhibit enhanced sterilizing effects by increasing their concentrations. However, this poses a problem in safety. A bactericide containing the drug of the present invention can exhibit a sufficient sterilizing effect while the concentration of the drug is low and is highly safe.

### (Bactericide for hand washing)

The drug of the present invention can be used as a bactericide for hand washing to disinfect hands, for example. The bactericide for hand washing containing the drug of the present invention can exhibit a sufficient sterilizing effect while the concentration of the drug is low and is highly safe.

### (Deodorizer)

The drug of the present invention can be used as a deodorizer, for example. Commonly used bactericides, such as ethanol, do not have a deodorizing effect. While chlorine dioxide has a deodorizing effect, the safety level thereof is very low. Some other commercially available products purport to have sterilizing and deodorizing effects. For example, there are commercially available products purporting to exhibit sterilizing and deodorizing effects by spraying them onto clothes directly or spraying them in rooms, toilets, cars, or the like. Such commercially available products typically contain a quaternary ammonium salt as a sterilizing component. However, a commonly used quaternary ammonium salt is not used in combination with a radical source (e.g., an oxoacid or the like). Thus, in many cases, a sufficient sterilizing effect cannot be obtained unless the quaternary ammonium salt is contained at a high concentration, and this causes a problem of surface tackiness or the like after use. Further, since the quaternary ammonium salt does not have a deodorizing effect, the commercially available products contain a deodorant component as an additional component. While cyclodextrin typically is used as the deodorant component, the cyclodextrin is incapable of decomposing components causing offensive odors. The cyclodextrin merely masks the components causing offensive odors and cannot eliminate the offensive odors themselves. In contrast, the deodorizer containing the drug of the present invention, which has the above-described action mechanism, has a high sterilizing effect, for example, and also, is capable of decomposing substances that cause offensive odors and thus has a high deodorizing effect, for example.

### (Antibacterial agent for metals)

The drug of the present invention can be used as an antibacterial agent for metals, for example. An antibacterial agent containing the drug of the present invention is highly safe, so that it can be sprayed on or applied to metal products in the kitchen, for example. Also, the antibacterial agent containing the drug of the present invention is less liable to cause corrosion. Thus, even when the antibacterial agent is used on metals, corrosion of the metals is less liable to occur.

### (Oral care agent)

The drug of the present invention can be used as an oral care agent, for example. An oral care agent containing the drug of the present invention is highly safe and thus suitable for use in the oral cavity.

### (Acne treatment agent)

The drug of the present invention can be used as an acne treatment agent, for example. An acne treatment agent containing the drug of the present invention is highly safe and thus can be applied to the face.

### (Disinfectant for decubitus ulcers)

The drug of the present invention can be used as a disinfectant for decubitus ulcers, for example. The disinfectant for decubitus ulcers containing the drug of the present invention is highly safe and thus can be applied to the body.

### (Fungicide)

The drug of the present invention can be used as a fungicide for disinfecting an affected part caused by infection with fungi such as ringworm fungi, for example.

### (Bactericide for water purification)

The drug of the present invention can kill bacteria, such as Legionella bacteria, breeding in water in swimming pools and baths, for example. Besides, it does not corrode metals and does not generate gas. Therefore, the bactericide for water purification containing the drug of the present invention can be used safely.

Further, the drug of the present invention can be applicable to, for example, the following uses. As described above, the drug of the present invention is highly safe and has a high sterilizing effect. The drug of the present invention can thereby exert, for example, a deodorizing action or the like. Therefore, the drug of the present invention is useful, for example, for improving quality of life (QOL).

As described above, the drug of the present invention can also be applied to the human body by taking advantage of its high safety. More specifically, it can be applicable, for example, to the following uses.
(1) Prevention, treatment, symptom relief, etc. of cystitis
(2) Prevention, treatment, symptom relief, etc. of candidiasis (including oral cleansing and vaginal washing)
(2) Eye drops and eyewashes (including application to disease such as sty)
(3) Ear/nasal lavage (otitis media, otitis externa, sinusitis, etc.)
(4) Oral cleansing and professional mechanical tooth cleaning (PMTC), including oral cleansing and PMTC to prevent aspiration pneumonitis, and oral cleansing and PMTC to improve quality of life (QOL) for the elderly or disabled.
(5) Peritoneal lavage (including treatment for peritonitis, peritoneal dissemination, etc.)
(6) Intestinal lavage
(7) Disinfection, washing, etc. of skin tissues
(8) Disinfection, washing, etc. of hands
(9) Disinfection, washing, and debridement of affected areas (including wounds)
(10) Treatment of dermatitis such as atopy (disinfection, washing, debridement, etc. of affected areas)
(11) Disinfection and washing of affected areas due to bacterial infections such as perionychia (paronychia) folliculitis, carbuncle, furuncle, etc.

The drug of the present invention can also be used, for example, in general to prevent and treat infectious diseases. Specifically, it can be applicable, for example, to the following uses.
(1) Prevention of upper respiratory tract infections (including flu, SARS, and MERS)
(2) Prevention of food poisoning (noro, salmonella, etc.)
(3) Cleaning of vomit
(4) Inactivation of hepatitis B and hepatitis C viruses
(5) Prevention, treatment, symptom relief, etc. of ulcerative colitis.
(6) Mold and fungi control (i.e., the drug can be applicable to control (other than fungi and viruses))
(7) Prevention, treatment, symptom relief, etc. of stomatitis (e.g., side effects of molecular target drug)
(8) Preoperative and postoperative care (including cancer operation, etc.)
(9) Care of affected areas of cancer (including oral cavity, mammogram, self-destructive wound, etc.)

In addition, the drug of the present invention can be applicable, for example, to the following uses, by taking advantage of its sterilizing action and high safety.
(1) Denture cleaning
(2) Baby bottle sterilization
(3) Sterilization (prevention of infection), deodorization, etc. of desks, door knobs, and other personal objects touched by many and unspecified people
(4) Sterilization, deodorization, etc. of public transportation systems such as trains, airplanes and buses
(5) Sterilization of entire environment of schools, kindergartens, and nursery schools (including sterilization of desks, doors, shelves, switches, and toys such as building blocks for prevention of infection, etc.)
(6) Sterilization, etc. of medical devices (including cleaning and sterilization of the inside and pipe of dialyzers)
(7) Wiping, cleaning, or washing of diagnostic equipment such as X-rays, CT, electrocardiograms, etc.
(8) Sterilization of materials for medical examinations and operations (including metal such as scalpels and resin)

In addition, the drug of the present invention can be applicable, for example, to the following uses, by taking advantage of its proteolytic action.
(1) Contact lens preservation solution
(2) Eyeglass cleaning solution
(3) Ultrasonic cleaner
(4) Cleaning agent
(5) Wiping and cleaning of glass (including windshields)

In addition, the drug of the present invention can be applicable to deodorize odors, for example, as described below, by taking advantage of its deodorizing action.
(1) Halitosis
(2) Body odor
(3) Stool odor
(4) Odors due to chemical substances, gases, etc. (including odors of factories in general such as chemical factories and food factories)
(5) Garbage-related
(6) Garbage, garbage collection sites, packer cars, recycling centers, incinerators
(7) Sewerage pipes-related
(8) Trapping equipment such as pipes and oil traps
(9) Cigarette
(10) Medical relations (including use for improving QOL of patients, including deodorization of offensive odors due to self-destructive wounds, etc.)
(11) Operation room
(12) Patient room
(13) Environmental odors (faecal odors, wastewater) in poultry houses, pig houses, and cattle houses
(14) Meat center (slaughterhouse)

The drug of the present invention may be used, for example, *in vitro.* The drug of the present invention may be used *in vivo*, for example, by taking advantage of its high safety as described above. For example, the radical generating catalyst of the drug of the present invention may catalyze radical generation from the radical source *in vivo.* The living body may be, for example, a human body, or may be a body of an animal other than a human.

The drug of the present invention may be used, for example, in the digestive organ. For example, the radical generating catalyst of the drug of the present invention may catalyze the radical generation from the radical source in a digestive organ. The digestive organ may be, for example, at least one selected from the group consisting of an oral cavity, a pharynx, an esophagus, a stomach, a duodenum, a small intestine, and a large intestine. The digestive organ may be, for example, the large intestine. The small intestine may be, for example, at least one selected from the group consisting of duodenum, jejunum and ileum. The large intestine may be at least one selected from the group consisting of, for example, cecum, colon and rectum. The drug of the present invention may be used, for example, for sterilizing in the digestive organ, induction of changes in intestinal bacterial flora, treatment or suppression of symptoms of ulcerative colitis, and the like.

The drug of the present invention may be dispersed using, for example, a sprayer, a humidifier, or the like. In this case, for example, in addition to the sterilizing effect or the like on an object to be dispersed, it is possible to obtain the sterilizing action and deodorizing action on the sprayer, the humidifier, and the like. The drug of the present invention is not limited to, for example, human use, and can also be used for animals other than human. Specifically, it can be used, for example, for deodorizing animals and for preventing infectious diseases such as avian influenza and swine influenza. Examples of the use of the drug for animals other than human include those described for human, (including for human body). Further, as the use of the drug for animals other than human, for example, the drug can be used as a drug for use in agriculture and livestock industry described below.

As described above, the drug for use in agriculture and livestock industry according to the present invention is highly safe and has a high sterilizing effect. Thus, the drug for use in agriculture and livestock industry can be used as a drug for use in agriculture, a drug for use in livestock industry, or the like, for example. The drug for use in agriculture can be used as, for example, a bactericide for use in agriculture, an antiviral agent for use in agriculture, a deodorizer for use in agriculture, an insectcide for use in agriculture, a repellent for use in agriculture, or a soil conditioner for use in agriculture. The drug for use in livestock industry can be used as, for example, a bactericides for use in livestock industry, an antiviral agent for use in livestock industry, a deodorizer for use in livestock industry, an insecticide for use in livestock industry, a repellent for use in livestock industry, or a soil conditioners for use in livestock industry. The drug for use in agriculture and livestock industry may be applied to one use or two or more uses, for example.

Examples of the agriculture include rice farming and dry-field farming. Examples of the dry-field farming include production of: vegetables such as cucumbers, tomatoes, green onions, Chinese cabbages, and soybeans; tubers and roots, such as potatoes; flowers and ornamental plants, such as chrysanthemums grown with artificial light, clematis, and Lady Banks' roses (*Rosa banksiae*); fruits such as strawberries; and fertilizers. Examples of the livestock include industrial animals such as cows, pigs, and chickens.

When the drug for use in agriculture and livestock industry according to the present invention is used for the rice farming, the drug for use in agriculture and livestock industry can be used as a bactericide, an insectcide, a repellent, or a soil conditioner, for example. Specifically, for example, by using the drug for use in agriculture and livestock industry during soaking of rice seeds, it is possible to prevent the generation of slime and to reduce the burden of water replacement operations. Further, for example, by using the drug for use in agriculture and livestock industry during seed soaking, stimulation of germination, and seeding, it is possible to prevent rice blast, spot blight, false smut, bakanae disease, and the like. For example, by spreading the drug for use in agriculture and livestock industry over rice fields, it is possible to protect rice from shield bugs, pest insects, and the like. For example, by spreading the drug for use in agriculture and livestock industry during plowing and irrigation of rice fields, it is possible to improve the soil.

When the drug for use in agriculture and livestock industry according to the present invention is used for dry-field farming, the drug for use in agriculture and livestock industry can be used as a bactericide, an antiviral agent, a soil conditioner, or the like, for example. Specifically, for example, by spreading the drug for use in agriculture and livestock industry over leaves of cucumbers, tomatoes, or strawberries, it is possible to prevent powdery mildew, mosaic disease, and the like. For example, by spreading the drug for use in agriculture and livestock industry over leaves of tomatoes, it is possible to prevent gray mold, leaf mold, and the like. For example, by spreading the drug for use in agriculture and livestock industry over leaves of green onions, it is possible to prevent brown leaf rust and the like. For example, by spreading the drug for use in agriculture and livestock industry over leaves of Chinese cabbages, it is possible to prevent root-knot disease and the like. For example, by spreading the drug for use in agriculture and livestock industry over a potato field after being cultivated using a tractor or the like and then cultivating the field again, it is possible to prevent replant failure and the like. For example, by immersing seed potatoes in the drug for use in agriculture and livestock industry, it is possible to disinfect (sterilize) the seed potatoes. For example, by spreading the drug for use in agriculture and livestock industry over leaves of potatoes a plurality of times in a period from germination to harvest of the potatoes, it is possible to prevent common scab and the like. For example, by spreading the drug for use in agriculture and livestock industry over chrysanthemums grown with artificial light, clematis, and Lady Banks' roses (*Rosa banksiae*), it is possible to prevent powdery mildew and the like.

When the drug for use in agriculture and livestock industry according to the present invention is used for the livestock industry, the drug for use in agriculture and livestock industry can be used as a bactericide, a deodorizer, or the like, for example. Specifically, for example, by using the drug for use in agriculture and livestock industry as a dipping agent for cows, it is possible to prevent mastitis and the like. For example, by using the drug for use in agriculture and livestock industry in a hoof bath for a cow or by applying the drug for use in agriculture and livestock industry to an affected area of a cow infected with a hoof disease, it is possible to prevent or treat the hoof disease and the like. For example, by spraying the drug for use in agriculture and livestock industry on a cow with a sprayer or the like, it is possible to prevent respiratory diseases, foot-and-mouth disease, and the like. For example, by spraying the drug for use in agriculture and livestock industry in a livestock barn or the like for cows, pigs, or chickens with a sprayer or the like, it is possible to deodorize the livestock barn or the like. For example, by using the drug for use in agriculture and livestock industry for hen eggs, it is possible to disinfect (sterilize) the hen eggs.

The drug for use in agriculture and livestock industry according to the present invention may be sprayed on, applied to, or spread over a target object, or the target object may be immersed in the drug for use in agriculture and livestock industry, for example. Specifically, when the drug is used to deodorize a space, the drug may be sprayed in the space, for example. When the drug is used for an affected area of a hoof disease or the like, absorbent cotton, gauze, or the like impregnated with the drug may be applied to the affected area, for example. When the drug is used for hand washing, the drug may be in the form of an aqueous solution so that it can be rubbed into hands, for example. Medical instruments and the like can be washed with the drug by spraying the drug on them or immersing them in an aqueous solution containing the drug. When the drug for use in agriculture and livestock industry is used for a machine used in livestock barns, such as an automobile, an agricultural machine, or a forklift, the drug may be sprayed on the machine, or the machine may be washed with the drug, for example. When the drug for use in agriculture and livestock industry is used for deodorization of the above-described industrial animals, the drug may be sprayed with a sprayer or the like or may be spread with a spreader or the like, for example. Hen eggs can be sterilized by applying the drug to the hen eggs, for example.

### <Disinfectant for use in agriculture and livestock industry>

A bactericide for use in agriculture and livestock industry according to the present invention is characterized in that it contains the drug for use in agriculture and livestock industry according to the present invention. The drug for use in agriculture and livestock industry according to the present invention can be used as a bactericide, for example. Although various types of substances have been used as a bactericide conventionally, the sterilizing effects of these substances are not sufficient. Some of them can exhibit enhanced sterilizing effects by increasing their concentrations. However, this poses a problem in safety. A bactericide for use in agriculture and livestock industry containing the drug for use in agriculture and livestock industry according to the present invention can exhibit a sufficient sterilizing effect while the concentration of the drug is low and is highly safe.

### <Bactericide for hand washing for use in agriculture and livestock industry>

The hand-washing bactericide for use in agriculture and livestock industry according to the present invention is characterized in that it contains the drug for use in agriculture and livestock industry according to the present invention. The drug for use in agriculture and livestock industry according to the present invention can be used as a bactericide for hand washing for use in agriculture and livestock industry to disinfect hands, for example. The hand-washing bactericide for use in agriculture and livestock industry including the drug for use in agriculture and livestock industry according to the present invention can exhibit a sufficient sterilizing effect while the concentration of the drug is low and is highly safe.

### <Deodorizer for use in agriculture and livestock industry>

The deodorizer for use in agriculture and livestock industry according to the present invention is characterized in that it contains the drug for use in agriculture and livestock industry according to the present invention. The drug for use in agriculture and livestock industry according to the present invention can be used as a deodorizer for use in agriculture and livestock industry, for example. As a commonly used sterilizing component, a quaternary ammonium salt typically is used. In many cases, a sufficient sterilizing effect cannot be obtained unless the quaternary ammonium salt is contained at a high concentration. This causes a problem in safety. Further, since the quaternary ammonium salt does not have a deodorizing effect, the commercially available products contain a deodorant component as an additional component. While cyclodextrin typically is used as the deodorant component, the cyclodextrin is incapable of decomposing components causing offensive odors. The cyclodextrin merely masks the components causing offensive odors and cannot eliminate the offensive odors themselves. The deodorizer for use in agriculture and livestock industry including the drug for use in agriculture and livestock industry according to the present invention has a high sterilizing effect, and also, is capable of removing substances that cause offensive odors and thus has a high deodorizing effect, for example.

### <Fungicide for use in agriculture and livestock industry>

A fungicide for use in agriculture and livestock industry according to the present invention is characterized in that it contains the drug for use in agriculture and livestock industry according to the present invention. The fungicide for use in agriculture and livestock industry according to the present invention can be used as a fungicide for disinfecting an affected part caused by infection with fungi such as ringworm fungi, for example.

### <Water purifying agent for use in agriculture and livestock industry>

The water purifying agent for use in agriculture and livestock industry according to the present invention is characterized in that it contains the drug for use in agriculture and livestock industry according to the present invention. The water purifying agent for use in agriculture and livestock industry according to the present invention can kill bacteria, such as Legionella bacteria, breeding in water used in agriculture and livestock industry, for example. The drug for use in agriculture and livestock industry according to the present invention does not corrode metals and does not generate gas. Therefore, the water purifying agent for use in agriculture and livestock industry containing the drug for use in agriculture and livestock industry according to the present invention can be used safely. The water purifying agent for use in agriculture and livestock industry according to the present invention can be used to kill bacteria contained in water or to improve quality of water, for example. Thus, the water purifying agent for use in agriculture and livestock industry according to the present invention also can be referred to as a bactericide for water for use in agriculture and livestock industry or a water quality improving agent for water for use in agriculture and livestock industry, for example.

### <Method of using drug for use in agriculture and livestock industry>

The method of using the drug for use in agriculture and livestock industry according to the present invention is characterized in that it includes the step of bringing the drug for use in agriculture and livestock industry according to the present invention into contact with a target object. By the method of using the drug for use in agriculture and livestock industry according to the present invention, it is possible to perform sterilization, deodorization, or the like of the target object, for example.

### Examples

Next, examples of the present invention will be described. It is to be noted, however, that the present invention is by no means limited to the following examples.

### [Reference Example 1]

In the present reference example, it was confirmed that efficient dihydroxylation of styrene can be performed by scandium triflate and sodium chlorite. Specifically, by the dihydroxylation of styrene by scandium triflate and chlorite ions (ClO₂⁻) at ordinary temperature and atmospheric pressure, 1-phenylethane-1,2-diol could be produced efficiently. It was confirmed that the scandium triflate working as a strong Lewis acid generates chlorine dioxide radicals (ClO₂˙) from the chlorite ions (ClO₂⁻) and increases the reactivity of the chlorine dioxide radicals (ClO₂˙).

Oxidization of an olefin to a 1,2-diol is an important industrial process for producing precursors of various types of chemical substances such as resins, pharmaceutical agents, dyes, insecticides, and perfume compounds in the fields of fine chemicals and speciality chemicals. Several methods for converting olefins to corresponding epoxides and alcohols by oxidization using inorganic metal oxo complexes and metallic oxides having heavy atoms have been reported. High-valent Os^{VIII}O₄ is an effective and selective reagent for oxidizing an olefin to a 1,2-diol (References, etc. 1 to 8). However, the toxicity, sublimation property, and waste of the osmium compound cause serious problems. Sodium chlorite (NaClO₂) is a non-toxic inexpensive oxidizing reagent and has been used as a precursor of a chlorine dioxide radical (ClO₂˙) (References, etc. 9 to 12 [the same as Non Patent Literatures 1 to 4]). ClO₂˙ is known as a reactive stable radical. ClO₂˙, however, is an explosive gas that is yellow at room temperature. ClO₂˙ can be experimentally prepared by oxidization of NaClO₂ by Cl₂ and reaction of chloric acid potassium (KClO₃) and oxalic acid (Reference, etc. 13). These methods may cause problems such as the toxicity of Cl₂ and the explosivity of ClO₃⁻. There has been an attempt for epoxidation of an olefin using NaClO₂ as a precursor of ClO₂˙. However, because the oxidization ability of ClO₂˙ was not strong enough to oxidize an olefin to a diol in the absence of an acid, a 1,2-diol product could not be obtained (References, etc. 14 to 17). The activation of Cl=O double bond of ClO₂˙ is a key for selectively dihydroxylating an olefin in one step.

The present reference example reports an efficient synthesis method of a dihydroxylated product of styrene at ordinary temperature and atmospheric pressure by the activation of ClO₂˙ using scandium triflate [Sc(OTf)₃] as a Lewis acid (Reference, etc. 18). The mechanism of dihydroxylation was disclosed on the basis of the detection of a radical intermediate by the EPR and UV-Vis absorption spectroscopy.

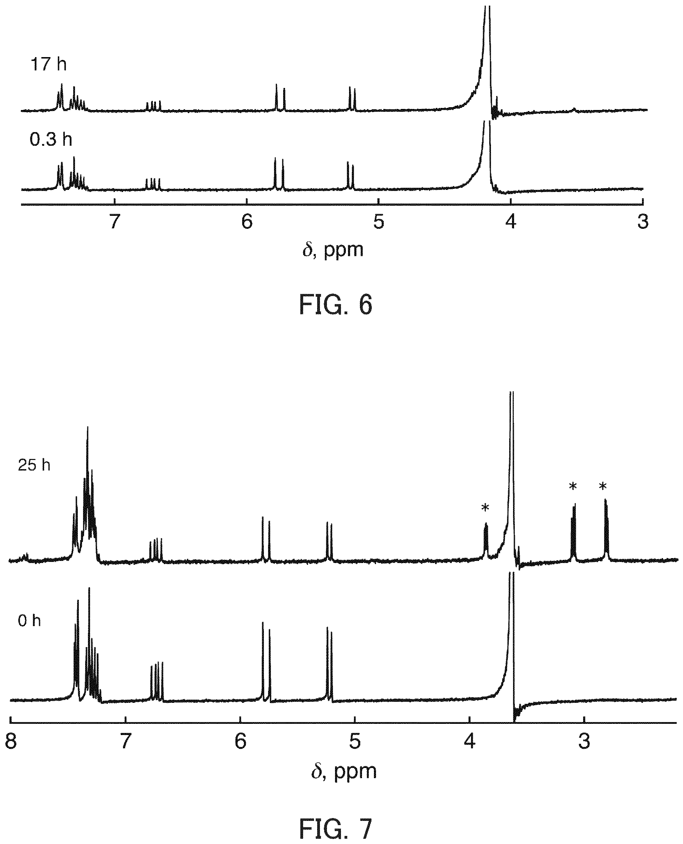

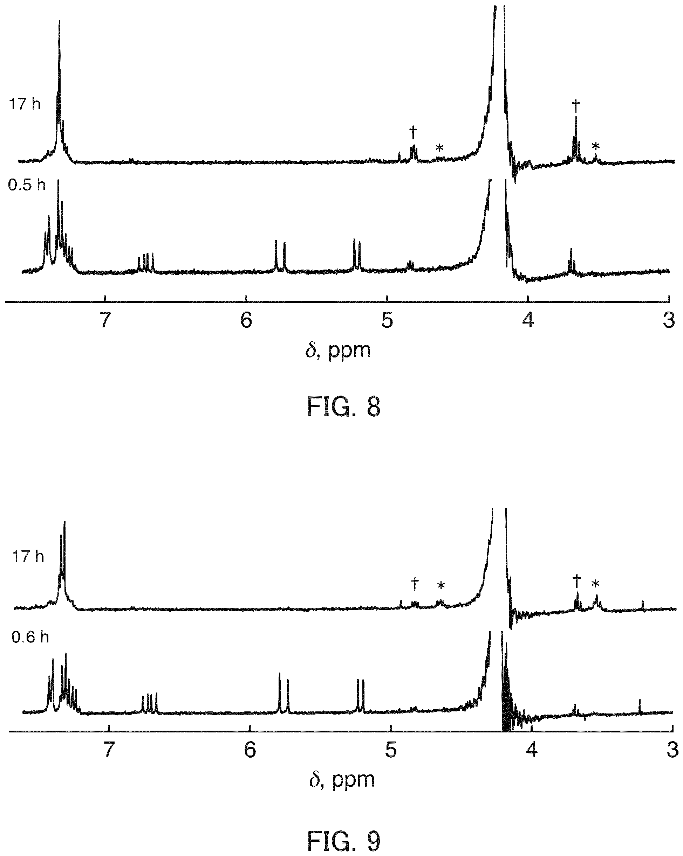

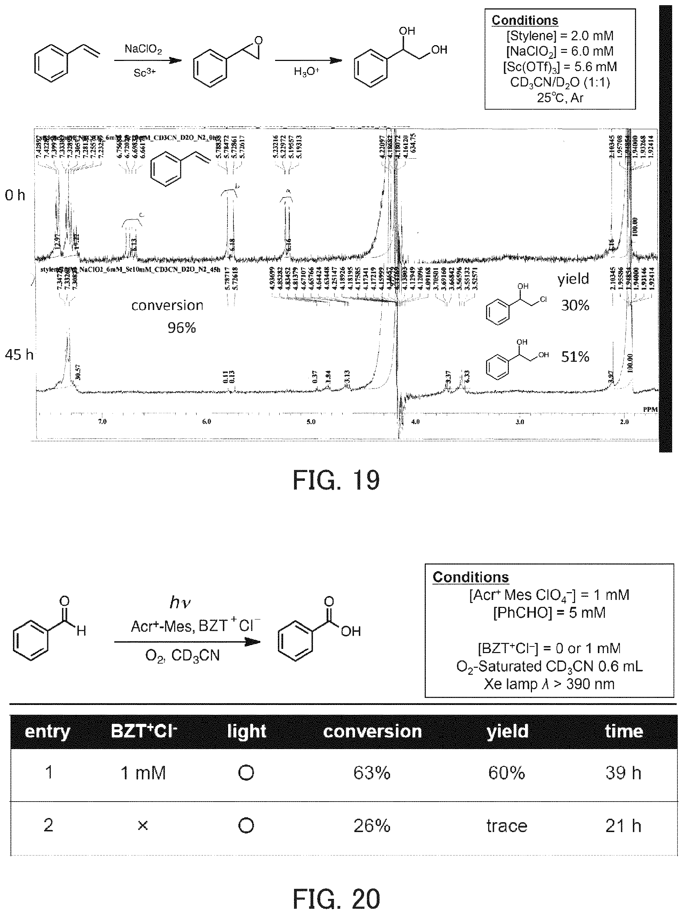

In the reaction of styrene (2.0 mM) by NaClO₂ (20 mM) in an aqueous MeCN solution (MeCN/H2O 1 : 1 v/v) at room temperature (25°C), dihydroxylation of the styrene was not caused (see FIG. 6). FIG. 6 shows the results obtained by performing the above-described reaction using a ¹HNMR spectrum measurement solvent CD₃CN/D₂O (1 : 1 v/v) as MeCN/H₂O and tracing the reaction utilizing ¹HNMR. FIG. 6 shows the ¹HNMR spectra of CD₃CN/D₂O (1 : 1 v/v) collected 0.3 hours and 17 hours after the start of the reaction. When the temperature was increased to 333 K, a dihydroxylated product was not formed but epoxidation was caused (FIG. 7) (References, etc. 14 and 19). FIG. 7 shows the ¹HNMR spectra of CD₃CN/D₂O (4 : 1 v/v) that contains styrene (66 mM) and NaClO₂ (200 mM) at 60°C (333 K) collected 0 hours and 25 hours after mixing. The mark "*" indicates the peak derived from styrene oxide. In contrast, in the case where CF₃COOH (30 mM) as a Brønsted acid was added as an additive, an epoxide was not formed at all 17 hours after mixing, instead, 1-phenylethane-1,2 diol (1) and 2-chloro-1-phenylethanol (2) were produced at the yield of 15% and 69%, respectively [reaction formula (1)]. They were measured utilizing the ¹HNMR spectrum (FIG. 8) (Reference, etc. 20). FIG. 8 shows the ¹HNMR spectra of CD₃CN/D₂O (1 : 1 v/v) that contains styrene (2.0 mM), NaClO₂ (20 mM), and Sc(OTf)₃ (30 mM) at 25°C collected 0.6 hours and 17 hours after mixing. The mark "*" and the mark "†" indicate the peak derived from 1-phenylethane-1,2-diol and the peak derived from 2-chloro-1-phenylethanol, respectively. When Sc(OTf)₃ (30 mM), which is a strong Lewis acid, was used instead of CF₃COOH, the yield of diol (1) increased remarkably to 51% [see the following reaction formula (1)] (FIG. 19) (Reference, etc. 21). FIG. 9 shows the ¹HNMR spectra of CD₃CN/D₂O (1 : 1 v/v) that contains styrene (2.0 mM), NaClO₂ (20 mM), and CF₃COOD (30 mM) collected 0.5 hours and 17 hours after mixing. The mark "*" and the mark "†" indicate the peak derived from 1-phenylethane-1,2-diol and the peak derived from 2-chloro-1-phenylethanol, respectively.

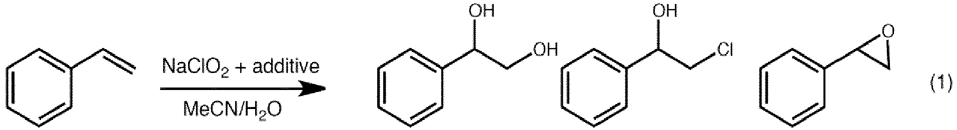

| Additive | Conversion | **1** | **2** | **3** |
|---|---|---|---|---|
| | | | | |
| none | 3 | 0 | 0 | 0 |
| CF₃COOH | 100 | 15 | 69 | 0 |
| Sc(OTf)₃ | 100 | 51 | 30 | 0 |

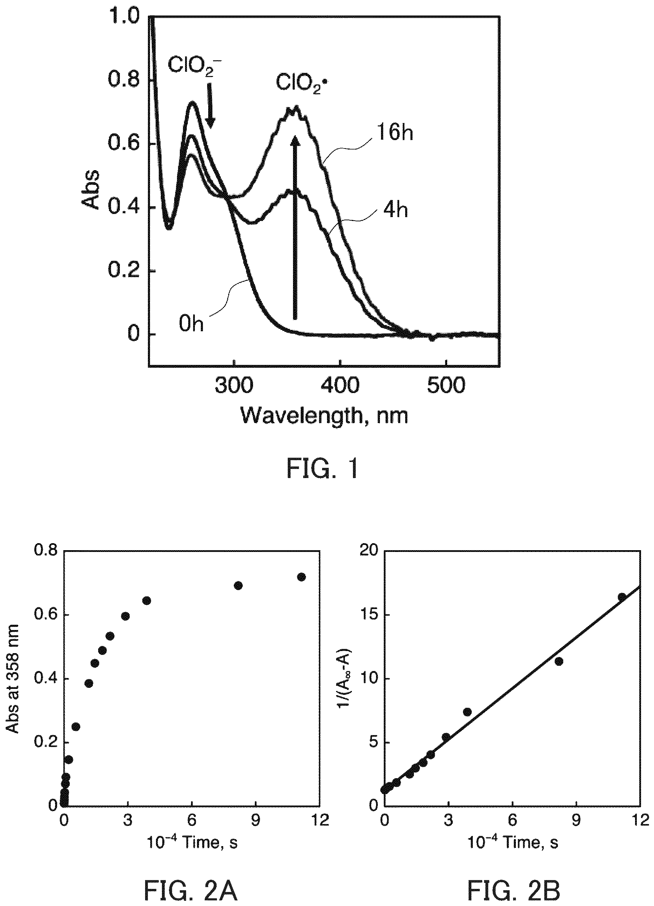

The UV-Vis absorption spectroscopy was adopted for clarifying the reaction mechanism and the detection of a reactive intermediate. As shown in FIG. 1, NaClO₂ showed the absorption band at 260 nm in an aqueous solution. The absorption band was quenched by adding Sc(OTf)₃ (10 mM), and in accordance with this, a new absorption band was increased at 358 nm, and it was identified (assigned) that this absorption band was based on ClO₂˙ (References, etc. 22, 23). Also in the presence of CF₃COOH, a similar change of the absorption spectrum was measured (Reference, etc. 24). FIG. 1 shows the change of occurrence of the absorption band at 358 nm with time. FIG. 1 shows the ultraviolet-visible absorption spectrum of NaClO₂ (5.0 mM) collected 0, 4, and 16 hours after mixing with Sc(OTf)₃ (10 mM) in an aqueous solution at 298 K. In FIG. 1, the horizontal axis indicates the wavelength (nm) and the vertical axis indicates the absorbance. FIG. 2A shows a time profile of UV-Vis absorption at 358 nm in the same reaction as shown in FIG. 1 (formation of Sc³⁺(ClO₂˙) by a reaction between Sc(OTf)₃ (10 mM) and NaClO₂ (5.0 mM) in an aqueous solution (0.20 M acetate buffer having a pH of 2.9) at 298 K). In FIG. 2A, the horizontal axis indicates the time (second) and the vertical axis indicates the absorbance at 358 nm. FIG. 2B shows the secondary plot of the measurement result of FIG. 2A. The time profile (FIG. 2A) meets the secondary plot (FIG. 2B) well. In generation of ClO₂˙ using Sc(OTf)₃, two molecules of ClO₂⁻ are involved in the rate-determining step (see below). The reaction rate constant of the two molecules was determined as 0.16 M⁻¹s⁻¹ based on the slope of the straight line.

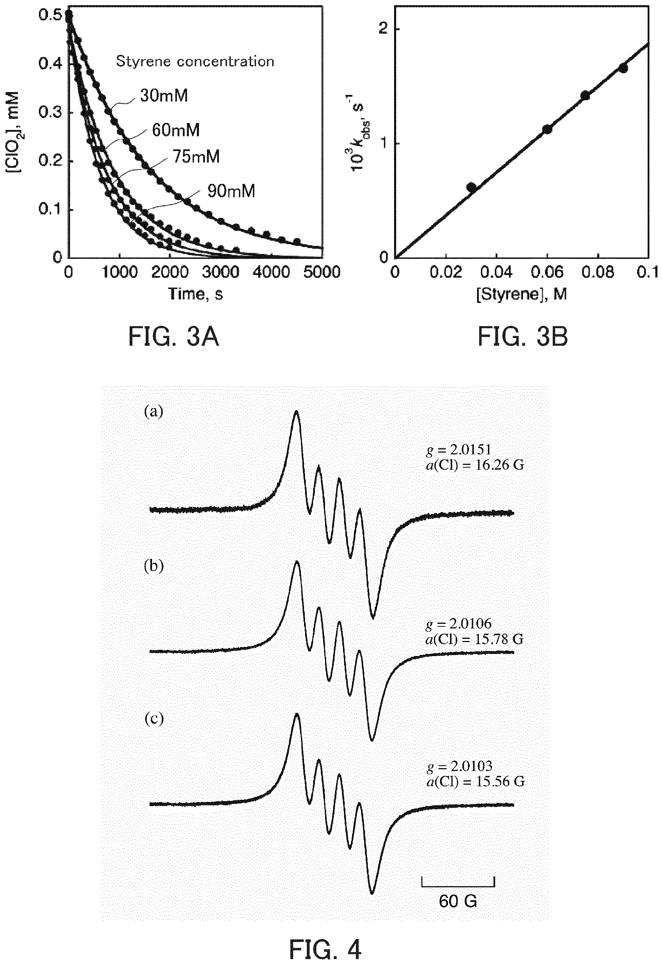

In the absence of a substrate, no decay of the absorbance at 358 nm based on ClO₂˙ generated from NaClO₂ using Sc(OTf)₃ was observed in MeCN at 298 K. FIG. 3A shows the time profile of UV-Vis absorption at 358 nm in consumption of Sc³⁺(ClO₂˙) in the presence of styrene (30 to 90 mM) in a MeCN/H₂O (1 : 1 v/v) solution at 298 K. In FIG. 3A, the horizontal axis indicates the time (second), and the vertical axis indicates the ClO₂˙ concentration. FIG. 3B shows the pseudo first-order rate-styrene concentration plot. In the presence of an excessive amount of styrene, the rate of decay was in accordance with the pseudo first order (FIG. 3B). The pseudo first-order rate (k_{obs}) observed on the increase in dihydroxyl was increased linearly with the increase in a styrene concentration (FIG. 3B). The two-molecule reaction rate constant of the consumption of ClO₂˙ and styrene was determined as 1.9 × 10⁻²M⁻¹s⁻¹ (Reference, etc. 25). For clarifying the radical structure, electronic paramagnetic resonance (EPR) was performed. Pure ClO₂˙ was prepared by refluxing a MeCN solution containing NaClO₂ at 353 K for 1 hour. The EPR spectrum of the thus-obtained pure ClO₂˙ was measured after being cooled to 298 K. As a result, a distinctive isotropic signal was observed with g = 2.0151 (±0.0002) together with four hyperfine lines derived from an unpaired electron of a Cl nucleus (I = 3/2 in ³⁵Cl and ³⁷Cl, each having the same type of magnetic moment of 0.821 and 0.683 ((a) of FIG. 4) (Reference, etc. 26). The G value was remarkably changed by addition of CF₃COOH (g = 2.0106) and Sc(OTf)₃ (g = 2.0103) ((b) and (c) of FIG. 4). The hyperfine binding constant of ClO₂˙ was decreased in the presence of CF₃COOH (15.78 G) and Sc(OTf)₃ (15.56 G) (a(Cl) = 16.26 G) (Reference, etc. 27). This shows that proton and Sc³⁺ bind to ClO₂˙ to form H⁺ClO₂˙ and Sc³⁺ClO₂˙ as reaction intermediates for strongly dihydroxylating styrene (Reference, etc. 28).

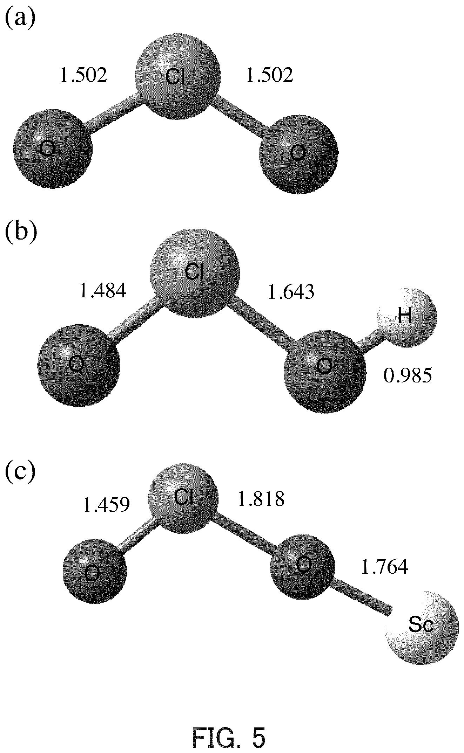

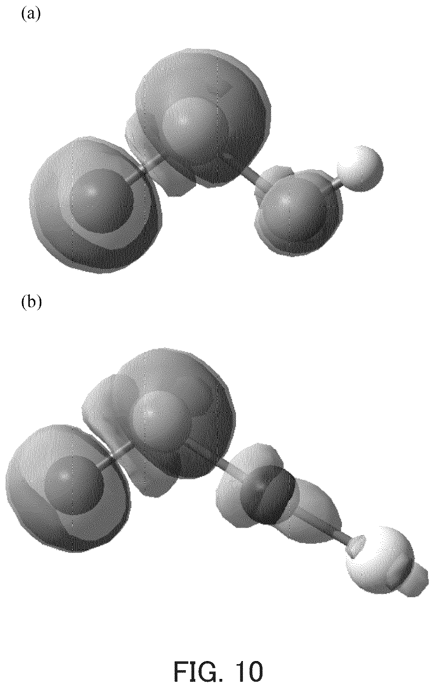

As shown in FIG. 5, properties of ClO₂˙, H⁺ClO₂˙, and Sc³⁺ClO₂˙ were calculated on the basis of the density functional theory (DFT), and the reaction mechanism for dihydroxylation was predicted. The optimization of a structure was performed by the theoretical calculation at the level of DFT CAM-B3LYP/6-311+G(d, p). FIG. 5 shows the bond lengths (Å) of the DFT-optimized structures obtained by the theoretical calculation at the level of CAM-B3LYP/6-311+G(d, p). In FIG. 5, (a) shows the result obtained regarding ClO₂˙; (b) shows the result obtained regarding H⁺ClO₂˙; and (c) shows the result obtained regarding Sc³⁺ClO₂˙. The bond length of the Cl-O double bond of ClO₂˙ was calculated as 1.502 Å ((a) of FIG. 5). The bond length of the Cl-O double bond of H⁺ClO₂˙ was calculated as 1.643 Å ((b) of FIG. 5). (c) of FIG. 5 shows that, as compared to ClO₂˙, the bond strength of Sc³⁺ClO₂˙ is also remarkably weakened (Cl-O: 1.818 Å). There is a possibility that the cleavage of the Cl-O bond may affect advantageously on generation of ClO· as a strong oxidizing agent in the presence of a substrate. FIG. 10 shows spin distributions obtained by the theoretical calculation at the level of CAM-B3LYP/6-311+G(d, p). In FIG. 10, (a) shows the spin distribution of H⁺ClO₂˙ and (b) shows the spin distribution of Sc³⁺ClO₂˙.

On the basis of the above-described results, the dihydroxylation mechanism of styrene by ClO₂˙ is shown in the following reaction formulae (2) to (5) and scheme 1. The disproportionation reaction of NaClO₂ is caused in the presence of H⁺ or Sc³⁺, thereby forming ClO⁻ and ClO₃⁻ [reaction formula (2)] (Reference, etc. 29). ClO⁻ easily reacts with ClO₂⁻ and protons, thereby generating Cl₂O₂ [reaction formula (3)]. Subsequently, Cl₂O₂ is reduced by ClO₂⁻, thereby generating a reactive species ClO₂˙ [reaction formula (4)]. An overall stoichiometry is given by the reaction formula (5). ClO₂˙ is activated by binding to acids such as H⁺ and Sc³⁺. When ClO₂ binds to H⁺, on the basis of the DFT calculation (see above), the Cl-O bond is not cleaved. The oxidization of styrene by H⁺ proceeds by addition of ClO₂˙ to the styrene double bond. In contrast, the dihydroxylation of styrene by Sc³⁺ is caused, as shown in scheme 1, by addition of ClO· and SC³⁺O· generated by homolytic fission of SC³⁺Cl-O bond of a Sc³⁺ClO₂˙ complex to the styrene double bond. Subsequently, a scandium complex is hydrolyzed for obtaining a diol and Sc³⁺ClO· as end products (scheme 1). Sc³⁺ClO· can be reused by adding a large excessive amount of ClO₂⁻ to cause Sc³⁺ClO₂˙ to be formed through oxidization. Also, ClO⁻ can be regenerated by ClO₂⁻ as shown in reaction formula (2). Addition of ClO· formed by cleaving the Cl-O bond of Sc³⁺ClO₂˙ to β carbon of styrene gave two isomers. When the β carbon-CIO bond is formed, as shown in scheme 1, a chlorine compound was obtained as a minor end product.

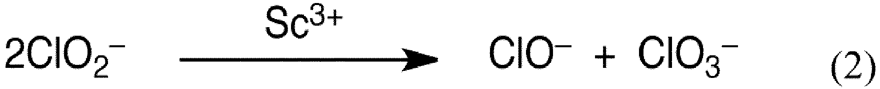

ClO⁻ + ClO₂⁻ + 2H⁺ → Cl₂O₂ + H₂O (3)

Cl₂O₂ + ClO₂⁻ → 2ClO₂˙ + Cl⁻ (4)

4ClO₂⁻ + 2H⁺ → 2ClO₂˙ + ClO₃⁻ + Cl⁻ + H₂O (5)

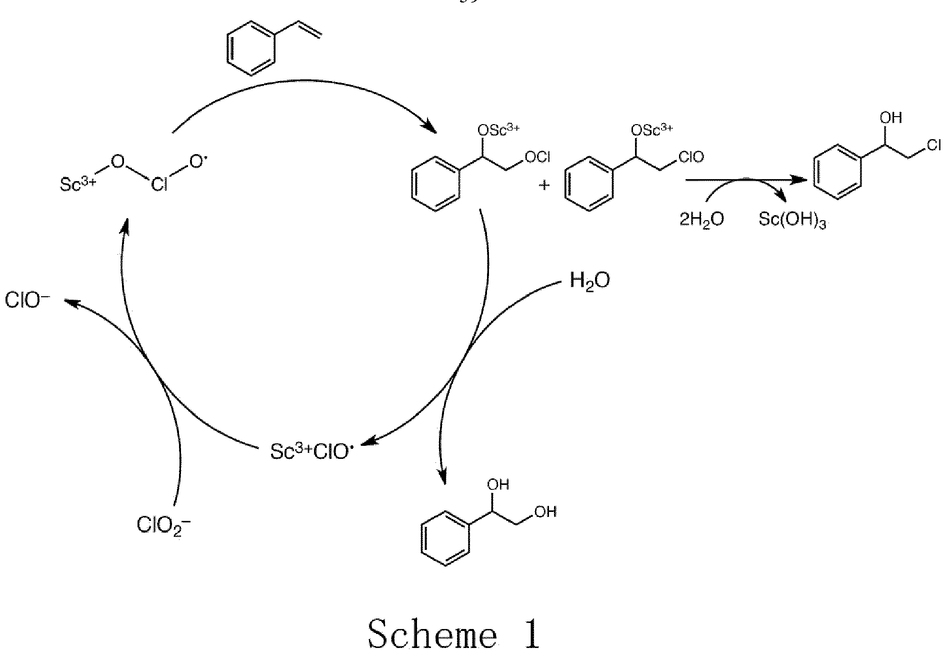

As described above, it was confirmed by the present reference example that ClO₂˙ is an effective dihydroxylation reagent for styrene as a Lewis acid in the presence of Sc³⁺. The present invention can provide a unique dihydroxylation pathway of an olefin without causing hazardous wastes such as heavy metals.

### [References, etc.]

1. M. Schroeder, Chem. Rev., 1980, 80, 187-213.
2. (a) E. N. Jacobsen, I. Marko, W. S. Mungall, G. Schroeder and K. B.Sharpless, J. Am. Chem. Soc., 1988, 110, 1968-1970; and (b) S. G.Hentges and K. B. Sharpless, J. Am. Chem. Soc., 1980, 102, 4263-4265.
3. W. Yu, Y. Mei, Y. Kang, Z. Hua and Z. Jin, Org. Lett., 2004, 6, 3217-3219.
4. (a) A. J. DelMonte, J. Haller, K. N. Houk, K. B. Sharpless, D. A. Singleton, T. Strassner, and A. A. Thomas, J. Am. Chem. Soc., 1997, 119, 9907-9908; and (b) J. S. M. Wai, I. Marko, J. S. Svendsen, M. G.Finn, E. N. Jacobsen and K. B. Sharpless, J. Am. Chem. Soc., 1989, 111, 1123-1125.
5. (a) S. Kobayashi, M. Endo and S. Nagayama, J. Am. Chem. Soc., 1999, 121, 11229-11230; and (b) S. Kobayashi, T. Ishida and R. Akiyama, Org. Lett., 2001, 3, 2649-2652.
6. H. C. Kolb, P. G. Andersson and K. B. Sharpless, J. Am. Chem. Soc., 1994, 116, 1278-1291.
7. E. J. Corey and M. C. Noe, J. Am. Chem. Soc., 1996, 118, 11038-11053.
8. S. Y. Jonsson, K. Faernegrdh and J.-E. Baeckvall, J. Am. Chem. Soc., 2001, 123, 1365-1371.
9. H. Dodgen and H. Taube, J. Am. Chem. Soc., 1949, 71, 2501-2504.
10. J. K. Leigh, J. Rajput, and D. E. Richardson, Inorg. Chem., 2014, 53, 6715-6727.
11. C. L. Latshaw, Tappi J., 1994, 163-166.
12. (a) J. J. Leddy, in Riegel's Handbook of Industrial Chemistry, 8th edn. Ed., J. A. Kent, Van Nostrand Reinhold Co. Inc, New York, 1983, pp. 212-235; and (b) I. Fabian, Coord. Chem. Rev., 2001, 216-217, 449-472.
13. M. J. Masschelen, J. Am. Works Assoc., 1984, 76, 70-76.
14. X.-L. Geng, Z. Wang, X.-Q. Li, and C. Zhang J. Org. Chem., 2005, 70, 9610-9613.
15. A. Jangam and D. E. Richardson, Tetrahedron Lett., 2010, 51, 6481-6484.
16. J. J. Kolar and B. O. Lindgren, Acta Chem. Scand. B, 1982, 36, 599-605.
17. B. O. Lindgren, T. Nilsson, Acta Chem. Scand. B, 1974, 28, 847-852.
18. (a) S. Fukuzumi and K. Ohkubo, J. Am. Chem. Soc., 2002, 124, 10270-10271; and (b) S. Fukuzumi and K. Ohkubo, Chem.-Eur. J., 2000, 6, 4532-4535.
19. Epoxidation of styrene (66 mM) by NaClO₂ (200 mM) was checked in a MeCN/H₂O mixture solution (4 : 1 v/v) at 333 K (Reference, etc. 14). The yield of styrene oxide was 44% and the conversion ratio of styrene was 61%.
20. E. V. Bakhmutova-Albert, D. W. Margerum, J. G. Auer and B. M. Applegate, Inorg. Chem., 2008, 47, 2205-2211.
21. As a result of measurement utilizing ¹HNMR, styrene epoxide as an intermediate in reaction by CF₃COOH or Sc(OTf)₃ was not observed.
22. C. Rav-Acha, E. Choushen (Goldstein) and S. Sarel, Helv. Chim. Acta, 1986, 69, 1728-1733.
23. There is a possibility that ClO₂˙ generated from acetic anhydride and NaClO₂ (Reference, etc. 22) is in the protonated form (H+ClO₂˙) in a ClO₂˙ aqueous solution.
24. W. Masschelein, Ind. Eng. Chem. Prod. Res. Devel., 1967, 6, 137-142.
25. This numerical value is slightly greater than the value of the conversion of styrene to epoxide by ClO₂˙ (1.17 × 10⁻² M⁻¹s⁻¹) (Reference, etc. 10).
26. (a) T. Ozawa and T. Kwan, Chem. Pharm. Bull., 1983, 31, 2864-2867; and (b) T. Ozawa, T. Trends Org. Chem., 1991, 2, 51-58.
27. The calculated values of the spin distribution of Sc³⁺ClO₂˙ and H+ClO₂˙ are shown in FIG. 10. According to this, each of Sc and H nuclei does not show a spin density. This means that the EPR spectrum does not show the hyperfine splitting derived from Sc (I = 7/2) or H (I = 1/2).
28. As to the bond between Sc³⁺ and an oxo group of a metal oxo complex, see the following references:
   (a) J. Chen, X. Wu, K. M. Davis, Y.-M. Lee, M. S. Seo, K.-B. Cho, H. Yoon, Y J. Park, S. Fukuzumi, Y. N. Pushkar and W. Nam, J. Am. Chem. Soc., 2013, 135, 6388-6391; (b) H. Yoon, Y.-M. Lee, X. Wu, K.-B. Cho, Y N. Pushkar, W. Nam and S. Fukuzumi, J. Am. Chem. Soc., 2013, 135, 9186-9194; and (c) S. Fukuzumi, K. Ohkubo, Y.-M. Lee and W. Nam, Chem.-Eur. J., 2015, 21, 17548-17559.
29. As to the disproportionation of a neutral radical by Sc³⁺, see the following reference: I. Nakanishi, T. Kawashima, K. Ohkubo, T. Waki, Y Uto, T. Kamada, T. Ozawa, K. Matsumoto and S. Fukuzumi, S. Chem. Commun., 2014, 50, 814-816.

### [Example 1]

In the present example, an oxygen reduction reaction was activated by benzethonium chloride. Research and development of Lewis acids have been carried out widely in various organic synthesis reactions. In most of the research, a metal ion or a metal complex was used as a Lewis acid site, and the ligand design around the Lewis acid site was the main focus of the research. In the present example, benzethonium chloride was used as an ammonium derivative having strong Lewis acidic properties, and whether the benzethonium chloride is widely useful in an oxygenation reaction of an aromatic organic compound using sodium chlorite was examined.

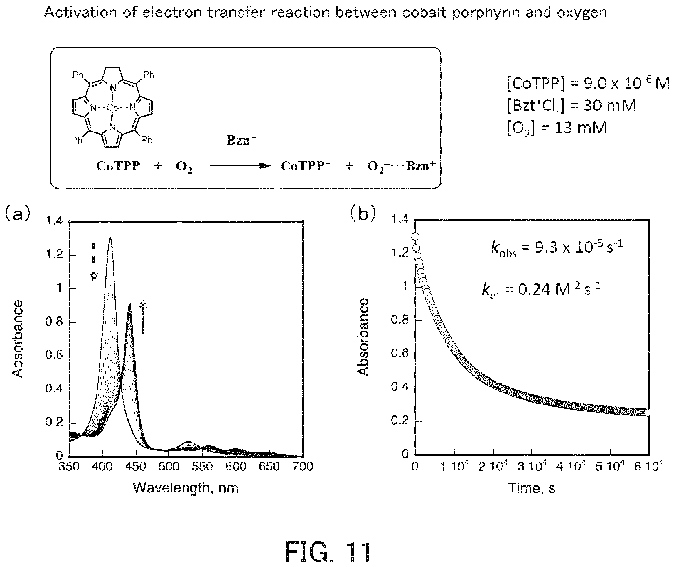

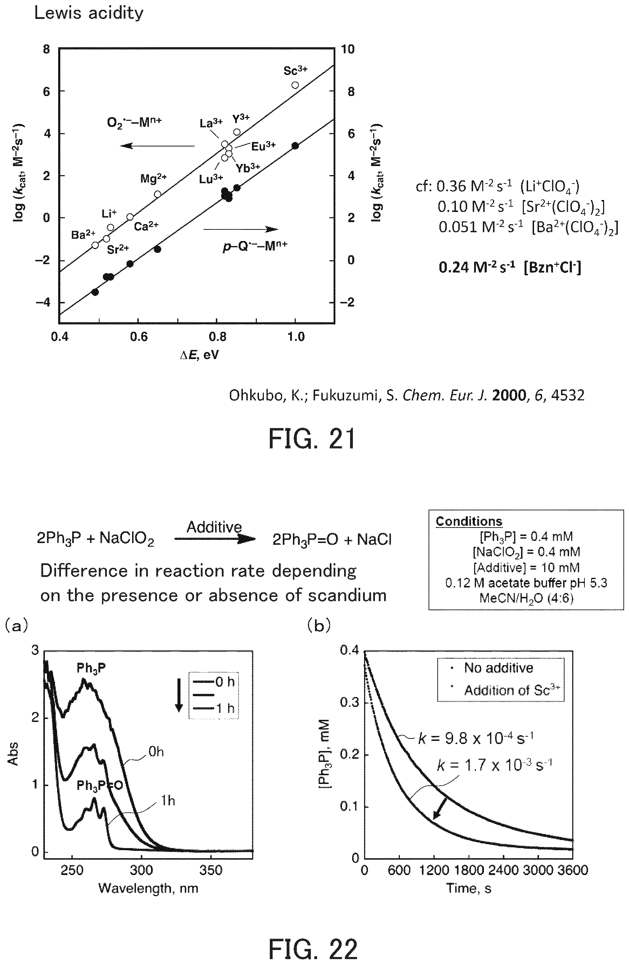

In acetonitrile, electron transfer does not proceed at all between cobalt (II) tetraphenylporphyrin complex Co(II)TPP (TPP = 5,10,15,20-tetraphenylporphyrin) (Eox = 0.35V vs SCE) and molecular oxygen (E_{red} = -0.86 V vs SCE). However, when benzethonium chloride (Bzn⁺) was added to this oxygen saturated solution ([CoTPP] = 9.0 × 10⁻⁶ M, [O₂] = 13 mM) ([Bzn⁺Cl⁻] = 30 mM), accompanying decay of the absorption band derived from Co(II)TPP at 411 nm, an increase in absorption band characteristic of Co(III)TPP⁺ at 433 nm was observed with an isosbestic point ((a) of FIG. 11). In FIG. 11, (a) is a graph showing the time course of the ultraviolet-visible absorption spectrum of the solution. The horizontal axis indicates the wavelength (nm), and the vertical axis indicates the absorbance. It is considered the above behavior indicates that an electron transfer reaction from Co(II)TPP to molecular oxygen proceeded and Co(III)TPP⁺ was generated. The time constant of the change in decay of the absorption band at 411 nm with time was substantially the same as the time constant of the change in increase in the absorption band at 433 nm, and the reaction rate constant was determined to be 9.3 × 10⁻⁵ s⁻¹ by pseudo-first-order curve fitting ((b) of FIG. 11). In the graph of (b) of FIG. 11, the horizontal axis indicates the time, and the vertical axis indicates the absorbance. This reaction rate constant exhibited first-order dependence on the oxygen concentration and the Bzn⁺ concentration, and the catalytic transfer reaction rate constant (k_{cat}) was determined to be 0.24 M⁻²s⁻¹ from the slope of the plot. Previous research (Ohkubo, K.; Fukuzumi, S. Chem. Eur. J., 2000, 6, 4532) has revealed that the electron transfer reaction from Co(II)TPP to molecular oxygen proceeds efficiently in the presence of a Lewis acid such as metal ions. In the case of Bzn⁺ used in the present research, it is considered that the reaction proceeded in a manner similar to the Lewis acid catalyzed reaction. The catalyst reaction rate constant of Bzn⁺ (0.24 M⁻²s⁻¹) obtained in the present example was slightly lower than that of lithium perchlorate (0.36) and larger than that of strontium perchlorate (0.10 M⁻²s⁻¹) and barium perchlorate (0.051 M⁻²s⁻¹). From these results, it is considered that Bzn⁺ has a relatively strong Lewis acidity. From this catalyst reaction rate constant, the ΔE value as the indicator of the Lewis acidity was determined to be 0.53 eV according to the method described in the literature. Indeed, it has been reported that ammonium salts served as Lewis acids. For example, from the fact that the ΔE value of the ammonium salt in the present example was larger than the ΔE value (0.32 eV) of ammonium hexafluorophosphate (NH₄PF₆) (e.g., References, etc. 33), it was confirmed that the ammonium salt in the present example exhibits strong Lewis acidity among various types of ammonium. The graph of FIG. 21 shows the Lewis acidities of benzethonium chloride [Bzn⁺Cl⁻] and various metal complexes. In FIG. 21, the horizontal axis indicates the ΔE value (eV), and the vertical axis indicates the logarithm of the reaction rate constant (log(k_{cat},M⁻²s⁻¹)).

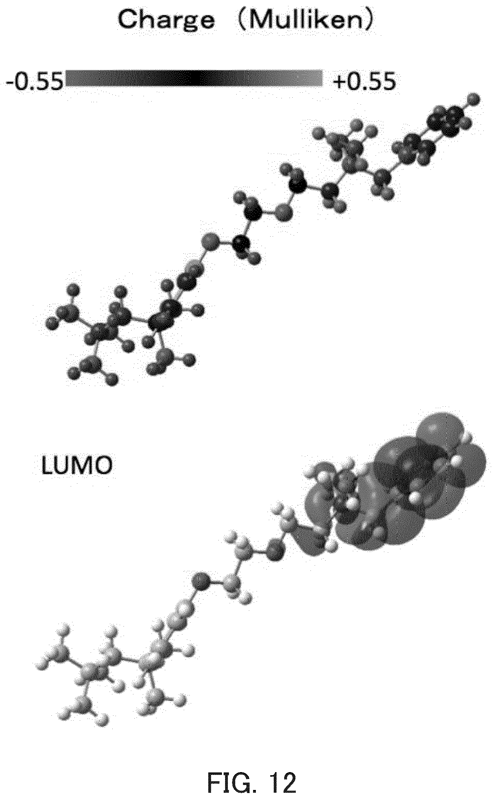

The structure of Bzn⁺ was optimized by density functional calculation (B3LYP/6-31G(d) level). The obtained structure is shown in FIG. 12. As can be seen from FIG. 12, from the localization of Mulliken charges and LUMO in the vicinity of ammonium nitrogen, it is expected that Bzn⁺ exhibits Lewis acidity.

### [Reference Example 2]

The present example examined the acceleration effect of a disproportionation reaction of NaClO₂ by a Lewis acid.

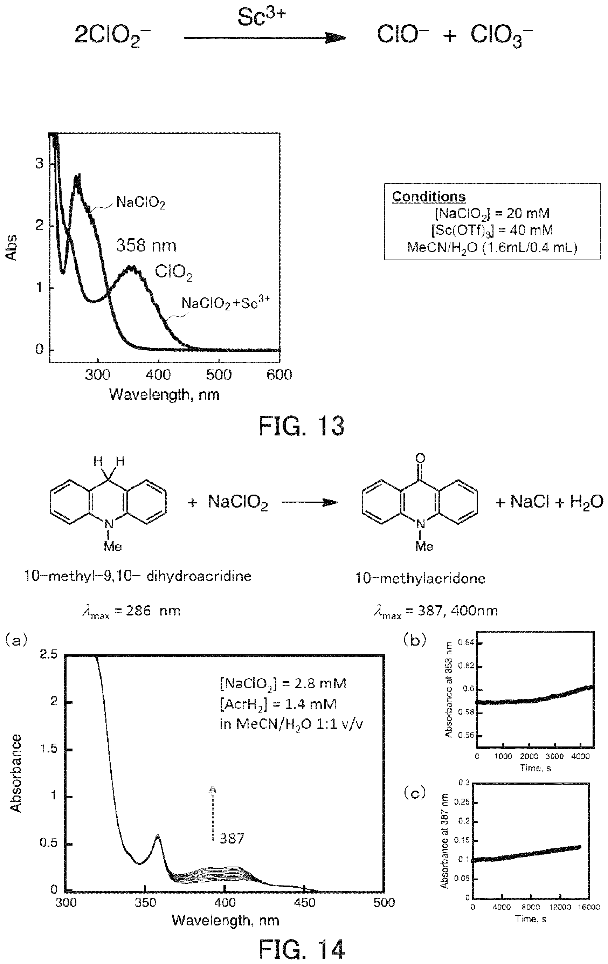

As confirmed in Reference Example 1, degradation of sodium chlorite (NaClO₂) is not observed because it is very stable in a mixed solution containing a neutral aqueous solution and acetonitrile. When Sc(OTf)₃ (40 mM) was added to this 20 mM solution, accompanying the decay of the absorption band of NaClO₂, an increase in absorption band characteristic of ClO₂ radicals (ClO₂˙) was observed at 358 nm immediately (FIG. 13). In FIG. 13, the horizontal axis indicates the wavelength (nm), and the vertical axis indicates the absorbance. The increase in this absorption band could be observed as a change over time by decreasing the concentration of Sc(OTf)₃, as confirmed in Reference Example 1 (FIG. 1). By conducting similar studies on magnesium ions, lithium ions, and the like having lower Lewis acidities than scandium ions, the reaction rate constants of the respective ions were determined. It is known that Lewis acids catalyze various disproportionation reactions. In this reaction, it is considered that ClO₂⁻ is disproportionated to ClO⁻ and ClO₃⁻ according to the reaction formula (2) of Reference Example 1 by a similar mechanism. Thereafter, it is considered that the generated ClO⁻ reacts with ClO₂⁻, which is present in a large excessive, in the presence of an acid and gives Cl₂O₂ (the reaction formula (3) of Reference Example 1). Thereafter, it is considered that Cl₂O₂ further reacts with ClO₂⁻ and gives ClO₂ radicals as active radical species (the reaction formula (4) of Reference Example 1).

### [Example 2]

The present example examined the generation of ClO₂ radicals and acceleration of an oxidation reaction using benzethonium chloride.

ClO₂ radicals are considered to exhibit strong oxygenation reaction activity. Thus, first, in a mixed solution containing deoxygenated acetonitrile and water (deoxygenated acetonitrile: water = 1 : 1 v/v), 10-methyl-9,10-dihydroacridine (AcrH₂) (1.4 mM) and sodium chlorite (NaClO₂) (2.8 mM) were added. In this case, there was almost no progress in an oxygenation reaction of AcrH₂ (FIG. 14). In FIGs. 14, (a) to (c) are graphs each showing the time course of the reaction. In the graph (a) of FIG. 14, the horizontal axis indicates the wavelength (nm), and the vertical axis indicates the absorbance. The graph (b) of FIG. 14 shows the time course of the absorbance at a wavelength of 358 nm. In the graph (b) of FIG. 14, the horizontal axis indicates the time (second), and the vertical axis indicates the absorbance. The graph (c) of FIG. 14 shows the time course of the absorbance at a wavelength of 387 nm. In the graph (c) of FIG. 14, the horizontal axis indicates the time (second), and the vertical axis indicates the absorbance.

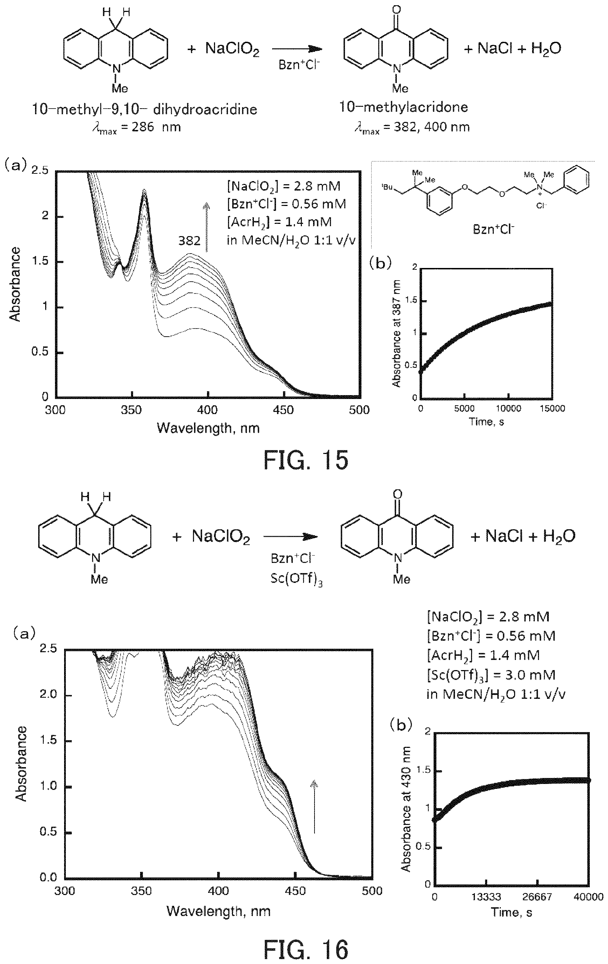

Next, the same mixed solution as that shown in FIG. 14 was prepared. When Bzn⁺ (0.56 mM) was further added to the mixed solution, an oxygenation reaction from AcrH₂ to 10-methylacridone proceeded (FIG. 15). In FIG. 15, (a) and (b) are graphs each showing the time course of the reaction. In the graph (a) of FIG. 15, the horizontal axis indicates the wavelength (nm), and the vertical axis indicates the absorbance. The graph (b) of FIG. 15 shows the time course of the absorbance at a wavelength of 387 nm. In the graph (b) of FIG. 15, the horizontal axis indicates the time (second), and the vertical axis indicates the absorbance. As can be seen from the graphs (a) and (b) of FIG. 15, an increase in absorption derived from 10-methylacridone (λmax = 382 nm) with time was observed. This demonstrates that the oxygenation (oxidation) reaction from AcrH₂ to 10-methylacridone proceeded.

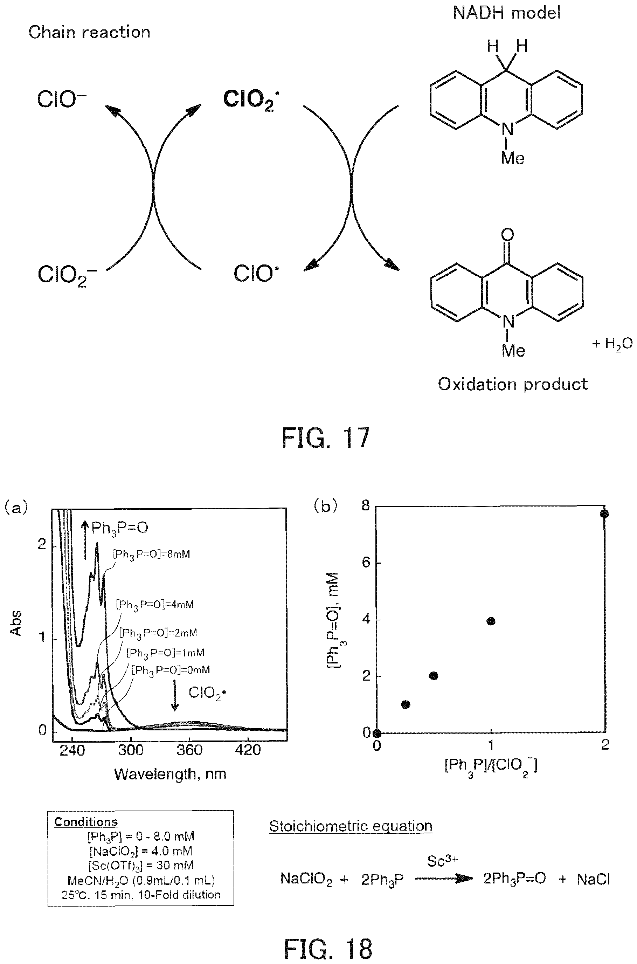

Also, when scandium trifluoromethanesulfonate (Sc(OTf)₃,3.0 mM) was further added to the same mixed solution as that shown in FIG. 15, an oxygenation reaction from AcrH₂ to 10-methylacridone proceeded (FIG. 16). In FIG. 16, (a) and (b) are graphs each showing the time course of the reaction. In the graph (a) of FIG. 16, the horizontal axis indicates the wavelength (nm), and the vertical axis indicates the absorbance. The graph (b) of FIG. 16 shows the time course of the absorbance at a wavelength of 430 nm. In the graph (b) of FIG. 16, the horizontal axis indicates the time (second), and the vertical axis indicates the absorbance. As can be seen from the graphs (a) and (b) of FIG. 16, an increase in absorption derived from 10-methylacridone with time was observed. This demonstrates that the oxygenation (oxidation) reaction from AcrH₂ to 10-methylacridone proceeded. It is considered that this oxygenation reaction proceeds through the chain reaction mechanism shown in FIG. 17. That is, it is considered that, in this reaction, ClO₂˙ abstracts hydrogen from 10-methylacridone and adds oxygen to the 10-methylacridone at the same time, thereby forming acridone. On the other hand, it is considered that ClO₂˙, which is a product obtained after the addition of the oxygen, caused an electron transfer reaction with ClO₂⁻ and regenerates while giving ClO⁻ and ClO₂˙.

### [Reference Example 3]

In the present reference example, an oxygenation reaction of a substrate by NaClO₂ using a Lewis acid was used for an oxygenation reaction from triphenylphosphine to triphenylphosphine oxide in order to examine whether it works. More specifically, the oxygenation reaction from triphenylphosphine to triphenylphosphine oxide by NaClO₂ was performed in the presence and the absence of scandium triflate Sc(OTf)₃, which is a Lewis acid in order to examine whether the Lewis acid promotes the reaction.

First, under the following conditions, in the presence or absence of Sc(OTf)₃, the reaction was performed at ordinary temperature and atmospheric pressure (no light irradiation), and the reaction was traced by the ultraviolet-visible absorption spectrum. The ultraviolet-visible absorption spectrum shown in (a) of FIG. 22 shows the conversion of triphenylphosphine to triphenylphosphine oxide over time. In (a) of FIG. 22, the horizontal axis indicates the wavelength (nm), and the vertical axis indicates the absorbance. The graph shown in (b) of FIG. 22 shows the changes of a triphenylphosphine (Ph₃P) concentration over time in the presence and the absence of Sc(OTf)₃ (Sc³⁺). In (b) of FIG. 22, the horizontal axis indicates the time (second), and the vertical axis indicates the triphenylphosphine (Ph₃P) concentration (mM). As shown in (b) of FIG. 22, while the reaction rate constant k calculated from the curve in the absence of Sc³⁺ was 9.8 × 10⁻⁴ S⁻¹, the reaction rate constant k calculated from the curve in the presence of Sc³⁺ was increased to 1.7 × 10⁻³ S⁻¹. Thus, it was confirmed that Sc³⁺ (a Lewis acid) promoted the reaction.
[Ph₃P] = 0.4 mM
[NaClO₂] = 0.4 mM
Sc(OTf)₃ = 0 or 10 mM
0.12M acetate buffer, pH5.3
MeCN/H₂O (4 : 6)

The reaction did not proceed at all by mixing triphenylphosphine and NaClO₂ (4.0 mM) in deoxygenated acetonitrile MeCN/H₂O (0.9 ml/0.1 ml). By adding scandium triflate Sc(OTf)₃ (30 mM) thereto, oxygenated products were produced efficiently. The initial concentration of triphenylphosphine was set to 1.0 mM, 2.0 mM, 4.0 mM, or 8.0 mM, and each reaction was performed at 25°C for 15 minutes. The reaction was traced by monitoring the change in the ultraviolet-visible absorption spectrum ((a) of FIG. 18). In (a) of FIG. 18, the horizontal axis indicates the wavelength (nm), and the vertical axis indicates the absorbance. As can be seen from (a) of FIG. 18, it can be considered that ClO₂ radicals as active radical species were generated by scandium ions Sc³⁺, and Ph₃P was oxygenated to Ph₃P=O. The stoichiometry is as represented by the following reaction formula (6), and it was confirmed that the reaction proceeds almost quantitatively ((b) of FIG. 18). In (b) of FIG. 18, the horizontal axis indicates the initial concentration of Ph₃P, and the vertical axis indicates the concentration of the generated Ph₃P=O.

2Ph₃P + NaClO₂ → 2Ph₃P=O + NaCl (6)

### [Reference Example 4]

In the present reference example, an oxidation reaction of a raw material aromatic compound (benzaldehyde) was performed in acetonitrile in the presence of perchlorate (Acr⁺-Mes ClO₄⁻) of 9-mesityl-10-methylacridinium (Acr⁺-Mes) and oxygen, thereby obtaining an oxidation reaction product (benzoic acid) (FIG. 20). The reaction was performed in the presence or absence of Bzn⁺Cl⁻.

As a reaction solvent, 0.6 ml of CD₃CN saturated with oxygen gas was used. As shown in FIG. 20, 1 mM of Acr⁺-Mes ClO₄⁻, 5 mM of benzaldehyde (PhCHO), and 0 or 1 mM of Bzn⁺Cl⁻ were added thereto, and the resultant mixture was or was not irradiated with light at a wavelength of 390 nm emitted from a xenon lamp. The reaction was traced by ¹HNMR. The results obtained are shown in the table in FIG. 20. In the table, "×" means the reagent was not added or light irradiation was not performed; "o" means light irradiation was performed; "conversion" indicates the conversion rate of the raw material aromatic compound (benzaldehyde); "yield" indicates the yield of the benzoic acid; and "time" indicates the reaction time. As can be seen from FIG. 20, in the case where Bzn⁺Cl⁻ was not added, the yield of the benzoic acid was a trace amount. In the case where Bzn⁺Cl⁻ was added, the yield of the benzoic acid was 60%, and the conversion rate of the benzaldehyde was 63%. It is considered this result indicates that, while the reactivity of Acr⁺-Mes was low in the absence of the Lewis acid (Bzn⁺Cl⁻), radical generation from Acr⁺-Mes was promoted in the presence of the Lewis acid (Bzn⁺Cl⁻), which suggests that the Lewis acid (Bzn⁺Cl⁻) served as a strong reaction promoter.

### [Reference Example 5]

In the present reference example, according to the measurement method described in the above section "Lewis acidity measuring method", oxidation reaction products of cobalt tetraphenylporphyrin were produced using various types of ammonium as radical generating catalysts and oxygen molecules as a radical source (also serving as an oxidizing agent). More specifically, as to acetonitrile (MeCN) that contains cobalt tetraphenylporphyrin in the following chemical reaction formula (1a), saturated O₂, and an object whose Lewis acidity is to be measured (e.g., a cation of a metal or the like, represented by Mⁿ⁺ in the following chemical reaction formula (1a)), the change of the ultraviolet-visible absorption spectrum was measured at room temperature, and whether CoTPP⁺ was obtained as an oxidation reaction product was examined.

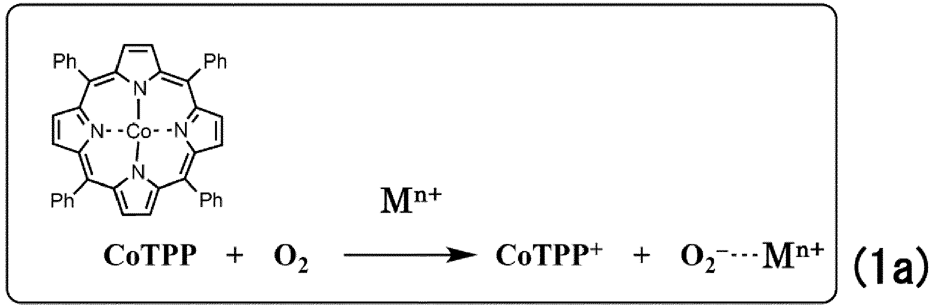

The oxidation reaction was performed using each type of ammonium shown in the following table as a radical generating catalyst. In the following table, the numerical value expressed in the unit "k_{cat},M⁻²s⁻¹" is a reaction rate constant of reaction between CoTPP and oxygen in the presence of Lewis acid, which is an indicator of the Lewis acidity of each ammonium. The numerical value expressed in the unit "LUMO, eV" is the energy level of LUMO. The "benzethonium chloride" means benzethonium chloride, "benzalkonium chloride" means benzalkonium chloride, "tetramethylammonium hexafluorophosphate" means tetramethylammonium hexafluorophosphate, "tetrabutylammonium hexafluorophosphate" means tetrabutylammonium hexafluorophosphate, and "ammonium hexafluorophosphate" means ammonium hexafluorophosphate (Note from translator: in the original text in Japanese, the above sentence explains the meanings of the English terms in the table in Japanese).

**[Table tpp]**

| | LUMO, eV | *k*_{cat}, M⁻² s⁻¹ |
|---|---|---|
| benzethonium chloride | -4.12 | 0.24 |
| benzalkonium chloride | -4.02 | 0.18 |
| tetramethylammonium hexafluorophosphate | -3.58 | > 0.1 |
| tetrabutylammonium hexafluorophosphate | -2.07 | > 0.1 |
| ammonium hexafluorophosphate | -5.73 | 20 |

### [Examples of Drug]

Next, specific examples of the drug of the present invention will be described. It is to be noted, however, that the drug of the present invention is not limited to the following examples.

### Example 3:

5 g of sodium chlorite was dissolved in purified water to obtain 100 ml of an aqueous solution. Thus, the 40,000 ppm sodium chlorite aqueous solution was obtained (solution A). 0.1 g of benzethonium chloride was dissolved in 100 ml of purified water to prepare a 100 ml of 1000 ppm aqueous solution (solution B). 0.1 M phosphate-NaOH buffer (pH = 9.5) was provided. To 600 ml of purified water at pH 7, 20 ml of the solution A diluted 10-fold and 80 ml of the buffer were added, and then 80 ml of the solution B was added. Purified water was further added to make the total amount 800 ml. In this manner, the drug according to Example 3 was obtained. This drug was an aqueous solution containing NaClO₂ and benzethonium chloride. The pH of the drug of Example 3 produced in this manner was 7.5.

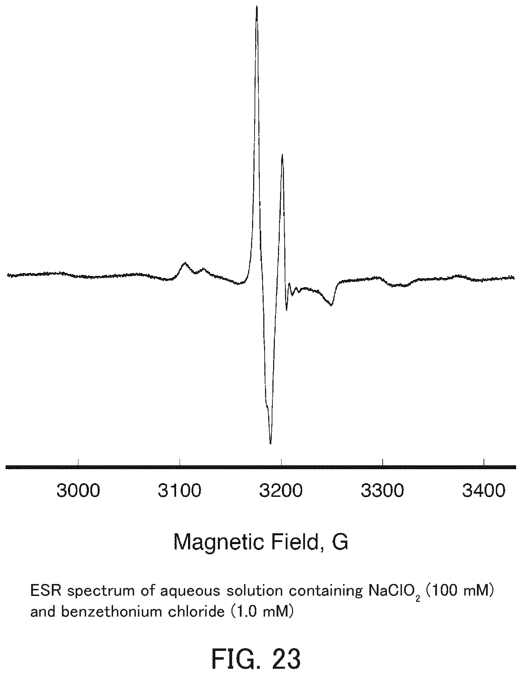

The drug was produced in the same manner as in Example 3 except that the NaClO₂ concentration was set to 100 mM and the benzethonium chloride concentration was set to 1.0 mM. This drug was sealed in an ESR tube, and the electron spin resonance (ESR) spectrum was measured at minus 196°C using an electron spin spectrum spectrophotometer (JES-ME-LX "X-band" [trade name], Japan Electron Optics Laboratory Co. Ltd.) to confirm the generation of chlorine dioxide radicals. It is to be noted that ESR and electron paramagnetic resonance (EPR) are synonymous. FIG. 23 shows the measured ESR spectral diagram. As shown in FIG. 23, since peaks indicating the presence of radicals appeared, it was confirmed that benzethonium chloride, which is ammonium, served as a radical generating catalyst for sodium chlorite (NaClO₂) to generate chlorine dioxide radicals.

### Example 4:

5 g of sodium chlorite was dissolved in purified water to obtain 100 ml of an aqueous solution. Thus, the 40,000 ppm sodium chlorite aqueous solution was obtained. 0.1 g of benzethonium chloride was dissolved in 100 ml of purified water to prepare a 1000 ppm aqueous solution. The 40,000 ppm sodium chlorite aqueous solution was diluted 40-fold to obtain a 1000 ppm aqueous solution. 10 ml of the sodium chlorite aqueous solution and 10 ml of the benzethonium chloride aqueous solution were added to 80 ml of purified water to obtain a 100 ppm aqueous solution. In this manner, a drug according to Example 4 (aqueous solution containing NaClO₂ and benzethonium chloride) was obtained. The pH of the thus obtained drug according to Example 4 was 7.5. The ESR spectrum of the drug according to Example 4 was measured in the same manner as in Example 3. Likewise Example 3 confirmed that benzethonium chloride served as a radical generating catalyst for sodium chlorite (NaClO₂) to generate chlorine dioxide radicals.
Comparative Example 1:
   a bactericide containing sodium hypochlorite and water (commercially available product) Comparative Example 2:
   a sterilizing deodorizer containing sodium hypochlorite (commercially available product) Comparative Example 3:
   a sterilizing deodorizer containing hypochlorous acid and water (commercially available product)
Comparative Example 4:
   a sterilizing deodorizer containing sodium hypochlorite and water (commercially available product)
Comparative Example 5:
   a sterilizing deodorizer containing sodium hypochlorite and water (commercially available product)
Comparative Example 6:
   a sodium chlorite standard solution 1000 ppm (test product)
Comparative Example 7:
   5 g of sodium chlorite (Wako Pure Chemical Industries, Ltd.) was dissolved in 100 ml of purified water to prepare a 40,000 ppm aqueous solution. The 40,000 ppm aqueous solution was further diluted with purified water to obtain a 100 ppm aqueous solution. In this manner, a test product according to Comparative Example 7 was obtained.
Comparative Example 8:
   a benzethonium chloride aqueous solution (test product)

### (Experimental Example 1)

In Experimental Example 1, the following were provided first.

Bacterial strains to be used:
*Staphylococcus aureus*
*Escherichia coli* MV1184

### Bacterial solution:

Bacteria cultured in a BHI agar medium were collected with a platinum loop and placed in a BHI liquid medium, and the BHI liquid medium was shaken. The bacteria were allowed to grow in the BHI liquid medium for a whole day and night. 50 µl of the resultant culture solution was diluted 190-fold with a BHI liquid medium, and mixed well with the BHI liquid medium by stirring. The resultant mixture was used as a bacterial solution.

Using each bacterial strain and bacterial solution, the effect (sterilizing action) was examined in the following manner.

A microplate (with a lid) was sterilized for 10 minutes with a UV sterilization lamp. Next, a BHI liquid medium, the bacterial solution, and the drug according to Example 3 were injected in this order into each well with a micropipette. The bacteria were cultured at 37°C for 24 hours. Thereafter, the bacteria were examined using a microplate reader, and the minimum inhibitory concentration (MIC) was determined. As a control, the same examination was performed using the liquid medium only. Further, 10 µl of the culture solution was collected from the well in the vicinity of the MIC, and inoculated in a petri dish. The bacteria were cultured at 37°C for 24 hours, and the minimum bactericidal concentration (MBC) was determined. The results obtained are shown in Table 1.

Using the bactericide according to Comparative Example 1 instead of the drug according to Example 3, the MIC and MBC were determined in the same manner. The results obtained are shown in Table 1.

Using each of the sterilizing deodorizers according to Comparative Examples 2 to 5 instead of the drug according to Example 3, the MIC of the *Staphylococcus aureus* was determined in the same manner. The results obtained are shown in Table 1.

Using the test product according to Comparative Example 6 instead of the drug according to Example 3, the MIC of the *Staphylococcus aureus* and the MIC and MBC of the *Escherichia coli* were determined in the same manner. The results obtained are shown in Table 1.

**[Table 1]**

| | | Ex. 1 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|---|
| *S. aureus* | MIC (ppm) | 1.56 | 300 | ineffective | ineffective | ineffective | ineffective | 40 |
| | MBC (ppm) | 3.12 | 300 | | | | | |
| *E. coli* | MIC (ppm) | 12.5 | 220 | | | | | 30 |
| | MBC (ppm) | 20.0 | 220 | | | | | 50 |

### (Experimental Example 2)

Using the drug according to Example 4 or Comparative Example 7 or 8 instead of the drug according to Example 3, the MIC of the *Escherichia coli* was determined in the same manner. The results obtained are shown in Table 2.

**[Table 2]**

| | | Ex. 2 | Comp. Ex. 7 | Comp. Ex. 8 |
|---|---|---|---|---|
| *E. coli* | MIC (ppm) | 12.5 > | 25 < | 17.5 |

### (Experimental Example 3)

In Experimental Example 3, the following were provided first.

Bacterial strain to be used:
*Streptococcus pyogenes*

Bacterial solution:
A bacterial solution was obtained in the same manner as in Experimental Example 1.

Using the above bacterial strain and bacterial solution and the drug according to Example 3, the MIC and MBC were determined in the same manner as in Experimental Example 1. The results obtained are shown in Table 3.

**[Table 3]**

| | | Ex. 1 |
|---|---|---|
| *Streptococcus pyogenes* | MIC (ppm) | 0.1 |
| | MBC (ppm) | 1.0 |

### (Experimental Example 4)

In Experimental Example 4, the following were provided first.

Bacterial strains to be used:
*Streptococcus mutans*

Bacterial solution:
Bacteria cultured in a BHI agar medium were collected with a platinum loop and placed in a BHI liquid medium, and the BHI liquid medium was shaken. The bacteria were allowed to grow in the BHI liquid medium for a whole day and night. 50 µl of the resultant culture solution was diluted 190-fold with a BHI liquid medium, and mixed well with the BHI liquid medium by stirring. The resultant mixture was used as a bacterial solution.

Using each bacterial strain and bacterial solution, the effect was examined in the following manner.

The bacterial solution was injected with a micropipette into a BHI liquid medium placed in each of two test tubes. Saccharose was added thereto so that the concentration thereof was 0.2%. The bacteria were cultured at 37°C for 18 hours to allow them to form a biofilm. The medium in each test tube was discarded in a beaker, and the biofilm was washed twice with PBS. The drug according to Example 3 was injected into one of the test tube, and PBS was injected into the other test tube. Then, the test tubes were shaken at 37°C for 30 minutes. The liquid in each test tube was discarded in a beaker, and the biofilm was washed twice with PBS. A BHI liquid medium was injected into the test tubes, and the bacteria were cultured at 37°C for 24 hours. 10 µl of the medium collected from each test tube was inoculated into a nutrient agar medium, and the bacteria were cultured at 37°C for 24 hours. The presence or absence of colonies was checked through visual observation. As a result, while no colony was observed in the test tube to which the drug according to Example 3 had been injected, many colonies were observed in the test tube to which PBS had been injected.

In order to examine the effect of the drug on the bacterial cells in the biofilm, the following test was conducted.

The bacterial solution was injected with a micropipette into a BHI liquid medium placed in microtubes. Saccharose was added thereto so that the concentration thereof was 0.2%. The bacteria were cultured at 37°C for 18 hours to allow them to form a biofilm. The medium in each microtube was discarded in a beaker, and the biofilm was washed twice with PBS. The drug according to Example 3 was injected into one of the microtubes, and PBS was injected into the other microtube. The bacteria in the former microtube and the bacteria in the latter microtube were aged at 37°C for 15 minutes and 30 minutes, respectively. The liquid in each microtube was discarded in a beaker, and the biofilm was washed twice with PBS. A BHI liquid medium was injected into the test tubes and homogenized. Thereafter, the bacteria were cultured at 37°C for 24 hours. 10 µl of the medium collected from each microtube was inoculated into a nutrient agar medium, and the bacteria were cultured at 37°C for 24 hours. The presence or absence of colonies was checked through visual observation. As a result, while no colony was observed in the microtube to which the drug according to Example 3 had been injected, many colonies were observed in the microtube to which PBS had been injected. These results demonstrate that, by impregnating a biofilm with the drug according to Example 3, the drug acts on bacteria deep inside the biofilm to exhibit the sterilizing effect.

### (Experimental Example 5)

In Experimental Example 5, the following bacterial strains were used. Except for this, the MIC and MBC were determined using the drug according to Example 3 in the same manner as in Experimental Example 1. The results obtained are shown in Table 4.

Bacterial strains to be used:
Bacteria 1 (*Porphyromonas gingivalis*)
Bacteria 2 (*Treponema denticola*)
Bacteria 3 (*Tannerella forsythensis*)
Bacteria 4 (*Aggregatibacter actinomycetemcomitans*)

**[Table 4]**

| | | Ex. 1 |
|---|---|---|
| Bacteria 1 | MIC (ppm) | 20.0 |
| | MBC (ppm) | 20.0 |
| Bacteria 2 | MIC (ppm) | 25.0 |
| | MBC (ppm) | 25.0 |
| Bacteria 3 | MIC (ppm) | 12.5 |
| | MBC (ppm) | 12.5 |
| Bacteria 4 | MIC (ppm) | 35-45 |
| | MBC (ppm) | 35-50 |

### (Experimental Example 6)

Test pieces (25.4 mm × 25.4 mm) respectively made of iron, aluminum, tin plate, and stainless steel were washed. Thereafter, the test pieces made of each material were immersed in resin containers containing the drug according to Example 3, a 1.2% sodium hypochlorite aqueous solution, and tap water, respectively, and then, the resin containers were covered with a lid. The test pieces were taken out on a nonwoven fabric after a lapse of each time period shown in Tables 5 and 6, and the conditions of the test pieces were examined through visual observation. In the examination, pictures were taken when necessary, and a microscope was used when the change was subtle. The evaluation was made using the following evaluation criteria.

- -:: no corrosion
- ±:: generation of rust
- +:: fairly large amount of rust
- ++:: vary large amount of rust
- +++:: corrosion of metal surfaces

**[Table 5]**

| Test piece | | 10 min | 30 min | 1 hr | 3 hr | 6 hr |
|---|---|---|---|---|---|---|
| Iron | Example 1 | ± | ± | ± | + | + |
| | Hypochlorous acid | + | + | ++ | +++ | +++ |
| | Tap water | ± | ± | ± | ± | + |
| Aluminum | Example 1 | - | - | - | - | - |
| | Hypochlorous acid | ± | ± | ± | + | + |
| | Tap water | - | - | - | - | - |
| Tin plate | Example 1 | - | - | - | - | - |
| | Hypochlorous acid | - | - | - | ± | ± |
| | Tap water | - | - | - | - | - |
| Stainless steel | Example 1 | - | - | - | - | - |
| | Hypochlorous acid | - | - | - | - | - |
| | Tap water | - | - | - | - | - |

**[Table 6]**

| Test piece | | 1 day | 3 days | 1 week | 2 weeks | 3 weeks | 4 weeks |
|---|---|---|---|---|---|---|---|
| Iron | Example 1 | + | + | + | + | + | + |
| | Hypochlorous acid | +++ | +++ | +++ | +++ | +++ | +++ |
| | Tap water | + | + | + | + | + | + |
| Aluminum | Example 1 | - | - | - | - | - | - |
| | Hypochlorous acid | ++ | ++ | ++ | +++ | +++ | +++ |
| | Tap water | - | - | - | - | - | - |
| Tin plate | Example 1 | - | ± | ± | ± | ± | ± |
| | Hypochlorous acid | + | ++ | +++ | +++ | +++ | +++ |
| | Tap water | - | ± | ± | + | + | + |
| Stainless steel | Example 1 | - | - | - | - | - | - |
| | Hypochlorous acid | - | - | - | - | - | - |
| | Tap water | - | - | - | - | - | - |

### (Experimental Example 7)

The deodorizing performance test was conducted in accordance with JEM 1467 "domestic air cleaner" in the Standards of the Japan Electrical Manufacturers' Association. In the measurement, cigarettes were burned while operating a circulator in an acrylic container (1 m in height × 1 m in width × 1 m in depth) with an internal volume of 1 m³ to fill the container with smoke. After all the cigarettes were burned, the circulator was stopped, and the drug according to Example 3 was sprayed in the container by operating a sprayer. The concentrations of three components, namely, ammonia, acetaldehyde, and acetic acid, in the container were measured over 2 hours at regular intervals to trace the change in concentrations. Similarly, formaldehyde vapor was injected into an acrylic container and the formaldehyde concentration in the container was measured over 2 hours at regular intervals to trace the change in concentration. The sprayer was operated in "Manual" mode. As a control, a blank test in which the sprayer was not operated was also conducted. The results obtained are shown in Tables 7 to 10. The malodorous components were measured using detector tubes (Gastec Corporation). The detector tubes used for the measurement are shown below.

Detector tubes used for measurement

| | |
|---|---|
| ammonia: | No. 3L |
| acetaldehyde: | No. 92L |
| acetic acid: | No. 81L |
| formaldehyde: | No. 91 |

**[Table 7]**

| Elapsed time | Ammonia concentration (ppm) | | Example 1 |
|---|---|---|---|
| | Example 1 | Blank test | Removal rate (%) |
| Start | 30 | 32 | - |
| 5 min | 8 | 32 | 73 |
| 10 min | 5 | 32 | 83 |
| 20 min | 4 | 32 | 87 |
| 30 min | 2 | 32 | 93 |
| 45 min | 1 | 31 | 97 |
| 60 min | 1 > | 31 | 97 < |
| 90 min | 1 > | 31 | 97 < |
| 120 min | 1 > | 26 | 97 < |

**[Table 8]**

| Elapsed time | Acetaldehyde concentration (ppm) | | Example 1 |
|---|---|---|---|
| | Example 1 | Blank test | Removal rate (%) |
| Start | 14 | 14 | - |
| 5 min | 12 | 14 | 14 |
| 10 min | 10 | 14 | 29 |
| 20 min | 10 | 14 | 29 |
| 30 min | 7 | 14 | 50 |
| 45 min | 7 | 14 | 50 |
| 60 min | 7 | 14 | 50 |
| 90 min | 7 | 14 | 50 |
| 120 min | 6 | 14 | 57 |

**[Table 9]**

| Elapsed time | Acetic acid concentration (ppm) | | Example 1 |
|---|---|---|---|
| | Example 1 | Blank test | Removal rate (%) |
| Start | 12 | 10 | - |
| 5 min | 0.5 > | 10 | 96 < |
| 10 min | 0.5 > | 10 | 96 < |
| 20 min | 0.5 > | 10 | 96 < |
| 30 min | 0.5 > | 10 | 96 < |
| 45 min | 0.5 > | 10 | 96 < |
| 60 min | 0.5 > | 9.5 | 96 < |
| 90 min | 0.5 > | 9.0 | 96 < |
| 120 min | 0.5 > | 9.0 | 96 < |

**[Table 10]**

| Elapsed time | Formaldehyde concentration (ppm) | | Example 1 |
|---|---|---|---|
| | Example 1 | Blank test | Removal rate (%) |
| Start | 20 | 20 | - |
| 5 min | 10 | 20 | 50 |
| 10 min | 8 | 20 | 60 |
| 20 min | 5 | 20 | 75 |
| 30 min | 3 | 20 | 85 |
| 45 min | 2 | 20 | 90 |
| 60 min | 2 | 20 | 90 |
| 90 min | 2 | 18 | 90 |
| 120 min | 2 | 18 | 90 |

### (Experimental Example 8)

The drug according to Example 3 was sprayed using a sprayer to measure the deodorizing performance for cigarette odor. First, cigarettes were burned in a room with a 6-tatami mat size to fill the room with smoke at a predetermined concentration. Next, a sprayer was set in the room, and the odor intensity in the room was measured three times, namely, before operating the sprayer, one hour after operating the sprayer, and two hours after operating the sprayer. The sprayer was set near a wall in the room, and the odor was collected at a height of 1 m in the middle of the room. Two circulation fans were set in the room, and they were operated at all times to maintain the air-circulating conditions. The sprayer was operated in "Manual" mode. As a control, a blank test in which the sprayer was not operated was also conducted. The odor intensity was determined as follows according to the six-grade odor intensity measurement method. The results obtained are shown in Table 11.

The odor intensity was evaluated by six testers (test panel). The results were calculated by determining the average value of the odor intensities given by the respective testers. The six-grade odor intensity measurement method is a method for converting odor intensity to a numerical value using human olfaction. The members of the test panel who had joined the test were those who had taken the legally-required olfactometry and had been admitted as having normal olfaction.

In the six-grade odor intensity measurement method, the following numerical values are used as evaluation criteria.
- 0:: odorless
- 1:: barely perceivable odor (detection threshold concentration)
- 2:: weakly perceivable odor (recognition threshold concentration)
- 3:: easily perceivable odor
- 4:: strong odor
- 5:: very strong odor

**[Table 11]**

| Elapsed time | Odor intensity | |
|---|---|---|
| | Example 1 | Blank test |
| Start | 4.6 | 4.5 |
| 1 h | 3.5 | 4.5 |
| 2 h | 2.9 | 4.2 |

### (Experimental Example 9)

The drug according to Example 3 was sprayed with a sprayer to measure the performance thereof to remove airborne bacteria (general bacteria, fungi). First, a sprayer was set in a room with a 6-tatami mat size, and the concentration of airborne bacteria in the air was measured three times, namely, before operating the sprayer, one hour after operating the sprayer, and two hours after operating the sprayer. The sprayer was set near a wall in the room, and the airborne bacteria were collected at a height of 1 m in the middle of the room. Two circulation fans were set in the room, and they were operated at all times to maintain the air-circulating conditions. The airborne bacteria were measured by a filtration method using a membrane filter. The sprayer was operated in "Manual" mode. As a control, a blank test in which the sprayer was not operated was also conducted. The results obtained are shown in Tables 12 and 13.

### Measurement conditions etc. in Experimental Example 9

| | |
|---|---|
| Filter to be used: | Toyo Roshi Kaisha, Ltd., 37 mm Monitors |
| Amount of sucked air: | 300 l (sucked for 15 minutes at 20 l/min) |
| Medium to be used: | m-TGE Broth liquid medium for general bacteria (Toyo Seisakusho Kaisha, Ltd.) |
| | m-Green Y & M Broth liquid medium for fungi (Toyo Seisakusho Kaisha, Ltd.) |
| Culture conditions: | 30°C for 72 hours for general bacteria |
| | 30°C for 5 days for fungi |

**[Table 12]**

| Elapsed time | The number of airborne general bacteria (the number of bacteria/300 l) | | Example 1 |
|---|---|---|---|
| | Example 1 | Blank test | Removal rate (%) |
| Start | 13 | 11 | - |
| 1 h | 0 | 11 | 100 |
| 2 h | 0 | 11 | 100 |

**[Table 13]**

| Elapsed time | The number of airborne fungi (the number of fungi/300 l) | | Example 1 |
|---|---|---|---|
| | Example 1 | Blank test | Removal rate (%) |
| Start | 10 | 11 | - |
| 1 h | 0 | 10 | 100 |
| 2 h | 0 | 9 | 100 |

### (Experimental Example 10)

In Experimental Example 10, the following bacterial strains were used. Except for this, the MIC or MBC was determined using the drug according to Example 3 in the same manner as in Experimental Example 1. The results obtained are shown in Table 14.

Bacterial strains to be used:
*Streptococcus mutans*
*hemolytic streptococcus*
*Bacillus subtilis*
*Candida albicans*

**[Table 14]**

| | | Example 1 |
|---|---|---|
| *Streptococcus mutans* | MIC (ppm) | 5 |
| | MBC (ppm) | 15 |
| *Hemolytic streptococcus* | MIC (ppm) | 0.1 |
| | MBC (ppm) | 1.0 |
| *Bacillus subtilis* | MIC (ppm) | 12.5 |
| | MBC (ppm) | |
| *Candida albicans* | MIC (ppm) | 5 > |
| | MBC (ppm) | |

### (Experimental Example 11)

Using the drug according to Example 3, a deodorization test was performed in accordance with an instrumental analysis implementation manual; a detector tube method, a gas chromatography method (the Certification Standards of Antibacterial Finished Textile Products of Japan Textile Evaluation Technology Council were applied with necessary modifications). The results obtained are shown in Table 15.

**[Table 15]**

| Odor component | Impression of odor | Concentration 1 (ppm) | Concentration 2 (ppm) | Gas reduction rate (%) |
|---|---|---|---|---|
| ammonia | excrement | 100 | 7 | 93 |
| acetic acid | vinegar | 50 | 1 | 98 |
| hydrogen sulfide | rotten egg | 4.00 | 0.12 | 97 |
| methyl mercaptan | rotten onion | 8.00 | 4.96 | 38 |
| tritylamine | rotten fish | 28.00 | 3.08 | 89 |
| isovaleric acid | musty socks | 38.00 | 0.38 | 99 |

| | | | | |
|---|---|---|---|---|
| Concentration 1: initial gas concentration Concentration 2: gas concentration after a lapse of 2 hours Gas reduction rate: ([concentration 1 - concentration 2]/concentration 1) × 100 | | | | |

### (Experimental Example 12)

The drug according to Example 3 was applied to acne lesions for 14 consecutive days (a few times a day, about 2 ml/time). As a result, it was clear that the acne was healed by the application of the drug. This result demonstrates that the drug of the present invention is useful as an acne treatment agent.

### Example 5

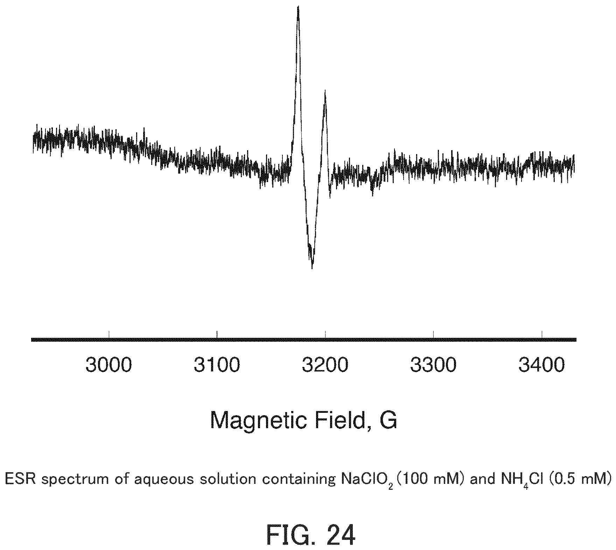

The drug according to Example 5 was produced in the same manner as in Example 3 except that a half amount (mole number) of ammonium chloride (NH₄Cl) was used as ammonium instead of benzethonium chloride. The pH of the drug according to Example 5 was 7.5. Further, a drug was produced in the same manner as in Example 5 except that the NaClO₂ concentration was set to 100 mM and the ammonium chloride (NH₄Cl) concentration was set to 0.5 mM, and the ESR spectrum of the drug was measured in the same manner as in Example 3. FIG. 24 shows the measured ESR spectral diagram. As shown in FIG. 24, since peaks indicating the presence of radicals appeared, it was confirmed that ammonium chloride (NH₄Cl) served as a radical generating catalyst for sodium chlorite (NaClO₂) to generate chlorine dioxide radicals.

### Example 6

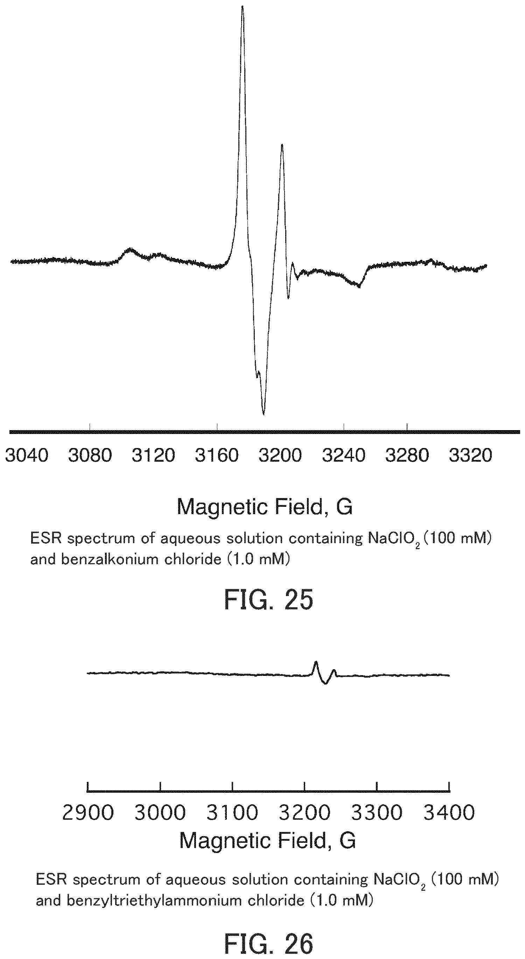

The drug according to Example 6 was obtained in the same manner as in Example 3 except that the same amount (mole number) of benzalkonium chloride (the following chemical formula) was used as ammonium instead of benzethonium chloride. The pH of the drug according to Example 6 was 7.5. Further, a drug was produced in the same manner as in Example 6 except that the NaClO₂ concentration was set to 100 mM and the benzalkonium chloride concentration was set to 1.0 mM, and the ESR spectrum of the drug was measured in the same manner as in Example 3. FIG. 25 shows the measured ESR spectral diagram. As shown in FIG. 25, since peaks indicating the presence of radicals appeared, it was confirmed that benzalkonium chloride, which is ammonium, served as a radical generating catalyst for sodium chlorite (NaClO₂) to generate chlorine dioxide radicals.

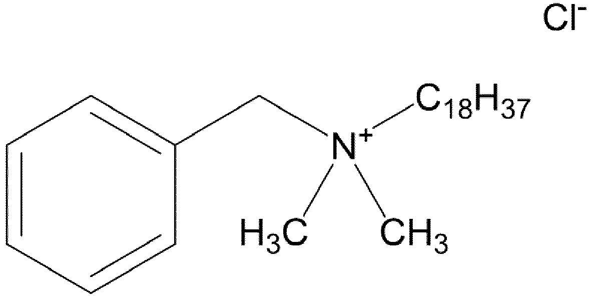

### Example 7

The drug according to Example 7 was obtained in the same manner as in Example 3 except that the same amount (mole number) of benzyltriethylammonium chloride (the following chemical formula) was used as ammonium instead of benzethonium chloride. The pH of the drug according to Example 7 was 7.5. Further, a drug was produced in the same manner as in Example 3 except that the NaClO₂ concentration was set to 100 mM and the benzyltriethylammonium chloride concentration was set to 1.0 mM, and the ESR spectrum of the drug was measured in the same manner as in Example 3. FIG. 26 shows the spectral diagram of the measured ESR. As shown in FIG. 26, since peaks indicating the presence of radicals appeared, it was confirmed that benzyltriethylammonium chloride served as a radical generating catalyst for sodium chlorite (NaClO₂) to generate chlorine dioxide radicals.

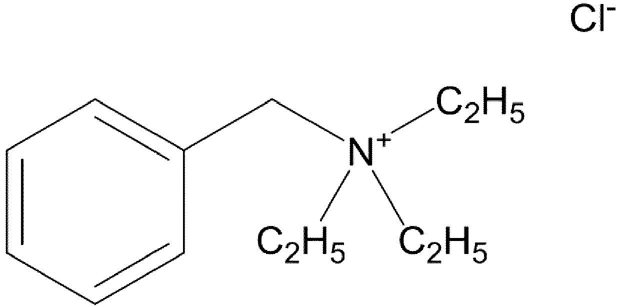

### Example 8

The drug according to Example 7 was obtained in the same manner as in Example 3 except that the same amount (mole number) of methylammonium chloride (the following chemical formula) was used as ammonium instead of benzethonium chloride. The pH of the drug according to Example 8 was 7.5. Further, a drug was produced in the same manner as in Example 3 except that the NaClO₂ concentration was set to 100 mM and the benzyltriethylammonium chloride concentration was set to 1.0 mM, and the ESR spectrum of the drug was measured in the same manner as in Example 3. As a result, since peaks indicating the presence of radicals appeared, it was confirmed that methylammonium chloride served as a radical generating catalyst for sodium chlorite (NaClO₂) to generate chlorine dioxide radicals.

### [Demonstrative data on Lewis acidity]

### (1) Measurement condition

As described above, the Lewis acidity of the ammonium salt was measured and calculated by the method described in J. Org. Chem. 2003, 68, 4720-4726. Specifically, as described in J. Org. Chem. 2003, 68, 4720-4726 (from line 21 on left on page 4724 to line 6 on left on page 4724), the reaction rate of oxidization of CoTPP with O₂ as a radical source was determined, and the Lewis acidity ΔE (eV) was calculated according to the linear relation (y = 14 (ΔE) - 8.0) (y is a common logarithm value of rate constant) given in the graph of FIG. 6 on page 4725.

### (2) Lewis acidity value

The values of Lewis acidity measured and calculated by the method (1) are shown below. Table 16 below shows the values of the Lewis acidity of the four types of ammonium in Examples 3 to 7. Table 17 below shows the values of Lewis acidity of various ammonium other than them. In addition to Tables 16 and 17 below, the Lewis acidity of pralidoxime methyl iodide (PAM) was measured in the same manner and found to be 0.60 eV.

**[Table 16]**

| **Substance name (chemical structure)** | **Lewis acidity (eV)** |
|---|---|
| **Benzethonium chloride** | **0.53** |
| 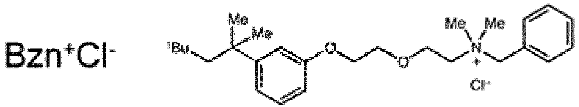 | |
| **Ammonium chloride (NH₄NCl)** | **0.66** |
| **Benzalkonium chloride** | **0.52** |
| 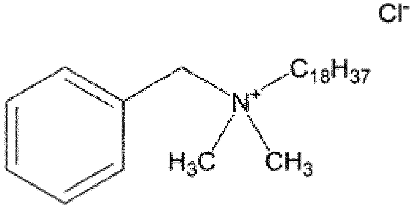 | |
| **Benzyltriethyl ammonium chloride** | **0.54** |
| 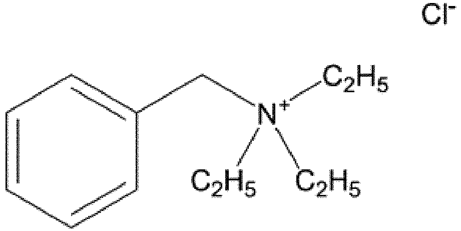 | |

**[Table 17]**

| **Substance name (chemical structure)** | **Lewis acidity (eV)** |
|---|---|
| **Choline** | **0.82** |
| **(CH₃)3N⁺(CH₂)₂OH X⁻ (X⁻ is anion)** | |
| **methylammonium chloride (MeH₃NCl)** | **0.70** |
| **Trigonelline** | **0.69** |
| 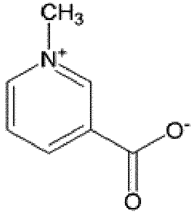 | |
| **Betaine** | **0.67** |
| **Safranine** | **0.67** |
| 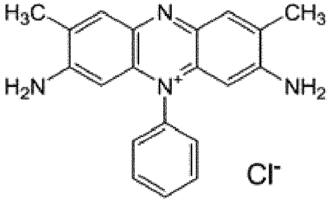 | |
| **Tetrabutylammonium chloride (Bu₄NCl)** | **0.63** |
| **Carbamylcholine** | **0.52** |
| 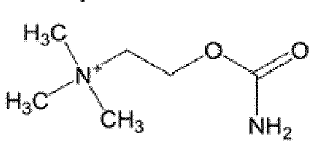 | |
| **X⁻ (X⁻ is anion)** | |
| **Acetylcholine** | **0.51** |
| 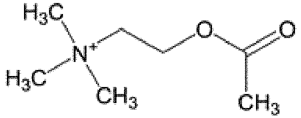 | |
| **X⁻ (X⁻ is anion)** | |
| **Tetramethylammonium chloride (Me₄NCl)** | **0.50** |

### (Experimental Example 13)

Using each of the drug according to Example 5 containing ammonium chloride, the drug according to Example 6 containing benzalkonium chloride, the drug according to Example 7 containing benzyltriethylammonium chloride, and the drug according to Example 8 containing methylammonium chloride, the sterilizing effect was examined.

The sterilizing effect was measured in the same manner as in Experimental Example 1 except that same amounts of the drug containing ammonium chloride and sodium chlorite (Example 5), the drug containing benzalkonium chloride and sodium chlorite (Example 6), or the drug containing benzyltriethylammonium chloride and sodium chlorite (Example 7) were used instead of the drug according to Example 3 (the drug containing benzethonium chloride and sodium chlorite). As the bacterial species, *Escherichia coli* MV1184 was used. In addition, using an aqueous solution containing only ammonium chloride, benzalkonium chloride, benzyltriethylammonium chloride, or methylammonium chloride and containing no sodium chlorite (comparative example) instead of the drug according to Examples 5, 6, 7, or 8, the sterilizing effect was measured by the same method.

### (2) Results of measurement of sterilizing effect

The results of minimum inhibitory concentration (MIC) measured according to the above (1) were as shown in Table 18 below.

**[Table 18]**

| | MIC (ppm) |
|---|---|
| Drug containing ammonium chloride and sodium chlorite | 10 |
| Drug containing benzalkonium chloride and sodium chlorite | 10 |
| Drug containing benzyltriethylammonium chloride and sodium chlorite | 10 |
| Drug containing methylammonium chloride and sodium chlorite | 10 |
| Aqueous solution containing only ammonium chloride and no sodium chlorite | No sterilizing effect |
| Aqueous solution containing only benzalkonium chloride and no sodium chlorite | No sterilizing effect |
| Aqueous solution containing only benzyltriethylammonium chloride and no sodium chlorite | No sterilizing effect |
| Aqueous solution containing only methylammonium chloride and no sodium chlorite | No sterilizing effect |

As shown in Table 18, when the drug containing ammonium chloride and sodium chlorite (Example 5), the drug containing benzalkonium chloride and sodium chlorite (Example 6), the drug containing benzyltriethylammonium chloride and sodium chlorite (Example 7), or the drug containing methylammonium chloride and sodium chlorite (Example 8) was used, the sterilizing effect as the drug of the present invention was confirmed. In contrast, the aqueous solution containing only ammonium chloride, benzalkonium chloride, benzyltriethylammonium chloride, or methylammonium chloride and containing no sodium chlorite had no sterilizing effect. From this, it was confirmed that the sterilizing effect was achieved by the chlorine dioxide radical generated from sodium chlorite by the catalytic action of ammonium chloride, benzalkonium chloride, benzyltriethylammonium chloride or methylammonium chloride.

### (Experimental Example 14: Suppression of symptoms of ulcerative colitis)

The pH was adjusted to pH5. 35 by adding a pH buffer to the drug according to Example 3. This drug is referred to as "MA-T" hereinafter.

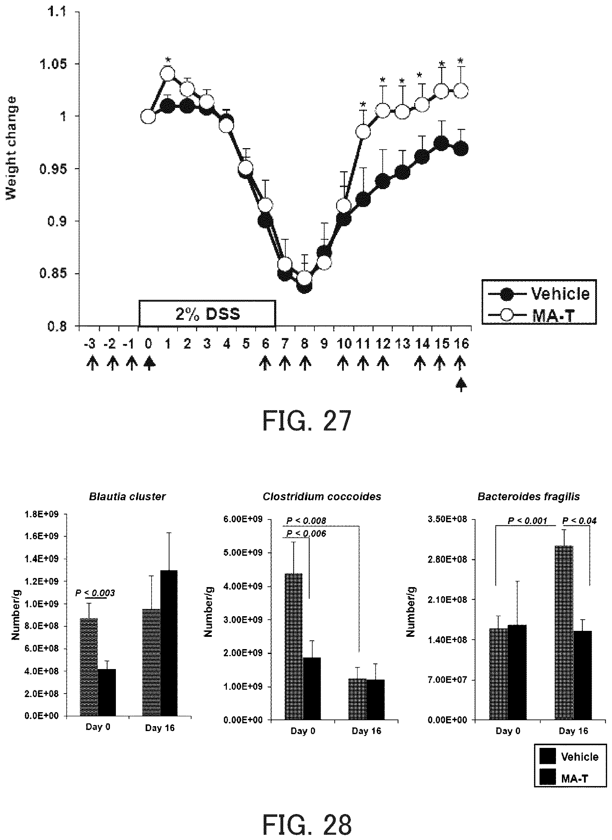

Next, C57BL/6J male mice around 12 weeks of age (housed at animal facility 5-05, Faculty of Medicine, Osaka University) were anal-administered 150 µl of MA-T (pH 5.35) or vehicle (water only) using a disposable feeding needle (ball tip diameter: 2 mm, tube diameter: 1.18 mm, tube length: 50 mm) for 3 days prior to administration of a 2% aqueous dextran sulfate solution (DSS) (MP Biomedicals) and on days 6 to 8, days 10 to 12, and days 14 to 16 after administration of DSS. During this time, 2% DSS was placed in a water bottle and allowed to drink freely. Six days after the start of 2% DSS administration, 2% DSS administration was replaced with normal water administration (water drinking). The body weights of mice were measured during the 2% DSS-drinking period and the normal water-drinking period. The body weight loss after DSS administration was analyzed with reference to the body weights of mice on the starting day of 2% DSS administration. The results obtained are shown in the graph of FIG. 27. FIG. 27 is a graph showing the *P < 0.05. (t. test) results of the male mice. The horizontal axis indicates the number of days after or before the start of DSS administration, and the vertical axis indicates the amount of change in body weight in grams. In FIG. 27, "●" represents the vehicle administration (water drinking) group, and "○" represents the MA-T administration group. As shown in FIG. 27, after the start of 2% DSS administration and until the start of MA-T or vehicle administration, the body weight of the mouse group continued to decrease. This suggests that dextran sodium sulfate-induced colitis occurred. Thereafter, when the MA-T or vehicle administration has started after the start of 2% DSS, the body weight recovered (re-increased) in both groups. This suggests that dextran sodium sulfate-induced colitis was suppressed. The MA-T administration group recovered (re-increased) more body weight than the vehicle administration group. This suggests that MA-T administration suppressed dextran-sodium sulfate-induced colitis more strongly than vehicle administration. That is, according to this experimental example, it was confirmed that MA-T was effective in suppressing dextran-sodium sulfate-induced colitis. From this, it can be inferred that MA-T has a sterilizing action (sterilizing effect). This inference was further examined in Experimental Example 15 below.

### (Experimental Example 15: changes in intestinal bacteria flora)

In Experimental Example 14 (FIG. 27), feces (3 to 5 pieces) of the vehicle administration group and the MA-T (pH 5.35) administration group were collected immediately before the DSS administration (on the day of the start of the DSS administration) and 16 days after the DSS administration. The fecal weight was measured, and then RNAlater (RNAlater (ml) = fecal weight (g) × 9) (Invitrogen) was added to prepare 10-fold diluted (v/w) fecal debris using a vortex mixer. 200 ul of fecal debris was transferred to a 2 ml screw-cap microtube, 1 ml PBS (-) was added and stirred using the vortex mixer, followed by centrifugation (13,000 × g at 4°C) for 5 minutes. The supernatant was removed, 1 ml PBS (-) was added again and stirred using the vortex mixer, centrifuged for 5 minutes (13,000 × g at 4°C), and then the supernatant was removed. 0.3g glass beads (diameter: 0.1 mm), 300 µl Tris-SDS solution (100 mM Tris-HCl, 40 mM EDTA (pH9.0), 1% SDS), and 500 µl TE-saturated phenol (nacalai) were added and stirred using FastPrep (power level: 5.0, 30 seconds) (MP Biomedicals). After centrifugation (20000 × g at 4°C) for 5 minutes, 400 µl of the supernatant was transferred to a 2 ml screw-cap microtube, and the same amount of phenol/chloroform/isoamyl alcohol (25:24:1) (nacalai) was added and stirred again using FastPrep (power level :4.0, 45 seconds). After centrifugation (20000 × g at 4°C) for 5 minutes, 250 µl of the supernatant was transferred to a 1.5 ml screw-cap microtube, and 25 µl of 3M sodium acetate (pH 5.2) and 300 µl of isopropanol (nacalai) were added and stirred using the vortex mixer, followed by centrifugation (20000 × g at 4°C) for 5 minutes. The supernatant was removed, 800 µl of 80% ethanol was added and centrifuged (20000 × g at 4°C) for 5 minutes. The supernatant was removed and the tube was placed in a 60°C block incubator for 30 minutes to dry. 200 µl of TE (pH 8.0) (nacalai) was added to dissolve the recovered nucleic acid (DNAs). Using the prepared DNA-solution and intestinal bacterial standard plasmid DNAs, the number of intestinal bacteria (*Blautia cluster, Clostridium coccoides, Bacteroides fragilis*) in per grum of faecal was analyzed by quantitative PCR ((1) at 94°C for 5 minutes, (2) at 94°C for 20 seconds, (3) at 55°C for 20 seconds, (4) at 72°C for 50 seconds, (1) 1 cycle/ (2) to (4) 45cycles) (Step One Plus, Applied Biosystems). The primer sets used in the PCR method are as follows:

### Primer sets;

Blautia cluster, 5'-gtgaaggaagaagtatctcgg-3' and 5'-ttggtaaggttcttcgcgtt-3';
Clostridium coccoides, 5'-aaatgacgggtacctgactaa-3' and 5'-ctttgagtttcattcttgcgaa-3';
Bacteroides fragilis, 5'-atagcctttcgaaagaagat-3' and 5'-ccagtatcaactgcaatttta-3'.

The detected number of each of the bacteria is shown in the bar graphs of FIG. 28. In the bar graphs, "Day 0" represents immediately before DSS administration (on the day of the start of DSS administration) and "Day 16" represents 16 days after the start of DSS administration. In each graph, the left bar represents the vehicle administration group and the right bar represents the MA-T administration group. The vertical axis represents the number of bacteria (Number) per gram (g) of feces. As shown in FIG. 28, it was confirmed that MA-T induced changes in intestinal bacteria flora. That is, from this, it was confirmed that the MA-T had a sterilizing action (sterilizing effect).

### [Examples of drug for use in agriculture and livestock industry]

Next, specific examples of a drug for use in agriculture and livestock industry will be described. However, the drug for use in agriculture and livestock industry of the present invention is not limited by the following examples. In the following description, the drug for use in agriculture and livestock industry according to the examples also may be referred to simply as the "drug" in the examples of the present invention.

The drugs according to Examples 3 to 4 and Comparative Examples 1 to 8 were used as the drugs for use in agriculture and livestock industry in the experimental examples of the drug for use in agriculture and livestock industry described below.

### (Experimental Example 1 of drug for use in agriculture and livestock industry)

In Experimental Example 1 of the drug for use in agriculture and livestock industry, the following were provided first.

Bacterial strains to be used:
*Staphylococcus aureus*
*Escherichia coli* MV1184

Bacterial solution:
Bacteria cultured in a BHI agar medium were collected with a platinum loop and placed in a BHI liquid medium, and the BHI liquid medium was shaken. The bacteria were allowed to grow in the BHI liquid medium for a whole day and night. 50 µl of the resultant culture solution was diluted 190-fold with a BHI liquid medium, and mixed well with the BHI liquid medium by stirring. The resultant mixture was used as a bacterial solution.

Using each bacterial strain and bacterial solution, the effect was examined in the following manner.

A microplate (with a lid) was sterilized for 10 minutes with a UV sterilization lamp. Next, a BHI liquid medium, the bacterial solution, and the drug for use in agriculture and livestock industry according to Example 3 were injected in this order into each well with a micropipette. The bacteria were cultured at 37°C for 24 hours. Thereafter, the bacteria were examined using a microplate reader, and the minimum inhibitory concentration (MIC) was determined. As a control, the same examination was performed using the liquid medium only. Further, 10 µl of the culture solution was collected from the well in the vicinity of the MIC, and inoculated in a petri dish. The bacteria were cultured at 37°C for 24 hours, and the minimum bactericidal concentration (MBC) was determined. The results obtained are shown in Table 19.

Using the bactericide according to Comparative Example 1 instead of the drug for use in agriculture and livestock industry according to Example 3, the MIC and MBC were determined in the same manner. The results obtained are shown in Table 19.

Using each of the sterilizing deodorizers according to Comparative Examples 2 to 5 instead of the drug for use in agriculture and livestock industry according to Example 3, the MIC of the *Staphylococcus aureus* was determined in the same manner. The results obtained are shown in Table 19.

Using the test product according to Comparative Example 6 instead of the drug for use in agriculture and livestock industry according to Example 3, the MIC of the *Staphylococcus aureus* and the MIC and MBC of the *Escherichia coli* were determined in the same manner. The results obtained are shown in Table 19.

**[Table 19]**

| | | Ex. 1 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|---|
| *S. aureus* | MIC (ppm) | 1.56 | 300 | ineffective | ineffective | ineffective | ineffective | 40 |
| | (ppm) | 3.12 | 300 | | | | | |
| *E. coli* | MIC (ppm) | 12.5 | 220 | | | | | 30 |
| | MBC (ppm) | 20.0 | 220 | | | | | 50 |

### (Experimental Example 2 of drug for use in agriculture and livestock industry)

Using the drug according to Example 4 or Comparative Example 7 or 8 instead of the drug for use in agriculture and livestock industry according to Example 3, the MIC of the *Escherichia coli* was determined in the same manner. The results obtained are shown in Table 20.

**[Table 20]**

| | | Ex. 2 | Comp. Ex. 7 | Comp. Ex. 8 |
|---|---|---|---|---|
| *E. coli* | MIC (ppm) | 12.5 > | 25 < | 17.5 |

### (Experimental Example 3 of drug for use in agriculture and livestock industry)

In Experimental Example 3 of the drug for use in agriculture and livestock industry, the following were provided first.

Bacterial strain to be used:
*Streptococcus pyogenes*

Bacterial solution:
A bacterial solution was obtained in the same manner as in Experimental Example 1 of the drug for use in agriculture and livestock industry.

Using the above bacterial strain and bacterial solution and the drug for use in agriculture and livestock industry according to Example 3, the MIC and MBC were determined in the same manner as in Experimental Example 1. The results obtained are shown in Table 21.

**[Table 21]**

| | | Ex. 1 |
|---|---|---|
| *Streptococcus pyogenes* | MIC (ppm) | 0.1 |
| | MBC (ppm) | 1.0 |

### (Experimental Example 4 of drug for use in agriculture and livestock industry)

In Experimental Example 4 of the drug for use in agriculture and livestock industry, the following were provided first.

Bacterial strains to be used:
*Streptococcus mutans*

Bacterial solution:
Bacteria cultured in a BHI agar medium were collected with a platinum loop and placed in a BHI liquid medium, and the BHI liquid medium was shaken. The bacteria were allowed to grow in the BHI liquid medium for a whole day and night. 50 µl of the resultant culture solution was diluted 190-fold with a BHI liquid medium, and mixed well with the BHI liquid medium by stirring. The resultant mixture was used as a bacterial solution.

Using each bacterial strain and bacterial solution, the effect was examined in the following manner.

The bacterial solution was injected with a micropipette into a BHI liquid medium placed in each of two test tubes. Saccharose was added thereto so that the concentration thereof was 0.2%. The bacteria were cultured at 37°C for 18 hours to allow them to form a biofilm. The medium in each test tube was discarded in a beaker, and the biofilm was washed twice with PBS. The drug for use in agriculture and livestock industry according to Example 3 was injected into one of the test tube, and PBS was injected into the other test tube. Then, the test tubes were shaken at 37°C for 30 minutes. The liquid in each test tube was discarded in a beaker, and the biofilm was washed twice with PBS. A BHI liquid medium was injected into the test tubes, and the bacteria were cultured at 37°C for 24 hours. 10 µl of the medium collected from each test tube was inoculated into a nutrient agar medium, and the bacteria were cultured at 37°C for 24 hours. The presence or absence of colonies was checked through visual observation. As a result, while no colony was observed in the test tube to which the drug for use in agriculture and livestock industry according to Example 3 had been injected, many colonies were observed in the test tube to which PBS had been injected.

In order to examine the effect of the drug on the bacterial cells in the biofilm, the following test was conducted further.

The bacterial solution was injected with a micropipette into a BHI liquid medium placed in microtubes for BioMasher. Saccharose was added thereto so that the concentration thereof was 0.2%. The bacteria were cultured at 37°C for 18 hours to allow them to form a biofilm. The medium in each microtube was discarded in a beaker, and the biofilm was washed twice with PBS. The drug for use in agriculture and livestock industry according to Example 3 was injected into one of the microtubes, and PBS was injected into the other microtube. The bacteria in the former microtube and the bacteria in the latter microtube were aged at 37°C for 15 minutes and 30 minutes, respectively. The liquid in each microtube was discarded in a beaker, and the biofilm was washed twice with PBS. A BHI liquid medium was injected into the test tubes and homogenized. Thereafter, the bacteria were cultured at 37°C for 24 hours. 10 µl of the medium collected from each microtube was inoculated into a nutrient agar medium, and the bacteria were cultured at 37°C for 24 hours. The presence or absence of colonies was checked through visual observation. As a result, while no colony was observed in the microtube to which the drug for use in agriculture and livestock industry according to Example 3 had been injected, many colonies were observed in the microtube to which PBS had been injected. These results demonstrate that, by impregnating a biofilm with the drug for use in agriculture and livestock industry according to Example 3, the drug acts on bacteria deep inside the biofilm to exhibit the sterilizing effect.

### (Experimental Example 5 of drug for use in agriculture and livestock industry)

In Experimental Example 5 of the drug for use in agriculture and livestock industry, the following bacterial strains were used. Except for this, the MIC and MBC were determined using the drug for use in agriculture and livestock industry according to Example 3 in the same manner as in Experimental Example 1 of the drug for use in agriculture and livestock industry. The results obtained are shown in Table 22.

Bacterial strains to be used:
Bacteria 1 (*Porphyromonas gingivalis*)
Bacteria 2 (*Treponema denticola*)
Bacteria 3 (*Tannerella forsythensis*)
Bacteria 4 (*Aggregatibacter actinomycetemcomitans*)

**[Table 22]**

| | | Ex. 1 |
|---|---|---|
| Bacteria 1 | MIC (ppm) | 20.0 |
| | MBC (ppm) | 20.0 |
| Bacteria 2 | MIC (ppm) | 25.0 |
| | MBC (ppm) | 25.0 |
| Bacteria 3 | MIC (ppm) | 12.5 |
| | MBC (ppm) | 12.5 |
| Bacteria 4 | MIC (ppm) | 35-45 |
| | MBC (ppm) | 35-50 |

### (Experimental Example 6 of drug for use in agriculture and livestock industry)

Test pieces (25.4 mm × 25.4 mm) respectively made of iron, aluminum, tin plate, and stainless steel were washed. Thereafter, the test pieces made of each material were immersed in resin containers containing the drug for use in agriculture and livestock industry according to Example 3, a 1.2% sodium hypochlorite aqueous solution, and tap water, respectively, and then, the resin containers were covered with a lid. The test pieces were taken out on a nonwoven fabric after a lapse of each time period shown in Tables 23 and 24, and the conditions of the test pieces were examined through visual observation. In the examination, pictures were taken when necessary, and a microscope was used when the change was subtle. The evaluation was made using the following evaluation criteria.

- -:: no corrosion
- ±:: generation of rust
- +:: fairly large amount of rust
- ++:: vary large amount of rust
- +++:: corrosion of metal surfaces

**[Table 23]**

| Test piece | | 10 min | 30 min | 1 hr | 3 hr | 6 hr |
|---|---|---|---|---|---|---|
| Iron | Example 1 | ± | ± | ± | + | + |
| | Hypochlorous acid | + | + | ++ | +++ | +++ |
| | Tap water | ± | ± | ± | | + |
| Aluminum | Example 1 | - | - | - | - | - |
| | Hypochlorous acid | ± | ± | ± | + | + |
| | Tap water | - | - | - | - | - |
| Tin plate | Example 1 | - | - | - | - | - |
| | Hypochlorous acid | - | - | - | ± | ± |
| | Tap water | - | - | - | - | - |
| Stainless steel | Example 1 | - | - | - | - | - |
| | Hypochlorous acid | - | - | - | - | - |
| | Tap water | - | - | - | - | - |

**[Table 24]**

| Test piece | | 1 day | 3 days | 1 week | 2 weeks | 3 weeks | 4 weeks |
|---|---|---|---|---|---|---|---|
| Iron | Example 1 | + | + | + | + | + | + |
| | Hypochlorous acid | +++ | +++ | +++ | +++ | +++ | +++ |
| | Tap water | + | + | + | + | + | + |
| Aluminum | Example 1 | - | - | - | - | - | - |
| | Hypochlorous acid | ++ | ++ | ++ | +++ | +++ | +++ |
| | Tap water | - | - | - | - | - | - |
| Tin plate | Example 1 | - | ± | ± | ± | ± | ± |
| | Hypochlorous acid | + | ++ | +++ | +++ | +++ | +++ |
| | Tap water | - | ± | ± | + | + | + |
| Stainless steel | Example 1 | - | - | - | - | - | - |
| | Hypochlorous acid | - | - | - | - | - | - |
| | Tap water | - | - | - | - | - | - |

### (Experimental Example 7 of drug for use in agriculture and livestock industry)

The deodorizing performance test was conducted in accordance with JEM 1467 "domestic air cleaner" in the Standards of the Japan Electrical Manufacturers' Association. In the measurement, cigarettes were burned while operating a circulator in an acrylic container (1 m in height × 1 m in width × 1 m in depth) with an internal volume of 1 m³ to fill the container with smoke. After all the cigarettes were burned, the circulator was stopped, and the drug for use in agriculture and livestock industry according to Example 3 was sprayed in the container by operating a sprayer. The concentrations of three components, namely, ammonia, acetaldehyde, and acetic acid, in the container were measured over 2 hours at regular intervals to trace the change in concentrations. Similarly, formaldehyde vapor was injected into an acrylic container and the formaldehyde concentration in the container was measured over 2 hours at regular intervals to trace the change in concentration. The sprayer was operated in "Manual" mode. As a control, a blank test in which the sprayer was not operated was also conducted. The results obtained are shown in Tables 25 to 28. The malodorous components were measured using detector tubes (Gastec Corporation). The detector tubes used for the measurement are shown below.

Detector tubes used for measurement

| | |
|---|---|
| ammonia: | No. 3L |
| acetaldehyde: | No. 92L |
| acetic acid: | No. 81L |
| formaldehyde: | No. 91 |

**[Table 25]**

| Elapsed time | Ammonia concentration (ppm) | | Example 1 |
|---|---|---|---|
| | Example 1 | Blank test | Removal rate (%) |
| Start | 30 | 32 | - |
| 5 min | 8 | 32 | 73 |
| 10 min | 5 | 32 | 83 |
| 20 min | 4 | 32 | 87 |
| 30 min | 2 | 32 | 93 |
| 45 min | 1 | 31 | 97 |
| 60 min | 1 > | 31 | 97 < |
| 90 min | 1 > | 31 | 97 < |
| 120 min | 1 > | 26 | 97 < |

**[Table 26]**

| Elapsed time | Acetaldehyde concentration (ppm) | | Example 1 |
|---|---|---|---|
| | Example 1 | Blank test | Removal rate (%) |
| Start | 14 | 14 | - |
| 5 min | 12 | 14 | 14 |
| 10 min | 10 | 14 | 29 |
| 20 min | 10 | 14 | 29 |
| 30 min | 7 | 14 | 50 |
| 45 min | 7 | 14 | 50 |
| 60 min | 7 | 14 | 50 |
| 90 min | 7 | 14 | 50 |
| 120 min | 6 | 14 | 57 |

**[Table 27]**

| Elapsed time | Acetic acid concentration (ppm) | | Example 1 |
|---|---|---|---|
| | Example 1 | Blank test | Removal rate (%) |
| Start | 12 | 10 | - |
| 5 min | 0.5 > | 10 | 96 < |
| 10 min | 0.5 > | 10 | 96 < |
| 20 min | 0.5 > | 10 | 96 < |
| 30 min | 0.5 > | 10 | 96 < |
| 45 min | 0.5 > | 10 | 96 < |
| 60 min | 0.5 > | 9.5 | 96 < |
| 90 min | 0.5 > | 9.0 | 96 < |
| 120 min | 0.5 > | 9.0 | 96 < |

**[Table 28]**

| Elapsed time | Formaldehyde concentration (ppm) | | Example 1 |
|---|---|---|---|
| | Example 1 | Blank test | Removal rate (%) |
| Start | 20 | 20 | - |
| 5 min | 10 | 20 | 50 |
| 10 min | 8 | 20 | 60 |
| 20 min | 5 | 20 | 75 |
| 30 min | 3 | 20 | 85 |
| 45 min | 2 | 20 | 90 |
| 60 min | 2 | 20 | 90 |
| 90 min | 2 | 18 | 90 |
| 120 min | 2 | 18 | 90 |

### (Experimental Example 8 of drug for use in agriculture and livestock industry)

The drug for use in agriculture and livestock industry according to Example 3 was sprayed using a sprayer to measure the deodorizing performance for cigarette odor. First, cigarettes were burned in a room with a 6-tatami mat size to fill the room with smoke at a predetermined concentration. Next, a sprayer was set in the room, and the odor intensity in the room was measured three times, namely, before operating the sprayer, one hour after operating the sprayer, and two hours after operating the sprayer. The sprayer was set near a wall in the room, and the odor was collected at a height of 1 m in the middle of the room. Two circulation fans were set in the room, and they were operated at all times to maintain the air-circulating conditions. The sprayer was operated in "Manual" mode. As a control, a blank test in which the sprayer was not operated was also conducted. The odor intensity was determined as follows according to the six-grade odor intensity measurement method. The results obtained are shown in Table 29.

The odor intensity was evaluated by six testers (test panel). The results were calculated by determining the average value of the odor intensities given by the respective testers. The six-grade odor intensity measurement method is a method for converting odor intensity to a numerical value using human olfaction. The members of the test panel who had joined the test were those who had taken the legally-required olfactometry and had been admitted as having normal olfaction.

In the six-grade odor intensity measurement method, the following numerical values are used as evaluation criteria.
- 0:: odorless
- 1:: barely perceivable odor (detection threshold concentration)
- 2:: weakly perceivable odor (recognition threshold concentration)
- 3:: easily perceivable odor
- 4:: strong odor
- 5:: very strong odor

**[Table 29]**

| Elapsed time | Odor intensity | |
|---|---|---|
| | Example 1 | Blank test |
| Start | 4.6 | 4.5 |
| 1 h | 3.5 | 4.5 |
| 2 h | 2.9 | 4.2 |

### (Experimental Example 9 of drug for use in agriculture and livestock industry)

The drug for use in agriculture and livestock industry according to Example 3 was sprayed with a sprayer to measure the performance thereof to remove airborne bacteria (general bacteria, fungi). First, a sprayer was set in a room with a 6-tatami mat size, and the concentration of airborne bacteria in the air was measured three times, namely, before operating the sprayer, one hour after operating the sprayer, and two hours after operating the sprayer. The sprayer was set near a wall in the room, and the airborne bacteria were collected at a height of 1 m in the middle of the room. Two circulation fans were set in the room, and they were operated at all times to maintain the air-circulating conditions. The airborne bacteria were measured by a filtration method using a membrane filter. The sprayer was operated in "Manual" mode. As a control, a blank test in which the sprayer was not operated was also conducted. The results obtained are shown in Tables 30 and 31.

### Measurement conditions etc. in Experimental Example 9 of drug for use in agriculture and livestock industry

| | |
|---|---|
| Filter to be used: | Toyo Roshi Kaisha, Ltd., 37 mm Monitors |
| Amount of sucked air: | 300 l (sucked for 15 minutes at 20 l/min) |
| Medium to be used: | m-TGE Broth liquid medium for general bacteria (Toyo Seisakusho Kaisha, Ltd.) |
| | m-Green Y & M Broth liquid medium for fungi (Toyo Seisakusho Kaisha, Ltd.) |
| Culture conditions: | 30°C for 72 hours for general bacteria |
| | 30°C for 5 days for fungi |

**[Table 30]**

| Elapsed time | The number of airborne general bacteria (the number of bacteria/300 l) | | Example 1 |
|---|---|---|---|
| | Example 1 | Blank test | Removal rate (%) |
| Start | 13 | 11 | - |
| 1 h | 0 | 11 | 100 |
| 2 h | 0 | 11 | 100 |

**[Table 31]**

| Elapsed time | The number of airborne fungi (the number of fungi/300 l) | | Example 1 |
|---|---|---|---|
| | Example 1 | Blank test | Removal rate (%) |
| Start | 10 | 11 | - |
| 1 h | 0 | 10 | 100 |
| 2 h | 0 | 9 | 100 |

### (Experimental Example 10 of drug for use in agriculture and livestock industry)

In Experimental Example 10 of the drug for use in agriculture and livestock industry, the following bacterial strains were used. Except for this, the MIC or MBC was determined using the drug for use in agriculture and livestock industry according to Example 3 in the same manner as in Experimental Example 1 of the drug for use in agriculture and livestock industry. The results obtained are shown in Table 32.

Bacterial strains to be used:
*Streptococcus mutans*
*hemolytic streptococcus*
*Bacillus subtilis*
methicillin-resistant *Staphylococcus aureus* (MRSA)

**[Table 32]**

| | | Example 1 |
|---|---|---|
| *Streptococcus mutans* | MIC (ppm) | 5 |
| | MBC (ppm) | 15 |
| *Hemolytic streptococcus* | MIC (ppm) | 0.1 |
| | MBC (ppm) | 1.0 |
| *Bacillus subtilis* | MIC (ppm) | 12.5 |
| | MBC (ppm) | |
| MRSA | MIC (ppm) | 2 |
| | MBC (ppm) | |

### (Experimental Example 11 of drug for use in agriculture and livestock industry)

Using the drug for use in agriculture and livestock industry according to Example 3, a deodorization test was performed in accordance with an instrumental analysis implementation manual; a detector tube method, a gas chromatography method (the Certification Standards of Antibacterial Finished Textile Products of Japan Textile Evaluation Technology Council were applied with necessary modifications). The results obtained are shown in Table 33.

**[Table 33]**

| Odor component | Impression of odor | Concentration 1 (ppm) | Concentration 2 (ppm) | Gas reduction rate (%) |
|---|---|---|---|---|
| ammonia | excrement | 100 | 7 | 93 |
| acetic acid | vinegar | 50 | 1 | 98 |
| hydrogen sulfide | rotten egg | 4.00 | 0.12 | 97 |
| methyl mercaptan | rotten onion | 8.00 | 4.96 | 38 |
| tritylamine | rotten fish | 28.00 | 3.08 | 89 |
| isovaleric acid | musty socks | 38.00 | 0.38 | 99 |

| | | | | |
|---|---|---|---|---|
| Concentration 1: initial gas concentration Concentration 2: gas concentration after a lapse of 2 hours Gas reduction rate: ([concentration 1 - concentration 2]/concentration 1) × 100 | | | | |

### (Experimental Example 12 of drug for use in agriculture and livestock industry)

The drug for use in agriculture and livestock industry according to the present invention was administered to mice in order to examine whether the drug for use in agriculture and livestock industry according to the present invention is highly safe.

Using the drug for use in agriculture and livestock industry according to Example 3, an acute oral toxicity test was performed on mice in accordance with OECD TG 420 (fixed dose method). The test was conducted by Japan Food Research Laboratories. As a result, the LD50 value of the drug for use in agriculture and livestock industry was 2000 mg/kg or more in both the female and male mice. This result demonstrates that the drug for use in agriculture and livestock industry according to the present invention is highly safe.

### (Experimental Example 13 of drug for use in agriculture and livestock industry)

The drug for use in agriculture and livestock industry according to the present invention was administered to rabbits in order to examine whether the drug for use in agriculture and livestock industry according to the present invention is highly safe.

Using the drug for use in agriculture and livestock industry according to Example 3, an eye irritation test was performed on rabbits in accordance with OECD TG 405 Acute Eye Irritation/Corrosion. The test was conducted by Japan Food Research Laboratories. As a result, it was found that the drug for use in agriculture and livestock industry was non-irritating. This result demonstrates that the drug for use in agriculture and livestock industry according to the present invention is highly safe.

### (Experimental Example 14 of drug for use in agriculture and livestock industry)

The drug for use in agriculture and livestock industry according to the present invention was administered to rabbits in order to examine whether the drug for use in agriculture and livestock industry according to the present invention is highly safe.

Using the drug for use in agriculture and livestock industry according to Example 3, a primary skin irritation test was performed on rabbits in accordance with OECD TG 404 Acute Skin Irritation/Corrosion. The test was conducted by Japan Food Research Laboratories. As a result, it was found that the drug for use in agriculture and livestock industry was slightly irritating. This result demonstrates that the drug for use in agriculture and livestock industry according to the present invention is highly safe.

### (Experimental Example 15 of drug for use in agriculture and livestock industry)

The drug for use in agriculture and livestock industry according to the present invention was administered to guinea pigs in order to examine whether the drug for use in agriculture and livestock industry according to the present invention is highly safe.

Using the drug for use in agriculture and livestock industry according to Example 3, a continuous skin irritation test was performed on guinea pigs by applying the drug for use in agriculture and livestock industry on their skin for 14 consecutive days. The test was conducted by Life Science Laboratories, Ltd. As a result, it was found that the drug for use in agriculture and livestock industry was non-irritating. This demonstrates that the drug for use in agriculture and livestock industry according to the present invention is highly safe.

### (Experimental Example 16 of drug for use in agriculture and livestock industry)

The drug for use in agriculture and livestock industry according to the present invention was administered to guinea pigs in order to examine whether the drug for use in agriculture and livestock industry according to the present invention is highly safe.

Using the drug for use in agriculture and livestock industry according to Example 3, a skin sensitization test was performed on guinea pigs by the maximization test method. The test was conducted by Life Science Laboratories, Ltd. As a result, it was found that the drug for use in agriculture and livestock industry did not cause skin sensitization. This result demonstrates that the drug for use in agriculture and livestock industry according to the present invention is highly safe.

### (Experimental Example 17 of drug for use in agriculture and livestock industry)

The drug for use in agriculture and livestock industry according to the present invention was administered to human in order to examine whether the drug for use in agriculture and livestock industry according to the present invention is highly safe.

Using the drug for use in agriculture and livestock industry according to Example 3, a human patch test was conducted by attaching patches impregnated with the drug for use in agriculture and livestock industry to human for 24 hours. The test was conducted by Life Science Laboratories, Ltd. As a result, it was found that the drug for use in agriculture and livestock industry was non-irritating. This result demonstrates that the drug for use in agriculture and livestock industry according to the present invention is highly safe.

### (Experimental Example 18 of drug for use in agriculture and livestock industry)

The present example examined whether the drug for use in agriculture and livestock industry according to the present invention can inhibit the occurrence of rice blast.

Seeds of Koshihikari (rice cultivar) were subjected to seed selection with a salt solution, and diseased seeds were removed by removing floating seeds. The thus-selected seeds were washed with water, drained, and packed in a coarse saran fiber bag. Next, a diluted solution was prepared by diluting the drug for use in agriculture and livestock industry according to Example 3 200-fold (also referred to as "200-fold dilution" hereinafter). Then, the seeds packed in the saran fiber bag were immersed in the 200-fold dilution twice as heavy as the seeds for 24 hours. During the immersion treatment, water replacement was not performed. After the immersion treatment, the seeds were air-dried, and then subjected to the immersion treatment again for 6 days. During the immersion treatment, water replacement was not performed. After the immersion treatment, the seeds were further subjected to the immersion treatment again for 6 days.

Next, the seeds were seeded in seedling boxes, and further, the 200-fold dilution was sprayed (500 ml/seedling box). Thereafter, the seeds were grown. The obtained seedlings were planted in a rice field, and cultivated by an ordinary method. Then, occurrence of rice blast during the cultivation was examined. As a control, the occurrence of rice blast was examined in the same manner, except that seeds of Hitomebore (rice cultivar) were used instead of the seeds of Koshihikari, the immersion treatments in the 200-fold dilution were not performed, and seedlings of Hitomebore were planted in a rice field adjacent to the rice field where the seedlings of Koshihikari were planted.

As a result, the occurrence of rice blast was not observed in the rice field where the seedlings obtained from the seeds subjected to the immersion treatments with the 200-fold dilution were planted. In contrast, in the control, the occurrence of rice blast was observed. These results demonstrate that the drug for use in agriculture and livestock industry according to the present invention can inhibit the occurrence of rice blast.

### (Experimental Example 19 of drug for use in agriculture and livestock industry)

The present example examined whether the drug for use in agriculture and livestock industry according to the present invention can restrict the spread of rice blast.

A rice field with a high incidence of rice blast was plowed and irrigated while adding a diluted solution obtained by diluting the drug for use in agriculture and livestock industry according to Example 3 10-fold (also referred to as "10-fold dilution" hereinafter) to the rice field (10 l of the 10-fold dilution per 10 a of the rice field). Next, the seedlings of Koshihikari used in Experimental Example 18 of the drug for use in agriculture and livestock industry were planted in a rice field after being plowed and irrigated, and cultivated. Then, when the occurrence of rice blast was observed during the cultivation, stalks infected with rice blast were removed, and the 10-fold dilution was sprayed around areas where the stalks infected with rice blast had been cultivated (1 l of the 10-fold dilution per 10 a of the rice field). As a control, instead of the seedlings of Koshihikari obtained in Experimental Example 18 of the drug for use in agriculture and livestock industry, the control seedlings in Experimental Example 18 of the drug for use in agriculture and livestock industry were cultivated in the same manner, except that the control seedlings were planted in a rice field adjacent to the rice field where the seedlings of Koshihikari were planted, without adding or spraying the 10-fold dilution to the rice field. Then, whether the rice blast that had occurred in the rice field where the control seedlings were planted spread out to the rice field where the seedlings of Koshihikari were planted during the cultivation was examined.

As a result, in the rice field where the control seedlings were planted, the occurrence and spread of rice blast were observed. In contrast, in the rice field where the seedlings of Koshihikari were planted, while the occurrence of rice blast was observed slightly above primary rachis-branches in an area within about 2 to 3 m from the boundary to the rice field where the control seedlings were planted, the spread of the rice blast to the remaining area of the rice field was not observed. These results demonstrate that the drug for use in agriculture and livestock industry according to the present invention can restrict the spread of rice blast.

### (Experimental Example 20 of drug for use in agriculture and livestock industry)

The present example examined whether the drug for use in agriculture and livestock industry according to the present invention can repel shield bugs and pest insects.

The seedlings of Koshihikari obtained in Experimental Example 18 of the drug for use in agriculture and livestock industry were planted in rice fields owned by 23 farmers and cultivated in the same manner as in the Experimental Example 19 of the drug for use in agriculture and livestock industry, except that the rice fields were treated one by one by the respective farmers. Then, after the cultivation, each of the farmers was interviewed about the extent to which shield bugs and pest insects approached the rice field as compared with previous years.

As a result, eight farmers commented that repelling of shield bugs and pest insects was observed. These results demonstrate that the drug for use in agriculture and livestock industry according to the present invention can repel shield bugs and pest insects.

### [Examples of amino acids, peptides, and phospholipids]

Examples of amino acids, peptides, and phospholipids will be described below.

### [Reference Example 6]

The reaction rate constants of various amino acids, peptides, and phospholipids were measured by the above-described "Lewis acidity measuring method (2)" as described below. In the table below, "L-aspartate" means L-aspartate, "L-glutamate" means L-glutamate, "L-glycine" means L-glycine, "L-lysine" means L-lysine, "L-arginine" means L-arginine, "GSSG" means oxidized glutathione, "Cys-Cys" means cystine, "DPPS" means dipalmitoyl phosphatidylserine, "DPPC" means dipalmitoyl phosphatidylcholine, and "adenine" represents adenine (Note from translator: in the original text in Japanese, the above sentence explains the meanings of the English terms in the table in Japanese). Also, "K_{obs}" represents a measured value of the reaction rate constant (k_{cat}). As shown in the table below, it was confirmed that all amino acids, peptides, and phospholipids exhibited Lewis acidity.

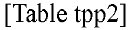

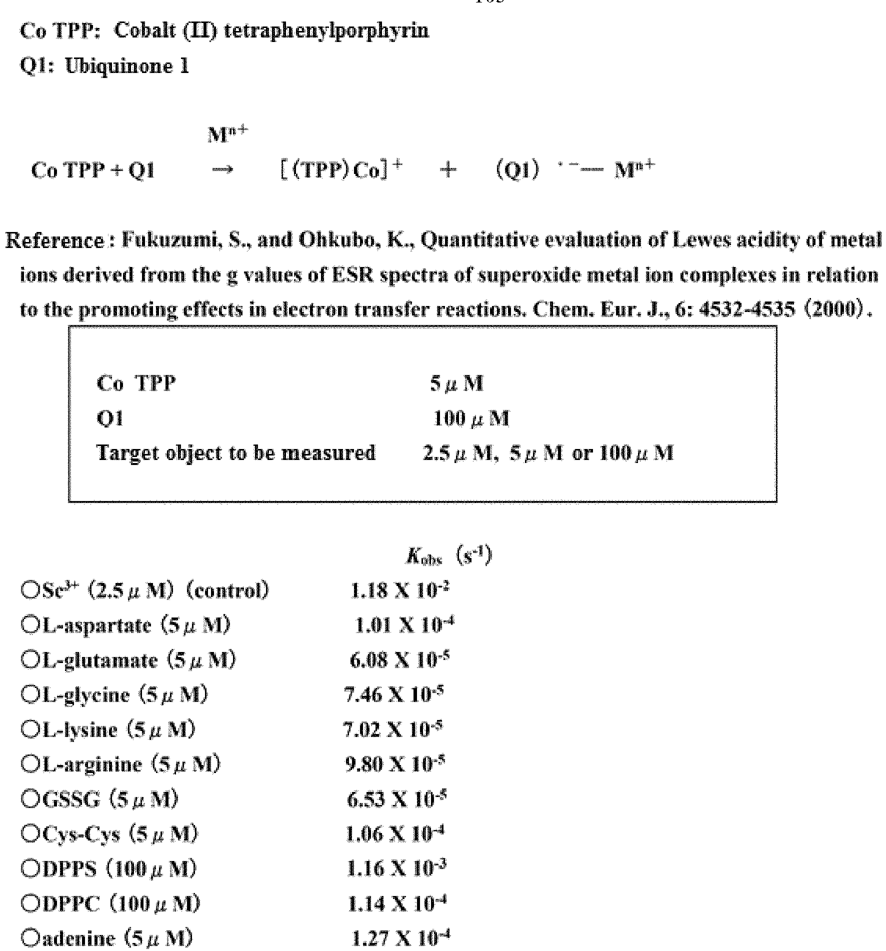

### [Reference Example 7]

The Lewis acidity of each of phosphatidylserine, phosphatidylcholine, phosphatidic acid, phosphatidylethanolamine, phosphatidylglycerol, cardiolipin, L-aspartate, and L-serine was measured by "Lewis acidity measuring method (1)" or "Lewis acidity measuring method (2)". As a result, as to phosphatidylserine, phosphatidylcholine, and L-aspartate, the Lewis acidity was confirmed by both "Lewis acidity measuring method (1)" and "Lewis acidity measuring method (2)". As to phosphatidic acid, the Lewis acidity was confirmed by "Lewis acidity measuring method (2)". As to cardiolipin, the Lewis acidity was confirmed by "Lewis acidity measuring method (1)". In addition, as to gangliosides GM1 and sphingomyelin, which are sphingolipids (phospholipids), the Lewis acidity was confirmed by the "Lewis acidity measuring method (2)".

### [Example 9]

Chlorine dioxide radicals were produced from chlorite or a salt thereof (sodium chlorite) using various amino acids, peptides, and phospholipids whose Lewis acidity was measured in Reference Examples 6 and 7. This demonstrates that the various amino acids, peptides, and phospholipids serve as chlorine dioxide radical generating catalysts for chlorite or a salt thereof (sodium chlorite).

### [Example 10]

Using the various ammonium, drugs were produced in the same manner as in Examples 3 to 8. Further, drugs were produced in the same manner as in Examples 3 to 8 except that the various amino acids, peptides, and phospholipids whose Lewis acidity was measured in Reference Examples 6 and 7 were used instead of the various ammonium. Then, these drugs were used in the same manner as the experimental examples of the drugs and the experimental examples of the drugs for use in agriculture and livestock industry in order to examine whether these drugs have a sterilizing action and the like.

The preparation of the drug was carried out as follows. First, sodium chlorite was dissolved in purified water to obtain an aqueous solution of sodium chlorite (solution A). On the other hand, each of the various ammonium, amino acids, peptides, and phospholipids was dissolved in purified water to obtain an aqueous solution (solution B). Further, a solution A having a concentration of 1 mM and a solution B having a concentration of 0.2 mM were mixed, or a solution A having a concentration of 5 mM and a solution B having a concentration of 1 mM were mixed and further diluted to obtain a drug. The concentration of each ammonium, amino acid, peptide, or phospholipid in the drug was 12.5 to 20 ppm. The concentration of sodium chlorite in the drug was about 0.14 mM to 0.22 mM.

Bacterial strains to be used:
*Escherichia coli* MV1184

Bacterial solution:
Bacteria cultured in a BHI agar medium were collected with a platinum loop and placed in a BHI liquid medium, and the BHI liquid medium was shaken. The bacteria were allowed to grow in the BHI liquid medium for a whole day and night. 50 µl of the resultant culture solution was diluted 190-fold with a BHI liquid medium, and mixed well with the BHI liquid medium by stirring. The resultant mixture was used as a bacterial solution.

Using each bacterial strain and bacterial solution, the effect (sterilizing effect) was examined in the following manner.

A microplate (with a lid) was sterilized for 10 minutes with a UV sterilization lamp. Next, a BHI liquid medium, the bacterial solution, and the drug were injected in this order into each well with a micropipette. The bacteria were cultured at 37°C for 24 hours. Thereafter, the bacteria were examined using a microplate reader, and the minimum inhibitory concentration (MIC) was determined. As a control, the same examination was performed using the liquid medium only. Further, 10 µl of the culture solution was collected from the well in the vicinity of the MIC, and inoculated in a petri dish. The bacteria were cultured at 37°C for 24 hours, and the minimum bactericidal concentration (MBC) was determined. Further, as a control, the effect (sterilizing effect) was examined in the same manner using only solution A (aqueous solution of sodium chlorite only; sodium chlorite concentration: 40 ppm, about 0.44 mM) or only solution B (aqueous solution of ammonium, amino acid, peptide, or phospholipid only) instead of the drug.

As a result of examining the sterilizing action as described above, it was confirmed that each of methylammonium chloride, ammonium chloride, tetrabutylammonium chloride, benzethonium chloride, and benzalkonium chloride, which are ammonium; glycine, L-serine, L-aspartate, and proline, which are amino acids; oxidized glutathione, which is peptide; and choline, which is phospholipid, has a sterilizing action (sterilizing effect).

On the other hand, in the case of using only solution A or only solution B, there was no sterilizing action (sterilizing effect). That is, it can be inferred that, although sodium chlorite alone or the above-described ammonium, amino acid, peptide, or phospholipid alone does not exhibit a sterilizing effect, when both of them exist together, the ammonium, amino acid, peptide, or phospholipid acts on sodium chlorite to generate chlorine dioxide radicals, thereby exhibiting a sterilizing effect.

While the present invention has been described above with reference to illustrative embodiments and examples, the present invention is by no means limited thereto. Various changes and modifications that may become apparent to those skilled in the art may be made in the configuration and specifics of the present invention without departing from the scope of the present invention.

### Industrial Applicability

As specifically described above, according to the radical generating catalyst and radical production method of the present invention, it is possible to generate (produce) radicals under mild conditions. The radical generating catalyst and radical production method of the present invention can be used in, for example, the oxidation reaction product production method of the present invention. The oxidation reaction product production method of the present invention is applicable to oxidation reactions of various substances to be oxidized, including organic compounds and inorganic substances, and has a wide range of application potential. Further, the use of the radical generating catalyst and radical production method of the present invention is not limited to the oxidation reaction product production method of the present invention, and they are applicable to a wide variety of uses.

According to the present invention, it is possible to provide a drug and a drug for use in agriculture and livestock industry that are highly safe and have a high sterilizing effect. The use of the drug and the drug for use in agriculture and livestock industry of the present invention is not particularly limited, and they are applicable to a wide variety of uses. The drug and the drug for use in agriculture and livestock industry of the present invention is very useful in the fields of agriculture, livestock industry, etc., for example.

## Claims

1. A radical generating catalyst comprising:
at least one selected from the group consisting of amino acids, proteins, peptides, phospholipids, and salts thereof.

2. The radical generating catalyst according to claim 1, further comprising:
ammonium.

3. The radical generating catalyst according to claim 2, wherein
the ammonium is an ammonium salt represented by the following chemical formula (XI): 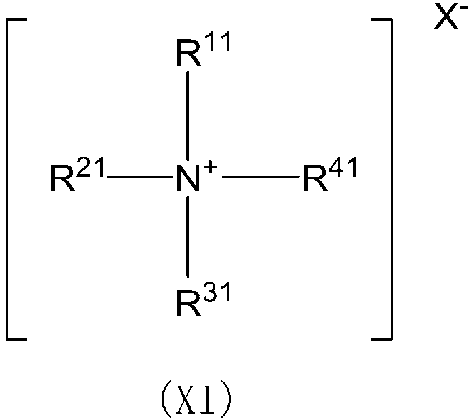 where in the chemical formula (XI),
R¹¹, R²¹, R³¹, and R⁴¹ are each a hydrogen atom or an aromatic ring, or an alkyl group, the alkyl group may comprise an ether bond, a carbonyl group, an ester bond, or an amide bond, or an aromatic ring, and R¹¹, R²¹, R³¹, and R⁴¹ may be identical to or different from each other, or
two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents, and
X⁻ is an anion (excluding peroxodisulfate ion).

4. The radical generating catalyst according to claim 3, wherein
the ammonium salt represented by the chemical formula (XI) is an ammonium salt represented by the following chemical formula (XII): 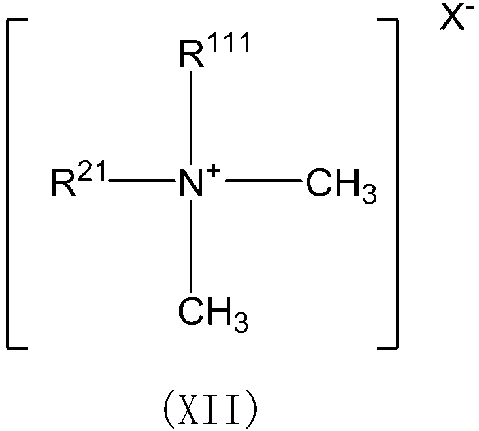 where in the chemical formula (XII),
R¹¹¹ is an alkyl group having 5 to 40 carbon atoms and may comprise an ether bond, a ketone (carbonyl group), an ester bond, or an amide bond, a substituent, or an aromatic ring, and
R²¹ and X⁻ are the same as those in the chemical formula (XI).

5. The radical generating catalyst according to claim 4, wherein
the ammonium salt represented by the chemical formula (XII) is an ammonium salt represented by the following chemical formula (XIII): 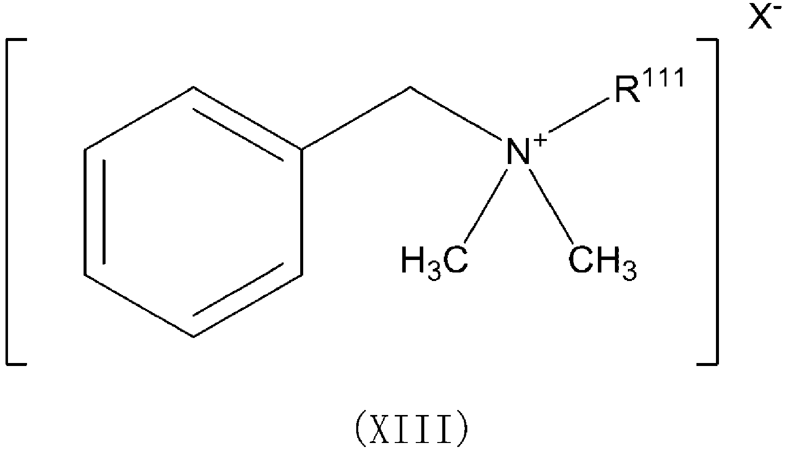 where in the chemical formula (XIII),
R¹¹¹ and X⁻ are the same as those in the chemical formula (XII).

6. The radical generating catalyst according to claim 4, wherein
the ammonium is at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines.

7. The radical generating catalyst according to claim 4, wherein
the ammonium salt represented by the chemical formula (XII) is benzethonium chloride.

8. The radical generating catalyst according to claim 3, wherein
the ammonium salt represented by the chemical formula (XI) is an ammonium salt represented by the following chemical formula (XIV): 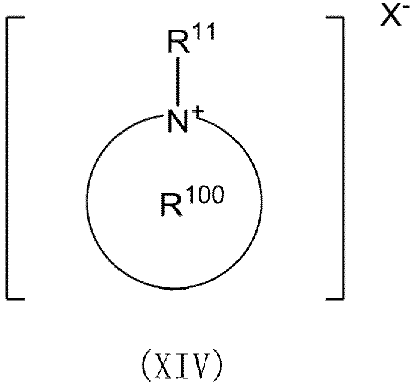 where in the chemical formula (XIV),
R¹⁰⁰ may form a ring structure, which may be saturated or unsaturated, aromatic or non-aromatic, and may or may not have one or more substituents, and
R¹¹ and X⁻ are the same as those in the chemical formula (XI).

9. The radical generating catalyst according to claim 3, wherein
the ammonium salt represented by the chemical formula (XI) is an ammonium salt represented by the following chemical formula (XV): 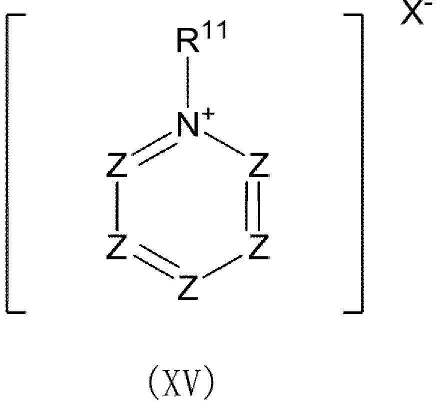 where in the chemical formula (XV),
Zs are each CH or N, may be identical to or different from each other, and in the case of CH, H may be substituted with a substituent, and
R¹¹ and X⁻ are the same as those in the chemical formula (XI).

10. The radical generating catalyst according to claim 3, wherein
the ammonium salt represented by the chemical formula (XI) is an ammonium salt represented by the following chemical formula (XVI): 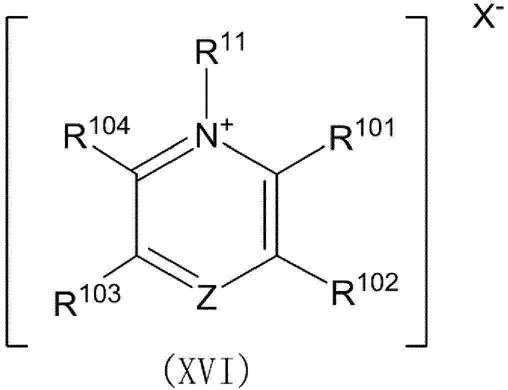 where in the chemical formula (XVI),
R¹⁰¹, R¹⁰², R¹⁰³, and R¹⁰⁴ are each a hydrogen atom or a substituent, and R¹⁰¹, R¹⁰², R¹⁰³, and R¹⁰⁴ may be identical to or different from each other, or
two or more of R¹⁰¹, R¹⁰², R¹⁰³, and R¹⁰⁴ may be integrated to form a ring structure with N⁺ to which they are bonded, and the ring structure may be saturated or unsaturated, aromatic or non-aromatic, and may or may not have one or more substituents,
Z is CH or N, and in the case of CH, H may be substituted with a substituent, and
R¹¹ and X⁻ are the same as those in the chemical formula (XI).

11. The radical generating catalyst according to claim 3, wherein
the ammonium salt represented by the chemical formula (XI) is an ammonium salt represented by the following chemical formula (XVII): 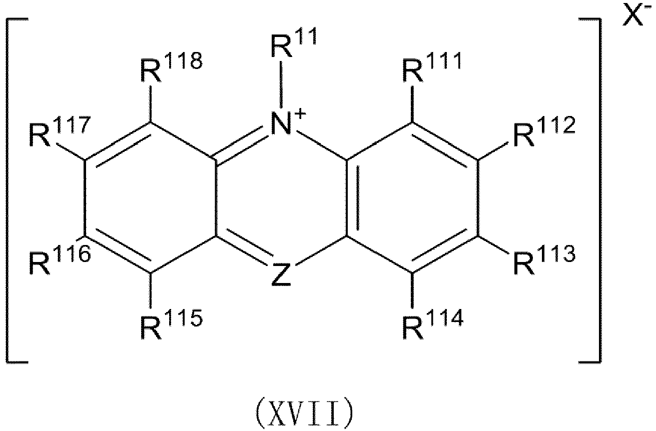 where in the chemical formula (XVII),
R¹¹¹ to R¹¹⁸ are each a hydrogen atom or a substituent, and may be identical to or different from each other, or
two or more of R¹¹¹ to R¹¹⁸ may be integrated to form a ring structure, which may be aromatic or non-aromatic and may or may not have one or more substituents,
Z is CH or N, and in the case of CH, H may be substituted with a substituent, and
R¹¹ and X⁻ are the same as those in the chemical formula (XI).

12. The radical generating catalyst according to any one of claims 1 to 11, wherein
the amino acid is at least one selected from the group consisting of glycine, alanine, valine, leucine, isoleucine, serine, threonine, aspartic acid, glutamic acid, asparagine, glutamine, lysine, hydroxylysine, arginine, cysteine, cystine, methionine, phenylalanine, tyrosine, tryptophan, histidine, proline, and 4-hydroxyproline.

13. The radical generating catalyst according to any one of claims 1 to 12, wherein
the peptide is at least one of oxidized glutathione (GSSG) or reduced glutathione (GSH).

14. The radical generating catalyst according to any one of claims 1 to 13, wherein
the phospholipid is at least one selected from the group consisting of phosphatidylserine, phosphatidylcholine, phosphatidic acid, phosphatidylethanolamine, phosphatidylglycerol, and cardiolipin.

15. The radical generating catalyst according to any one of claims 1 to 14, wherein
the radical generating catalyst has a Lewis acidity of 0.4 eV or more.

16. The radical generating catalyst according to any one of claims 1 to 15, wherein
the radical generating catalyst has an acid dissociation constant pKₐ of 5 or more as a Brønsted acid.

17. The radical generating catalyst according to any one of claims 1 to 16, wherein
the radical generating catalyst catalyzes radical generation from a radical source in a reaction system that is not acidic.

18. The radical generating catalyst according to any one of claims 1 to 16, wherein
the radical generating catalyst catalyzes radical generation from a radical source in a reaction system that is acidic.

19. The radical generating catalyst according to any one of claims 1 to 18, wherein
the radical generating catalyst catalyzes radical generation from a radical source in a liquid.

20. The radical generating catalyst according to any one of claims 1 to 19, wherein
the radical generating catalyst catalyzes radical generation from a radical source *in vivo.*

21. The radical generating catalyst according to any one of claims 1 to 20, wherein
the radical generating catalyst catalyzes radical generation from a radical source in a digestive organ.

22. The radical generating catalyst according to claim, wherein
the digestive organ is at least one selected from the group consisting of an oral cavity, a pharynx, an esophagus, a stomach, a duodenum, a small intestine and a large intestine.

23. The radical generating catalyst according to claim 21, wherein
the digestive organ is the large intestine.

24. The radical generating catalyst according to any one of claims 1 to 23, wherein
the radical source comprises an oxoacid.

25. The radical generating catalyst according to claim 24, wherein
the oxoacid is at least one selected from the group consisting of boric acid, carbonic acid, orthocarbonic acid, carboxylic acid, silicic acid, nitrous acid, nitric acid, phosphorous acid, phosphoric acid, arsenic acid, sulfurous acid, sulfuric acid, sulfonic acid, sulfinic acid, chromic acid, dichromic acid, permanganic acid, and a halogen oxoacid.

26. The radical generating catalyst according to claim 21, wherein
the halogen oxoacid is at least one selected from the group consisting of hypochlorous acid, chlorous acid, chloric acid, perchloric acid, hypobromous acid, bromous acid, bromic acid, perbromic acid, hypoiodous acid, iodous acid, iodic acid, and periodic acid.

27. The radical generating catalyst according to any one of claims 1 to 23, wherein
the radical source comprises at least one selected from the group consisting of halogen ions, hypohalite ions, halite ions, halate ions, and perhalate ions.

28. The radical generating catalyst according to claim 24, wherein
the oxoacid is a halogen oxoacid or a salt thereof.

29. The radical generating catalyst according to claim 28, wherein
the halogen oxoacid is chlorine oxoacid.

30. The radical generating catalyst according to any one of claims 1 to 19, wherein
the radical source comprises a chlorite ion

31. The radical generating catalyst according to any one of claims 1 to 26, wherein
the radical source comprises an electron donor-acceptor linked molecule.

32. The radical generating catalyst according to claim 31, wherein
the electron donor-acceptor linked molecule is at least one selected from the group consisting of: nitrogen-containing aromatic cation derivatives represented by the following formulae (A-1) to (A-8); 9-substituted acridinium ions represented by the following formula (A-9); quinolinium ion derivatives represented by the following formula (I); stereoisomers and tautomers thereof; and salts thereof: 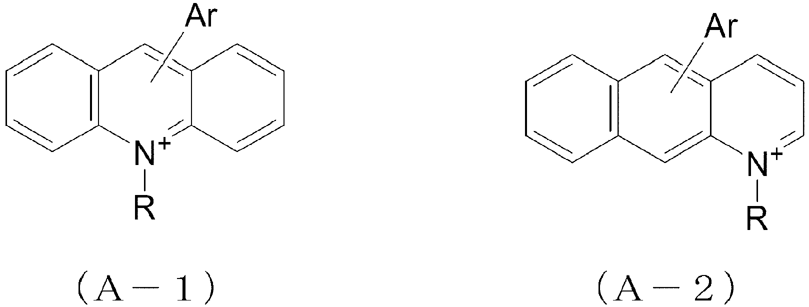 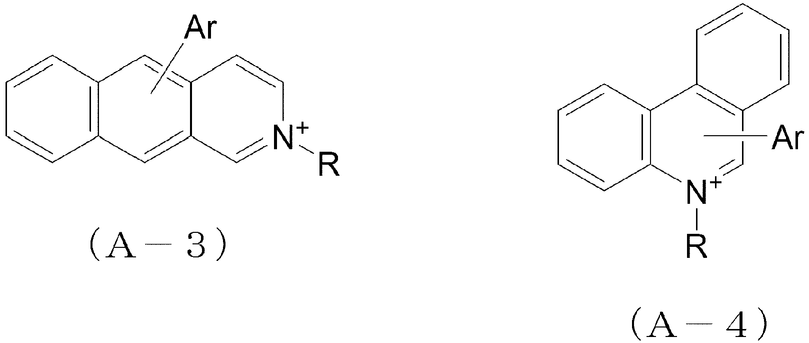 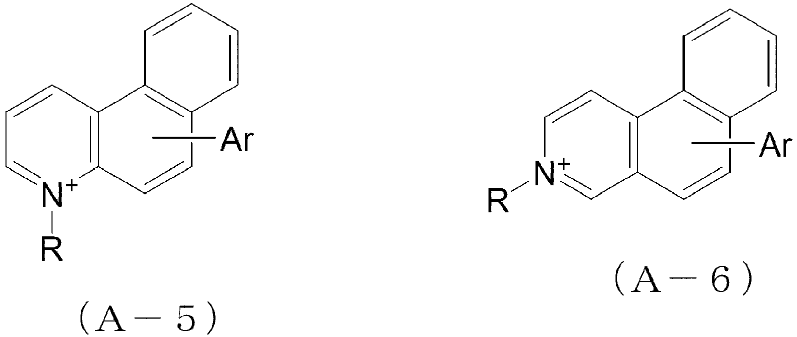 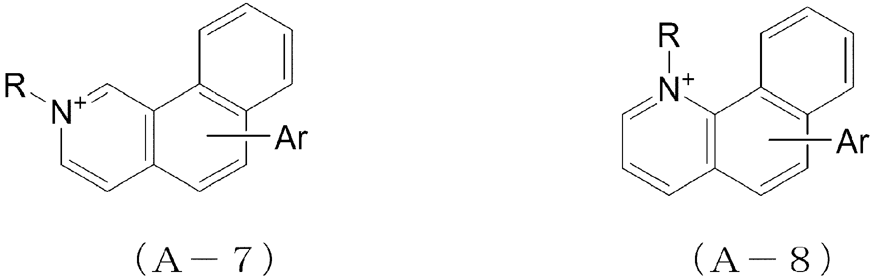 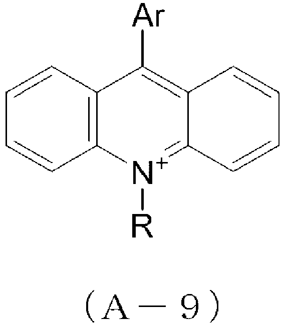 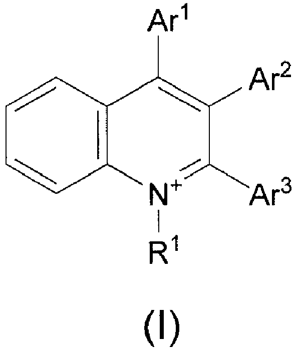 where in the formulae (A-1) to (A-8) and (A-9),
R is a hydrogen atom or any substituent,
Ar is the electron donor group, and the number of Ars may be one or more, and when a plurality of Ars are present, they may be identical to or different from each other,
a nitrogen-containing aromatic ring that forms a nitrogen-containing aromatic cation may or may not have at least one substituent other than R and Ar, and
in the formula (I),
R¹ is a hydrogen atom or any substituent,
Ar¹ to Ar³ are each a hydrogen atom or the electron donor group and may be identical to or different from each other, and at least one of Ar¹ to Ar³ is the electron donor group.

33. The radical generating catalyst according to claim 32, wherein
the electron donor-acceptor linked molecule is a 9-mesityl-10-methylacridinium ion represented by the following formula (A-10):

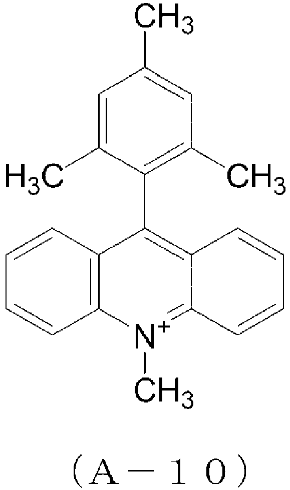

34. A radical generating catalyst comprising:
an ammonium salt represented by the following chemical formula (XI) (excluding peroxodisulfate) and having a Lewis acidity of 0.4 eV or more, wherein
the radical generating catalyst catalyzes radical generation from a radical source in a liquid that is not acidic, and
the radical source is at least one selected from the group consisting of halogenous acids, halite ions, and halites: 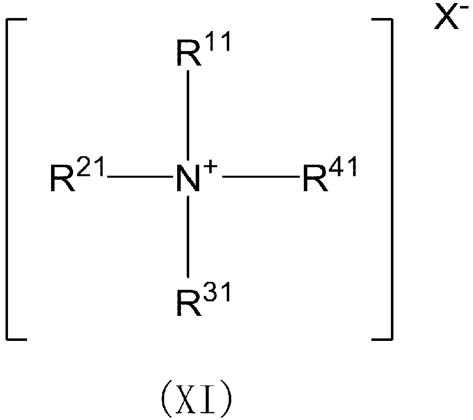
where in the chemical formula (XI),
R¹¹, R²¹, R³¹, and R⁴¹ are each a hydrogen atom or an aromatic ring, or an alkyl group, the alkyl group may comprise an ether bond, a carbonyl group, an ester bond, or an amide bond, or an aromatic ring, and R¹¹, R²¹, R³¹, and R⁴¹ may be identical to or different from each other, or
two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents, and
X⁻ is an anion (excluding peroxodisulfate ion).

35. The radical generating catalyst according to claim 34, wherein
in the ammonium salt represented by the chemical formula (XI),
R¹¹, R²¹, R³¹, and R⁴¹ are each a hydrogen atom or an alkyl group, and
R¹¹, R²¹, R³¹, and R⁴¹ may be identical to or different from each other.

36. The radical generating catalyst according to claim 35, wherein
the alkyl group is an alkyl group having 1 to 40 carbon atoms.

37. The radical generating catalyst according to claim 35, wherein
the alkyl group is an alkyl group having 1 to 6 carbon atoms.

38. The radical generating catalyst according to any one of claims 34 to 37, wherein
the ammonium salt represented by the chemical formula (XI) is an ammonium salt represented by the following chemical formula (XII): 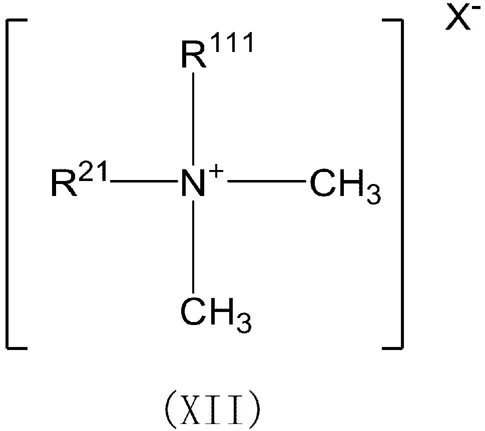 where in the chemical formula (XII),
R¹¹¹ is an alkyl group having 5 to 40 carbon atoms and may comprise an ether bond, a carbonyl group, an ester bond, or an amide bond, or an aromatic ring, and
R²¹ and X⁻ are the same as those in the chemical formula (XI).

39. The radical generating catalyst according to claim 34, wherein
the ammonium salt represented by the chemical formula (XI) is at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, lecithin, and cholines.

40. The radical generating catalyst according to claim 34, wherein
the ammonium salt represented by the chemical formula (XI) is an ammonium salt represented by the following chemical formula (XIII): 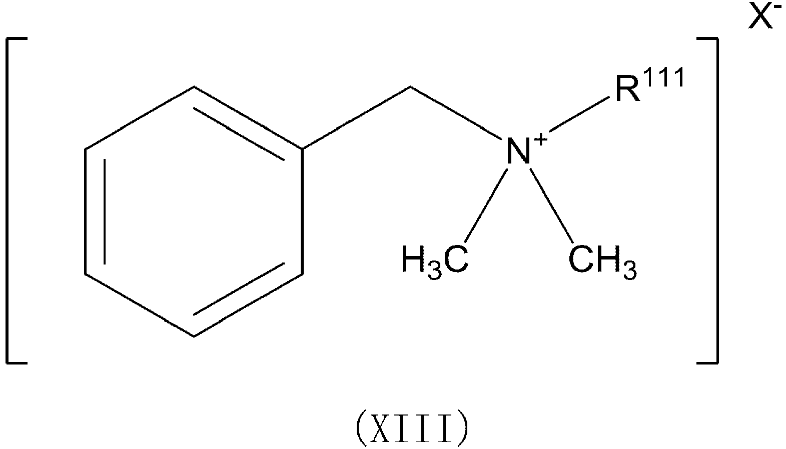 where in the chemical formula (XIII),
R¹¹¹ is an alkyl group having 5 to 40 carbon atoms, and
X⁻ is the same as that in the chemical formula (XI).

41. The radical generating catalyst according to any one of claims 34 to 40, wherein the ammonium salt is benzethonium chloride.

42. The radical generating catalyst according to claim 34, wherein
the ammonium salt represented by the chemical formula (XI) is an ammonium salt represented by the following chemical formula (XIV): 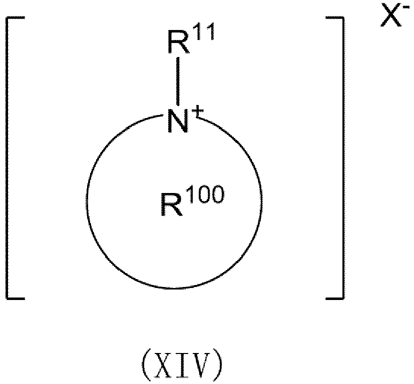 where in the chemical formula (XIV),
R¹⁰⁰ may form a ring structure, which may be saturated or unsaturated, aromatic or non-aromatic, and may or may not have one or more substituents, and
R¹¹ and X⁻ are the same as those in the chemical formula (XI).

43. The radical generating catalyst according to claim 34, wherein
the ammonium salt represented by the chemical formula (XI) is an ammonium salt represented by the following chemical formula (XV): 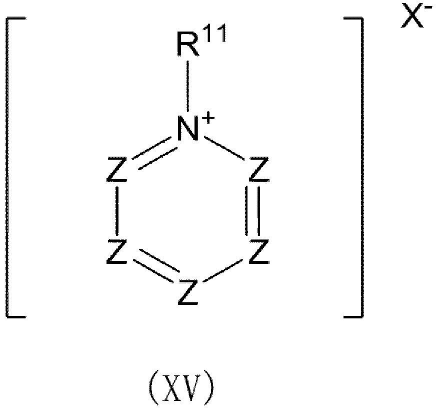 where in the chemical formula (XV),
Zs are each CH or N, may be identical to or different from each other, and in the case of CH, H may be substituted with a substituent, and
R¹¹ and X⁻ are the same as those in the chemical formula (XI).

44. The radical generating catalyst according to claim 34, wherein
the ammonium salt represented by the chemical formula (XI) is an ammonium salt represented by the following chemical formula (XVI): 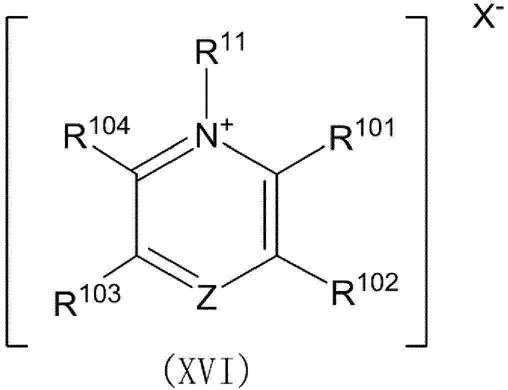 where in the chemical formula (XVI),
R¹⁰¹, R¹⁰², R¹⁰³, and R¹⁰⁴ are each a hydrogen atom or a substituent, and R¹⁰¹, R¹⁰², R¹⁰³, and R¹⁰⁴ may be identical to or different from each other, or
two or more of R¹⁰¹, R¹⁰², R¹⁰³, and R¹⁰⁴ may be integrated to form a ring structure with N⁺ to which they are bonded, and the ring structure may be saturated or unsaturated, aromatic or non-aromatic, and may or may not have one or more substituents,
Z is CH or N, and in the case of CH, H may be substituted with a substituent, and
R¹¹ and X⁻ are the same as those in the chemical formula (XI).

45. The radical generating catalyst according to claim 34, wherein
the ammonium salt represented by the chemical formula (XI) is an ammonium salt represented by the following chemical formula (XVII): 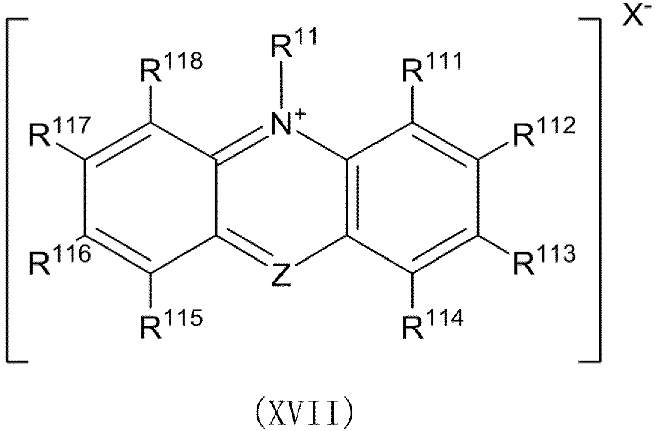 where in the chemical formula (XVII),
R¹¹¹ to R¹¹⁸ are each a hydrogen atom or a substituent, and may be identical to or different from each other, or
two or more of R¹¹¹ to R¹¹⁸ may be integrated to form a ring structure, and the ring structure may be aromatic or non-aromatic, and may or may not have one or more substituents,
Z is CH or N, and in the case of CH, H may be substituted with a substituent, and
R¹¹ and X⁻ are the same as those in the chemical formula (XI).

46. The radical generating catalyst according to claim 34, wherein
the ammonium salt is a salt of NH₄⁺.

47. The radical generating catalyst according to claim 46, wherein
the ammonium salt is NH₄Cl.

48. The radical generating catalyst according to any one of claims 34 to 38 and 42 to 46, wherein
the ammonium salt is a hexafluorophosphate of the ammonium.

49. The radical generating catalyst according to any one of claims 34 to 48, wherein
the halogenous acids are at least one selected from the group consisting of chlorous acid, bromous acid, and iodous acid.

50. The radical generating catalyst according to any one of claims 34 to 48, wherein
the radical source is a chlorite ion.

51. A radical generating catalyst comprising:
an ammonium salt represented by the following chemical formula (XI) and having a Lewis acidity of 0.4 eV or more, wherein
the radical generating catalyst catalyzes radical generation from a radical source in the presence of an oxidizing agent,
the oxidizing agent is O₂, and
the radical source is at least one selected from the group consisting of: nitrogen-containing aromatic cation derivatives represented by the following formulae (A-1) to (A-8); 9-substituted acridinium ions represented by the following formula (A-9); quinolinium ion derivatives represented by the following formula (I); stereoisomers and tautomers thereof; and salts thereof: 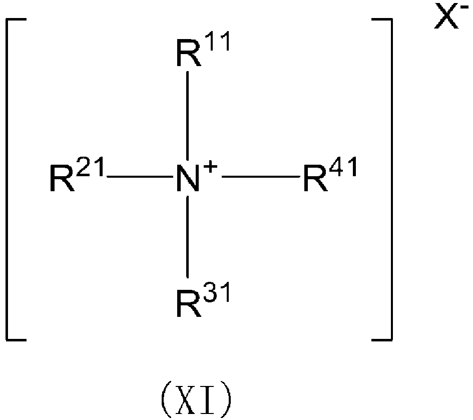
where in the chemical formula (XI),
R¹¹, R²¹, R³¹, and R⁴¹ are each a hydrogen atom or an aromatic ring, or an alkyl group, the alkyl group may comprise an ether bond, a carbonyl group, an ester bond, or an amide bond, or an aromatic ring, and R¹¹, R²¹, R³¹, and R⁴¹ may be identical to or different from each other, or
two or more of R¹¹, R²¹, R³¹, and R⁴¹ may be integrated to form a ring structure with N⁺ to which they are bonded, and the ring structure may be saturated or unsaturated, aromatic or non-aromatic, and may or may not have one or more substituents, and
X⁻ is an anion, 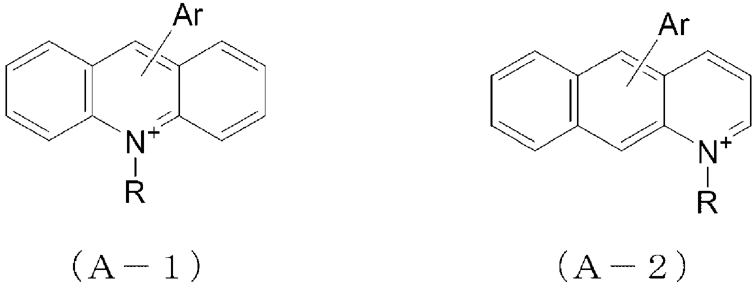 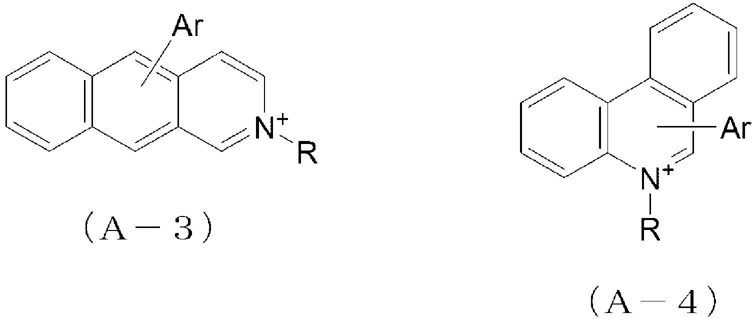 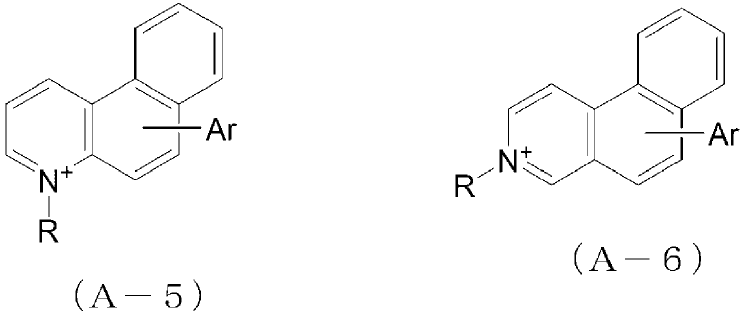 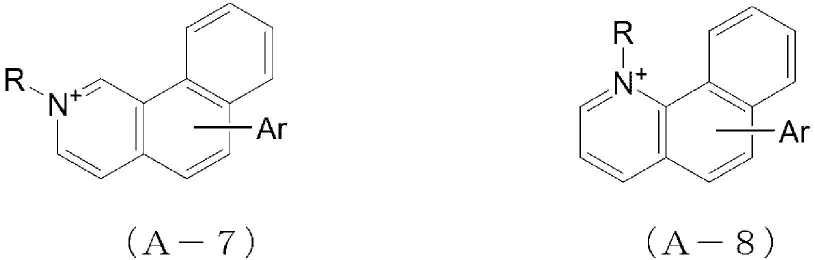 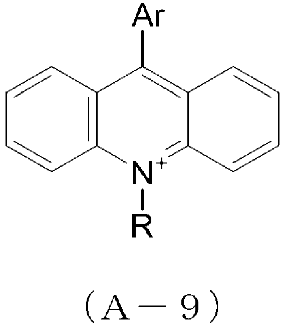 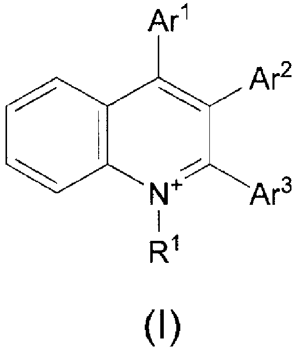
where in the formulae (A-1) to (A-8) and (A-9),
R is a hydrogen atom or any substituent,
Ar is an electron donor group, and the number of Ars may be one or more, and when a plurality of Ars are present, they may be identical to or different from each other,
a nitrogen-containing aromatic ring that forms a nitrogen-containing aromatic cation may or may not have at least one substituent other than R and Ar, and
in the formula (I),
R¹ is a hydrogen atom or any substituent,
Ar¹ to Ar³ are each a hydrogen atom or the electron donor group and may be identical to or different from each other, and at least one of Ar¹ to Ar³ is the electron donor group.

52. The radical generating catalyst according to claim 51, wherein
R¹¹, R²¹, R³¹, and R⁴¹ are each a hydrogen atom or an alkyl group, and
R¹¹, R²¹, R³¹, and R⁴¹ may be identical to or different from each other.

53. The radical generating catalyst according to claim 52, wherein
the alkyl group is an alkyl group having 1 to 40 carbon atoms.

54. The radical generating catalyst according to claim 52, wherein
the alkyl group is an alkyl group having 1 to 6 carbon atoms.

55. The radical generating catalyst according to any one of claims 51 to 54, wherein
the ammonium salt represented by the chemical formula (XI) is an ammonium salt represented by the following chemical formula (XII): 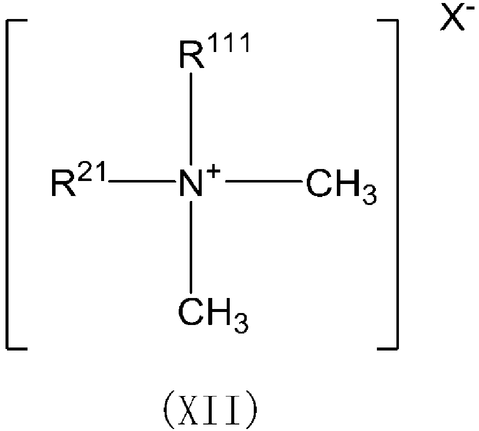 where in the chemical formula (XII),
R¹¹¹ is an alkyl group having 5 to 40 carbon atoms and may comprise an ether bond, a carbonyl group, an ester bond, or an amide bond, or an aromatic ring, and
R²¹ and X⁻ are the same as those in the chemical formula (XI).

56. The radical generating catalyst according to claim 51, wherein
the ammonium salt is at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, lecithin, and cholines.

57. The radical generating catalyst according to claim 51, wherein
the ammonium salt represented by the chemical formula (XI) is an ammonium salt represented by the following chemical formula (XIII): 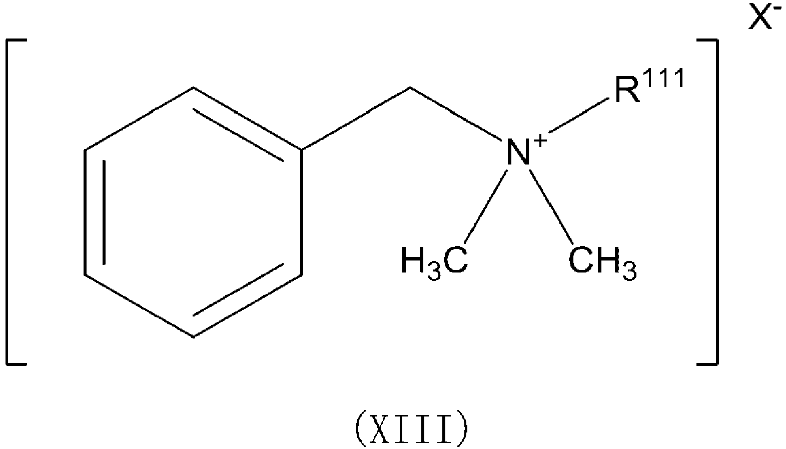 where in the chemical formula (XIII),
R¹¹¹ is an alkyl group having 5 to 40 carbon atoms, and
X⁻ is an anion.

58. The radical generating catalyst according to any one of claims 51 to 57, wherein
the ammonium salt is benzethonium chloride.

59. The radical generating catalyst according to claim 51, wherein
the ammonium salt represented by the chemical formula (XI) is an ammonium salt represented by the following chemical formula (XIV): 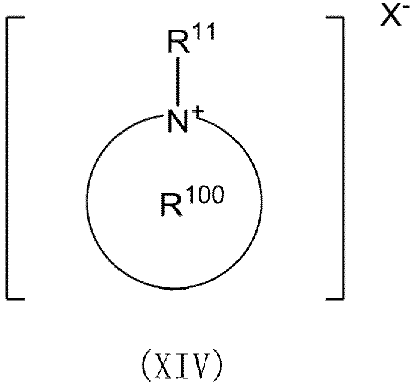 where in the chemical formula (XIV),
R¹⁰⁰ may form a ring structure, which may be saturated or unsaturated, aromatic or non-aromatic, and may or may not have one or more substituents, and
R¹¹ and X⁻ are the same as those in the chemical formula (XI).

60. The radical generating catalyst according to claim 51, wherein
the ammonium salt represented by the chemical formula (XI) is an ammonium salt represented by the following chemical formula (XV): 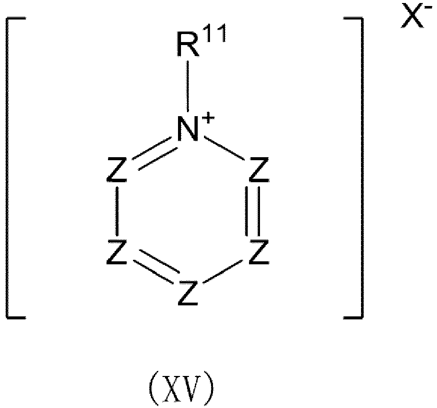 where in the chemical formula (XV),
Zs are each CH or N, may be identical to or different from each other, and in the case of CH, H may be substituted with a substituent, and
R¹¹ and X⁻ are the same as those in the chemical formula (XI).

61. The radical generating catalyst according to claim 51, wherein
the ammonium salt represented by the chemical formula (XI) is an ammonium salt represented by the following chemical formula (XVI): 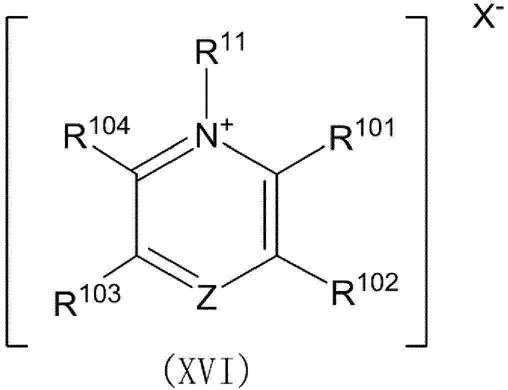 where in the chemical formula (XVI),
R¹⁰¹, R¹⁰², R¹⁰³, and R¹⁰⁴ are each a hydrogen atom or a substituent, and R¹⁰¹, R¹⁰², R¹⁰³, and R¹⁰⁴ may be identical to or different from each other, or
two or more of R¹⁰¹, R¹⁰², R¹⁰³, and R¹⁰⁴ may be integrated to form a ring structure with N⁺ to which they are bonded, and the ring structure may be saturated or unsaturated, aromatic or non-aromatic, and may or may not have one or more substituents,
Z is CH or N, and in the case of CH, H may be substituted with a substituent, and
R¹¹ and X⁻ are the same as those in the chemical formula (XI).

62. The radical generating catalyst according to claim 51, wherein
the ammonium salt represented by the chemical formula (XI) is an ammonium salt represented by the following chemical formula (XVII): 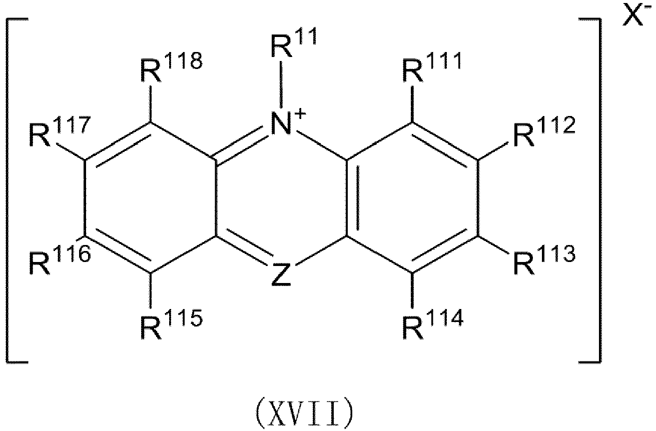 where in the chemical formula (XVII),
R¹¹¹ to R¹¹⁸ are each a hydrogen atom or a substituent, and may be identical to or different from each other, or
two or more of R¹¹¹ to R¹¹⁸ may be integrated to form a ring structure, and the ring structure may be aromatic or non-aromatic, and may or may not have one or more substituents,
Z is CH or N, and in the case of CH, H may be substituted with a substituent, and
R¹¹ and X⁻ are the same as those in the chemical formula (XI).

63. The radical generating catalyst according to claim 51, wherein
the ammonium salt is a salt of NH₄⁺.

64. The radical generating catalyst according to claim 63, wherein
the ammonium salt is NH₄Cl.

65. The radical generating catalyst according to any one of claims 51 to 55, 57, and 59 to 63, wherein
the ammonium salt is a hexafluorophosphate of the ammonium.

66. The radical generating catalyst according to any one of claims 51 to 65, wherein
the radical source is a 9-mesityl-10-methylacridinium ion represented by the following formula (A-10) and/or a salt thereof.

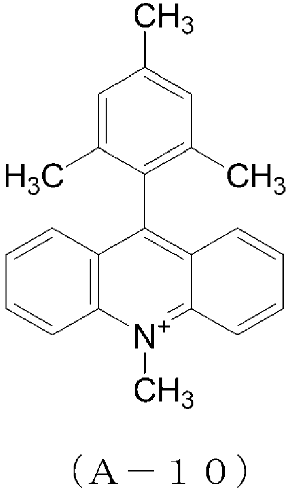

67. The radical generating catalyst according to any one of claims 1 to 66, wherein
a reaction rate constant (k_{cat}) for the chemical reaction formula (1b) below is 1.0×10⁻⁵S⁻¹ or more: 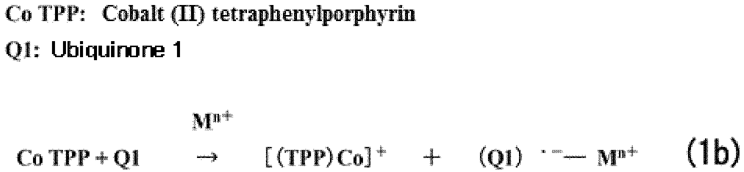 in the chemical formula (1b),
Mⁿ⁺ represents the radical generating catalyst,
CoTPP represents cobalt (II) tetraphenylporphyrin,
Q1 represents ubiquinone 1,
[(TPP)Co]⁺ represents cobalt (III) tetraphenylporphyrin cation, and
(Q1)˙⁻ represents an anionic radical of ubiquinone 1.

68. A method for producing a radical, the method comprising:
a mixing step of mixing the radical generating catalyst according to any one of claims 1 to 67 with the radical source.

69. The method according to claim 68, wherein
in the mixing step, a solvent further is mixed.

70. The method according to claim 69 or 70, further comprising:
a light irradiation step of irradiating a mixture obtained in the mixing step with light.

71. A method for producing a radical, the method comprising:
a mixing step of mixing a Lewis acid having a Lewis acidity of 0.4 eV or more (excluding peroxodisulfate) with a radical source; and
a reaction step of reacting the Lewis acid with the radical source in a liquid that is not acidic, wherein
the radical source is at least one selected from the group consisting of halogenous acids, halite ions, and halites.

72. The method according to claim 66, wherein
the halogenous acids are at least one selected from the group consisting of chlorous acid, bromous acid, and iodous acid.

73. The method according to claim 71 or 72, wherein
the radical source is a chlorite ion.

74. The method according to any one of claims 71 to 73, wherein
the Lewis acid is the radical generating catalyst according to any one of claims 34 to 50.

75. A method for producing a radical, the method comprising:
a mixing step of mixing a Lewis acid having a Lewis acidity of 0.4 eV or more, O₂, and a radical source together; and
a reaction step of reacting the Lewis acid, the O₂, and the radical source with each other in a liquid, wherein
the Lewis acid is the radical generating catalyst according to any one of claims 51 to 66, and
the radical source is at least one selected from the group consisting of: nitrogen-containing aromatic cation derivatives represented by the formulae (A-1) to (A-8); 9-substituted acridinium ions represented by the formula (A-9); quinolinium ion derivatives represented by the formula (I); stereoisomers and tautomers thereof; and salts thereof.

76. The method according to claim 70, wherein
the Lewis acid is the radical generating catalyst according to claim 66.

77. The method according to any one of claims 71 to 76, wherein
in the mixing step, a solvent further is mixed.

78. The method according to any one of claims 71 to 77, further comprising:
a light irradiation step of irradiating a mixture obtained in the mixing step with light.

79. A method for producing a radical, the method comprising:
a mixing step of mixing a Lewis acid having a Lewis acidity of 0.4 eV or more (excluding peroxodisulfate) with a radical source; and
a reaction step of reacting the Lewis acid with the radical source in a liquid, wherein
the Lewis acid having a Lewis acidity of 0.4 eV or more comprises an inorganic substance, and
the radical source is at least one selected from the group consisting of halogenous acids, halite ions, and halites.

80. The method according to claim 79, wherein
the inorganic substance comprises a metal ion.

81. The method according to claim 79, wherein
the inorganic substance is at least one selected from the group consisting of alkaline-earth metal ions, rare-earth ions, Mg²⁺, Sc³⁺, Li⁺, Fe²⁺, Fe³⁺, Al³⁺, silicate ions, and borate ions.

82. The method according to any one of claims 70 to 73 and 79, wherein
the Lewis acid having a Lewis acidity of 0.4 eV or more is at least one selected from the group consisting of AlCl₃, AlMeCl₂, AlMe₂Cl, BF₃, BPh₃, BMe₃, TiCl₄, SiF₄, and SiCl₄.

83. The method according to any one of claims 79 to 82, wherein
the halogenous acids are at least one selected from the group consisting of chlorous acid, bromous acid, and iodous acid.

84. The method according to any one of claims 79 to 83, wherein
the radical source is a chlorite ion.

85. A method for producing an oxidation reaction product by oxidizing a substance to be oxidized, the method comprising:
a radical production step of producing a radical by the method according to any one of claims 68 to 84; and
an oxidation reaction step of reacting the substance to be oxidized with an oxidizing agent by action of the radical, thereby generating the oxidation reaction product.

86. The method according to claim 85, wherein
the radical also serves as the oxidizing agent.

87. A drug comprising:
a radical generating catalyst; and
a radical source, wherein
the radical generating catalyst is the radical generating catalyst according to any one of claims 1 to 67.

88. The drug according to claim 87, wherein
the radical source comprises an oxoacid.

89. The drug according to claim 88, wherein
the oxoacid is at least one selected from the group consisting of boric acid, carbonic acid, orthocarbonic acid, carboxylic acid, silicic acid, nitrous acid, nitric acid, phosphorous acid, phosphoric acid, arsenic acid, sulfurous acid, sulfuric acid, sulfonic acid, sulfinic acid, chromic acid, dichromic acid, permanganic acid, and a halogen oxoacid.

90. The drug according to claim 89, wherein
the halogen oxoacid is at least one selected from the group consisting of hypochlorous acid, chlorous acid, chloric acid, perchloric acid, hypobromous acid, bromous acid, bromic acid, perbromic acid, hypoiodous acid, iodous acid, iodic acid, and periodic acid.

91. The drug according to claim 87, wherein
the radical source comprises at least one selected from the group consisting of halogen ions, hypohalite ions, halite ions, halate ions, and perhalate ions.

92. The drug according to claim 88, wherein
the oxoacid is a halogen oxoacid or a salt thereof.

93. The drug according to claim 92, wherein
the halogen oxoacid is chlorine oxoacid.

94. The drug according to claim 88, wherein
the oxoacid comprises chlorite ion.

95. A liquid drug comprising:
a radical generating catalyst; and
at least one selected from the group consisting of halogenous acids, halite ions, and halites, wherein
the radical generating catalyst is the radical generating catalyst according to any one of claims 1 to 30 and 34 to 50 and has a Lewis acidity of 0.4 eV or more, and
the liquid drug is not acidic.

96. The drug according to claim 95, wherein
the halogenous acids are at least one selected from the group consisting of chlorous acid, bromous acid, and iodous acid.

97. The drug according to claim 95, wherein
the halogenous acid is chlorous acid.

98. The drug according to any one of claims 87 to 97, further comprising:
water and/or an organic solvent.

99. The drug according to any one of claims 87 to 98, which is a bactericide.

100. The drug according to any one of claims 87 to 99, which is used *in vivo.*

101. The drug according to any one of claims 87 to 100, which is used in a digestive organ.

102. The drug according to claim 101, wherein
the digestive organ is at least one selected from the group consisting of an oral cavity, a pharynx, an esophagus, a stomach, a duodenum, a small intestine and a large intestine.

103. The drug according to claim 101, wherein
the digestive organ is the large intestine.

104. The drug according to any one of claims 100 to 103, which is a drug for use in treatment or in suppression of symptom of ulcerative colitis.

105. The drug according to any one of claims 87 to 104, which is a drug for use in agriculture and livestock industry.

106. The drug according to claim 105, wherein
the drug for use in agriculture and livestock industry is at least one selected from the group consisting of bactericides for use in agriculture, antiviral agents for use in agriculture, deodorizers for use in agriculture, insectcides for use in agriculture, repellents for use in agriculture, soil conditioners for use in agriculture, bactericides for use in livestock industry, antiviral agents for use in livestock industry, deodorizers for use in livestock industry, insectcides for use in livestock industry, repellents for use in livestock industry, and soil conditioners for use in livestock industry.
